# EUROPEAN PATENT SPECIFICATION

(11) **EP 4 479 400 B1**
(45) Date of publication and mention of the grant of the patent: **19.08.2026**
(21) Application number: 23708272.2
(22) Date of filing: 17.02.2023
(51) Int. Cl.: C07D 487/04, A61K 31/5513, A61P 11/00, A61P 25/04, A61P 35/00

(54) **HETEROCYCLIC COMPOUNDS USEFUL FOR TREATING A ERK5-MEDIATED DISEASE**
HETEROZYKLISCHE VERBINDUNGEN ZUR BEHANDLUNG EINER ERK5-VERMITTELTEN KRANKHEIT
COMPOSÉS HÉTÉROCYCLIQUES UTILES POUR LE TRAITEMENT D'UNE MALADIE MÉDIÉE PAR ERK5

(30) Priority: 18.02.2022 GB 202202199
(43) Date of publication of application: 25.12.2024
(73) Proprietor: Cancer Research Technology Limited, London E20 1JQ (GB)
(72) Inventor: BUTTERWORTH, Sam, Manchester Greater Manchester M13 9NT (GB); SMITH, Oliver Richard, Manchester Greater Manchester M13 9NT (GB); EVANS, Charles Nicholas George, Manchester Greater Manchester M13 9NT (GB); FINEGAN, Katherine Georgina, Manchester Greater Manchester M13 9NT (GB)
(74) Representative: HGF
(86) International application number: PCT/GB2023/050369
(87) International publication number: WO 2023/156791

(56) References cited:
- WO-A1-2015/117087
- WO-A1-2015/129927
- US-B2- 9 695 172

## Description

### FIELD OF THE INVENTION

The present invention relates to certain compounds that function as a proteolysis targeting chimera (PROTAC) against ERK5 kinase. The present invention also relates to processes for the preparation of these compounds, to pharmaceutical compositions comprising them, and to their use in the treatment of proliferative disorders, such as cancer, as well as other diseases or conditions in which ERK5 activity is implicated.

### BACKGROUND OF THE INVENTION

The extracellular signal-regulated kinase 5 (ERK5, also known as big MAP kinase 1, BMK1) protein, encoded by the MAPK7 gene, is a member of the mitogen-activated protein kinase (MAPK) family. The ERK5 signaling cascade can be activated by environmental stresses, mitogens and cytokines. These stimuli activate MEKK2 and MEKK3, which are able to phosphorylate and activate MEK5. Once activated, MEK5 phosphorylates the TEY motif in the activation loop of the ERK5 kinase domain, thereby leading to ERK5 activation, (for review see Hoang et al, 2017. Cancer letters; Drew et al, 2012. Biochimica et Biophysica Acta; Nithianandarajah-Jones et al, 2012. Cellular Signalling).

ERK5 is a MAPK with unique funtions within the MAPK family, i.e. non-kinase functions in cell cycle progression and transcriptional activation. ERK5 is upregulated in almost all cancers. MEK5/ERK5 upregulation or hyperactivation correlates with poorer outcomes and resistance to therapy in several cancers, including Mesothelioma, Osteosarcoma, Triple-negative breast cancer, Oesophageal cancer, Prostate cancer and Lung Cancer. ERK5 has very little homeostatic functions, it is a stress kinase that is "switched on" in pathology, making it an attractive drug target.

To date, approaches to reduce ERK5 kinase activity have focused on kinase inhibition. This approach has several issues. It has been found that kinase inhibition can cause paradoxical activation of ERK5. Kinase inhibitors have also been non-specific and have had activity towards proteins needed for health e.g. BRD4. Furthermore, specific iterations of ERK5 kinase inhibitors fail to recapitulate the phenotype of ERK5 loss (genetic/siRNA). This is especially true of the immunological effects. This is due to the kinase inhibitors failing to inhibit all aspects of ERK5 function i.e. the kinase and non-kinase dependent functions. WO2015129927A1 discloses compounds that are useful in the treatment of a ERK5-mediated disease and/or a BET protein-mediated disease.

There is a need for alternative ways of reducing ERK5 activity without inhibition, for example compounds that can affect all aspects of ERK5 function (kinase and non-kinase dependent) and recapitulate the strong anti-cancer phenotype of ERK5 loss (genetic/siRNA) via a pharmacological approach. One such approach involves degradation.

An object of this invention is to provide compounds which degrade ERK5 kinase.

### SUMMARY OF THE INVENTION

In one aspect, the present invention provides a compound as defined herein, or a pharmaceutically acceptable salt or solvate thereof.

In another aspect, the present invention provides a pharmaceutical composition as defined herein which comprises a compound as defined herein, or a pharmaceutically acceptable salt or solvate thereof, and one or more pharmaceutically acceptable excipients.

In another aspect, the present invention provides a compound as defined herein, or a pharmaceutically acceptable salt or solvate thereof, or a pharmaceutical composition as defined herein, for use in therapy.

In another aspect, the present invention provides a compound as defined herein, or a pharmaceutically acceptable salt or solvate thereof, or a pharmaceutical composition as defined herein, for use in the treatment of a proliferative condition. Suitably, the proliferative condition is cancer.

In another aspect, the present invention provides a compound as defined herein, or a pharmaceutically acceptable salt or solvate thereof, or a pharmaceutical composition as defined herein, for use in the treatment of:
a) a fibrotic disease, e.g. impaired wound healing, pulmonary fibrosis,
b) an Inflammatory disease e.g. psoriasis, asthma,
c) a proliferative condition, e.g. cancers such as triple-negative breast cancer, mesothelioma, primary bone cancer, glioblastoma, lung cancer, squamous cell carcinoma,
d) diabetes,
e) pain, or
f) a central nervous system (CNS) disorder e.g. Parkinsons disease, dementia.

In another aspect, the present invention provides a compound as defined herein, or a pharmaceutically acceptable salt or solvate thereof, or a pharmaceutical composition as defined herein, for use in the treatment of cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin-sensitive rhinosinusitis, cystic fibrosis, multiple sclerosis, kidney diseases, diabetes, pain, fibrotic diseases, for example impaired wound healing and pulmonary fibrosis; and cancer pain.

In another aspect, the present invention provides a compound as defined herein, or a pharmaceutically acceptable salt or solvate thereof, or a pharmaceutical composition as defined herein, for use in the treatment of cancer. In a particular embodiment, the cancer is a human cancer. In a particular embodiment, the cancer is selected from triple-negative breast cancer, mesothelioma, primary bone cancer, glioblastoma, lung cancer or squamous cell carcinoma.

In another aspect, the present invention provides a compound as defined herein, or a pharmaceutically acceptable salt or solvate thereof, or a pharmaceutical composition as defined herein, for use in the treatment of a central nervous system (CNS) disorder. In a particular embodiment, the central nervous system (CNS) disorder is Parkinsons disease or dementia.

In another aspect, the present invention provides a compound as defined herein, or a pharmaceutically acceptable salt or solvate thereof, or a pharmaceutical composition as defined herein, for use in the degradation of, or reducing the amount of, ERK5 kinase.

Also disclosed herein is the use of a compound as defined herein, or a pharmaceutically acceptable salt or solvate thereof, in the manufacture of a medicament for use in the treatment of a proliferative condition.

Also disclosed herein is the use of a compound as defined herein, or a pharmaceutically acceptable salt or solvate thereof, in the manufacture of a medicament for use in the treatment of:
a) a fibrotic disease, e.g. impaired wound healing, pulmonary fibrosis,
b) an Inflammatory disease e.g. psoriasis, asthma,
c) a proliferative condition, e.g. cancers such as triple-negative breast cancer, mesothelioma, primary bone cancer, glioblastoma, lung cancer, squamous cell carcinoma,
d) diabetes,
e) pain, or
f) a central nervous system (CNS) disorder e.g. Parkinsons disease, dementia.

Also disclosed herein is the use of a compound as defined herein, or a pharmaceutically acceptable salt or solvate thereof, in the manufacture of a medicament for use in the treatment of cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin-sensitive rhinosinusitis, cystic fibrosis, multiple sclerosis, kidney diseases, diabetes, pain, fibrotic diseases, for example impaired wound healing and pulmonary fibrosis; and cancer pain.

Also disclosed herein is the use of a compound as defined herein, or a pharmaceutically acceptable salt or solvate thereof, in the manufacture of a medicament for use in the treatment of cancer. Suitably, the medicament is for use in the treatment of human cancers.

Also disclosed herein is the use of a compound as defined herein, or a pharmaceutically acceptable salt or solvate thereof, in the manufacture of a medicament for use in the degradation of, or reducing the amount of, ERK5 kinase.

Also disclosed herein is a method of degrading ERK5 *in vitro* or *in vivo,* said method comprising contacting a cell with an effective amount of a compound as defined herein, or a pharmaceutically acceptable salt or solvate thereof.

Also disclosed herein is a method of inhibiting cell proliferation *in vitro* or *in vivo,* said method comprising contacting a cell with an effective amount of a compound as defined herein, or a pharmaceutically acceptable salt or solvate thereof.

Also disclosed herein is a method of treating:
a) a fibrotic disease, e.g. impaired wound healing, pulmonary fibrosis,
b) an Inflammatory disease e.g. psoriasis, asthma,
c) a proliferative condition, e.g. cancers such as triple-negative breast cancer, mesothelioma, primary bone cancer, glioblastoma, lung cancer, squamous cell carcinoma,
d) diabetes,
e) pain, or
f) a central nervous system (CNS) disorder e.g. Parkinsons disease, dementia;
in a patient in need of such treatment, said method comprising administering to said patient a therapeutically effective amount of a compound as defined herein, or a pharmaceutically acceptable salt or solvate thereof, or a pharmaceutical composition as defined herein.

Also disclosed herein is a method of treating: cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin-sensitive rhinosinusitis, cystic fibrosis, multiple sclerosis, kidney diseases, diabetes, pain, fibrotic diseases, for example impaired wound healing and pulmonary fibrosis; and cancer pain.

Also disclosed herein is a method of treating a proliferative disorder in a patient in need of such treatment, said method comprising administering to said patient a therapeutically effective amount of a compound as defined herein, or a pharmaceutically acceptable salt or solvate thereof, or a pharmaceutical composition as defined herein.

Also disclosed herein is a method of treating cancer in a patient in need of such treatment, said method comprising administering to said patient a therapeutically effective amount of a compound as defined herein, or a pharmaceutically acceptable salt or solvate thereof, or a pharmaceutical composition as defined herein.

The present invention further provides a method of synthesising a compound, or a pharmaceutically acceptable salt or solvate thereof, as defined herein.

In another aspect, the present invention provides a compound as defined herein, or a pharmaceutically acceptable salt or solvate thereof, obtainable by, or obtained by, or directly obtained by a method of synthesis as defined herein.

In another aspect, the present invention provides novel intermediates as defined herein which are suitable for use in any one of the synthetic methods as set out herein.

Preferred, suitable, and optional features of any one particular aspect of the present invention are also preferred, suitable, and optional features of any other aspect.

### DETAILED DESCRIPTION OF THE INVENTION

### Definitions

Unless otherwise stated, the following terms used in the specification and claims have the following meanings set out below.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

In this specification the term "alkyl" includes both straight and branched chain alkyl groups. References to individual alkyl groups such as "propyl" are specific for the straight chain version only and references to individual branched chain alkyl groups such as "isopropyl" are specific for the branched chain version only. For example, "(1-6C)alkyl" includes (1-4C)alkyl, (1-3C)alkyl, propyl, isopropyl and t-butyl. A similar convention applies to other radicals, for example "phenyl(1-6C)alkyl" includes phenyl(1-4C)alkyl, benzyl, 1-phenylethyl and 2-phenylethyl.

The term "(m-nC)" or "(m-nC) group" used alone or as a prefix, refers to any group having m to n carbon atoms.

An "alkylene," "alkenylene," or "alkynylene" group is an alkyl, alkenyl, or alkynyl group that is positioned between and serves to connect two other chemical groups. Thus, "(1-6C)alkylene" means a linear saturated divalent hydrocarbon radical of one to six carbon atoms or a branched saturated divalent hydrocarbon radical of three to six carbon atoms, for example, methylene, ethylene, propylene, 2-methylpropylene, pentylene, and the like.

"(2-6C)alkenylene" means a linear divalent hydrocarbon radical of two to six carbon atoms or a branched divalent hydrocarbon radical of three to six carbon atoms, containing at least one double bond, for example, as in ethenylene, 2,4-pentadienylene, and the like.

"(2-6C)alkynylene" means a linear divalent hydrocarbon radical of two to six carbon atoms or a branched divalent hydrocarbon radical of three to six carbon atoms, containing at least one triple bond, for example, as in ethynylene, propynylene, and butynylene and the like.

"(3-8C)cycloalkyl" means a hydrocarbon ring containing from 3 to 8 carbon atoms, for example, cyclopropyl, cyclobutyl, cyclopentyl, cyclohexyl, cycloheptyl or bicyclo[2.2.1]heptyl.

"(3-8C)cycloalkenyl" means a hydrocarbon ring containing at least one double bond, for example, cyclobutenyl, cyclopentenyl, cyclohexenyl or cycloheptenyl, such as 3-cyclohexen-1-yl, or cyclooctenyl.

"(3-8C)cycloalkyl-(1-6C)alkylene" means a (3-8C)cycloalkyl group covalently attached to a (1-6C)alkylene group, both of which are defined herein.

The term "halo" or "halogeno" refers to fluoro, chloro, bromo and iodo.

The term "heterocyclyl", "heterocyclic" or "heterocycle" means a non-aromatic saturated or partially saturated monocyclic, fused, bridged, or spiro bicyclic heterocyclic ring system(s). The term heterocyclyl includes both monovalent species and divalent species. Monocyclic heterocyclic rings contain from about 3 to 12 (suitably from 3 to 7) ring atoms, with from 1 to 5 (suitably 1, 2 or 3) heteroatoms selected from nitrogen, oxygen or sulfur in the ring. Bicyclic heterocycles contain from 7 to 17 member atoms, suitably 7 to 12 member atoms, in the ring. Bicyclic heterocycles contain from about 7 to about 17 ring atoms, suitably from 7 to 12 ring atoms. Bicyclic heterocyclic(s) rings may be fused, spiro, or bridged ring systems. Examples of heterocyclic groups include cyclic ethers such as oxiranyl, oxetanyl, tetrahydrofuranyl, dioxanyl, and substituted cyclic ethers. Heterocycles containing nitrogen include, for example, azetidinyl, pyrrolidinyl, piperidinyl, piperazinyl, tetrahydrotriazinyl, tetrahydropyrazolyl, and the like. Typical sulfur containing heterocycles include tetrahydrothienyl, dihydro-1,3-dithiol, tetrahydro-2*H*-thiopyran, and hexahydrothiepine. Other heterocycles include dihydro-oxathiolyl, tetrahydro-oxazolyl, tetrahydro-oxadiazolyl, tetrahydrodioxazolyl, tetrahydro-oxathiazolyl, hexahydrotriazinyl, tetrahydro-oxazinyl, morpholinyl, thiomorpholinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or SO₂ groups are also included. Examples include the sulfoxide and sulfone forms of tetrahydrothienyl and thiomorpholinyl such as tetrahydrothiene 1,1-dioxide and thiomorpholinyl 1,1-dioxide. A suitable value for a heterocyclyl group which bears 1 or 2 oxo (=O) or thioxo (=S) substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. Particular heterocyclyl groups are saturated monocyclic 3 to 7 membered heterocyclyls containing 1, 2 or 3 heteroatoms selected from nitrogen, oxygen or sulfur, for example azetidinyl, tetrahydrofuranyl, tetrahydropyranyl, pyrrolidinyl, morpholinyl, tetrahydrothienyl, tetrahydrothienyl 1,1-dioxide, thiomorpholinyl, thiomorpholinyl 1,1-dioxide, piperidinyl, homopiperidinyl, piperazinyl or homopiperazinyl. As the skilled person would appreciate, any heterocycle may be linked to another group via any suitable atom, such as via a carbon or nitrogen atom. However, reference herein to piperidino or morpholino refers to a piperidin-1-yl or morpholin-4-yl ring that is linked via the ring nitrogen.

By "bridged ring systems" is meant ring systems in which two rings share more than two atoms, see for example Advanced Organic Chemistry, by Jerry March, 4th Edition, Wiley Interscience, pages 131-133, 1992. Examples of bridged heterocyclyl ring systems include, aza-bicyclo[2.2.1]heptane, 2-oxa-5-azabicyclo[2.2.1]heptane, aza-bicyclo[2.2.2]octane, aza-bicyclo[3.2.1]octane and quinuclidine.

"Heterocyclyl(1-6C)alkyl" means a heterocyclyl group covalently attached to a (1-6C)alkylene group, both of which are defined herein.

The term "heteroaryl" or "heteroaromatic" means an aromatic mono-, bi-, or polycyclic ring incorporating one or more (for example 1-4, particularly 1, 2 or 3) heteroatoms selected from nitrogen, oxygen or sulfur. The term heteroaryl includes both monovalent species and divalent species. Examples of heteroaryl groups are monocyclic and bicyclic groups containing from five to twelve ring members, and more usually from five to ten ring members. The heteroaryl group can be, for example, a 5- or 6-membered monocyclic ring or a 9- or 10-membered bicyclic ring, for example a bicyclic structure formed from fused five and six membered rings or two fused six membered rings. Each ring may contain up to about four heteroatoms typically selected from nitrogen, sulfur and oxygen. Typically the heteroaryl ring will contain up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of heteroaryl include furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, isoindolyl, benzothienyl, benzoxazolyl, benzimidazolyl, benzothiazolyl, benzothiazolyl, indazolyl, purinyl, benzofurazanyl, quinolyl, isoquinolyl, quinazolinyl, quinoxalinyl, cinnolinyl, pteridinyl, naphthyridinyl, carbazolyl, phenazinyl, benzisoquinolinyl, pyridopyrazinyl, thieno[2,3-b]furanyl, 2H-furo[3,2-b]-pyranyl, 5H-pyrido[2,3-d]-o-oxazinyl, 1H-pyrazolo[4,3-d]-oxazolyl, 4H-imidazo[4,5-d]thiazolyl, pyrazino[2,3-d]pyridazinyl, imidazo[2,1-b]thiazolyl, imidazo[1,2-b][1,2,4]triazinyl. "Heteroaryl" also covers partially aromatic bi- or polycyclic ring systems wherein at least one ring is an aromatic ring and one or more of the other ring(s) is a non-aromatic, saturated or partially saturated ring, provided at least one ring contains one or more heteroatoms selected from nitrogen, oxygen or sulfur. Examples of partially aromatic heteroaryl groups include for example, tetrahydroisoquinolinyl, tetrahydroquinolinyl, 2-oxo-1,2,3,4-tetrahydroquinolinyl, dihydrobenzthienyl, dihydrobenzfuranyl, 2,3-dihydro-benzo[1,4]dioxinyl, benzo[1,3]dioxolyl, 2,2-dioxo-1,3-dihydro-2-benzothienyl, 4,5,6,7-tetrahydrobenzofuranyl, indolinyl, 1,2,3,4-tetrahydro-1,8-naphthyridinyl, 1,2,3,4-tetrahydropyrido[2,3-*b*]pyrazinyl and 3,4-dihydro-2*H*-pyrido[3,2-*b*][1,4]oxazinyl.

Examples of five membered heteroaryl groups include but are not limited to pyrrolyl, furanyl, thienyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered heteroaryl groups include but are not limited to pyridyl, pyrazinyl, pyridazinyl, pyrimidinyl and triazinyl.

A bicyclic heteroaryl group may be, for example, a group selected from:
a benzene ring fused to a 5- or 6-membered ring containing 1, 2 or 3 ring heteroatoms;
a pyridine ring fused to a 5- or 6-membered ring containing 1, 2 or 3 ring heteroatoms;
a pyrimidine ring fused to a 5- or 6-membered ring containing 1 or 2 ring heteroatoms;
a pyrrole ring fused to a 5- or 6-membered ring containing 1, 2 or 3 ring heteroatoms;
a pyrazole ring fused to a 5- or 6-membered ring containing 1 or 2 ring heteroatoms;
a pyrazine ring fused to a 5- or 6-membered ring containing 1 or 2 ring heteroatoms;
an imidazole ring fused to a 5- or 6-membered ring containing 1 or 2 ring heteroatoms;
an oxazole ring fused to a 5- or 6-membered ring containing 1 or 2 ring heteroatoms;
an isoxazole ring fused to a 5- or 6-membered ring containing 1 or 2 ring heteroatoms;
a thiazole ring fused to a 5- or 6-membered ring containing 1 or 2 ring heteroatoms;
an isothiazole ring fused to a 5- or 6-membered ring containing 1 or 2 ring heteroatoms;
a thiophene ring fused to a 5- or 6-membered ring containing 1, 2 or 3 ring heteroatoms;
a furan ring fused to a 5- or 6-membered ring containing 1, 2 or 3 ring heteroatoms;
a cyclohexyl ring fused to a 5- or 6-membered heteroaromatic ring containing 1, 2 or 3 ring heteroatoms; and
a cyclopentyl ring fused to a 5- or 6-membered heteroaromatic ring containing 1, 2 or 3 ring heteroatoms.

Particular examples of bicyclic heteroaryl groups containing a six membered ring fused to a five membered ring include but are not limited to benzfuranyl, benzthiophenyl, benzimidazolyl, benzoxazolyl, benzisoxazolyl, benzthiazolyl, benzisothiazolyl, isobenzofuranyl, indolyl, isoindolyl, indolizinyl, indolinyl, isoindolinyl, purinyl (e.g., adeninyl, guaninyl), indazolyl, benzodioxolyl and pyrazolopyridinyl groups.

Particular examples of bicyclic heteroaryl groups containing two fused six membered rings include but are not limited to quinolinyl, isoquinolinyl, chromanyl, thiochromanyl, chromenyl, isochromenyl, chromanyl, isochromanyl, benzodioxanyl, quinolizinyl, benzoxazinyl, benzodiazinyl, pyridopyridinyl, quinoxalinyl, quinazolinyl, cinnolinyl, phthalazinyl, naphthyridinyl and pteridinyl groups.

"Heteroaryl(1-6C)alkyl" means a heteroaryl group covalently attached to a (1-6C)alkylene group, both of which are defined herein. Examples of heteroaralkyl groups include pyridin-3-ylmethyl, 3-(benzofuran-2-yl)propyl, and the like.

The term "aryl" means a cyclic or polycyclic aromatic ring having from 5 to 12 carbon atoms. The term aryl includes both monovalent species and divalent species. Examples of aryl groups include, but are not limited to, phenyl, biphenyl, naphthyl and the like. In particular embodiment, an aryl is phenyl.

The term "aryl(1-6C)alkyl" means an aryl group covalently attached to a (1-6C)alkylene group, both of which are defined herein. Examples of aryl-(1-6C)alkyl groups include benzyl, phenylethyl, and the like.

This specification also makes use of several composite terms to describe groups comprising more than one functionality. Such terms will be understood by a person skilled in the art. For example heterocyclyl(m-nC)alkyl comprises (m-nC)alkyl substituted by heterocyclyl.

The term "optionally substituted" refers to either groups, structures, or molecules that are substituted and those that are not substituted. The term "wherein a/any CH, CH₂, CH₃ group or heteroatom (i.e. NH) within a R¹ group is optionally substituted" suitably means that (any) one of the hydrogen radicals of the R¹ group is substituted by a relevant stipulated group.

Where optional substituents are chosen from "one or more" groups it is to be understood that this definition includes all substituents being chosen from one of the specified groups or the substituents being chosen from two or more of the specified groups.

The phrase "compound of the invention" means those compounds which are disclosed herein, both generically and specifically.

### Compounds of the invention

In one aspect, the present invention relates to a compound, or a pharmaceutically acceptable salt, hydrate or solvate thereof, having the structural Formula (I), shown below: 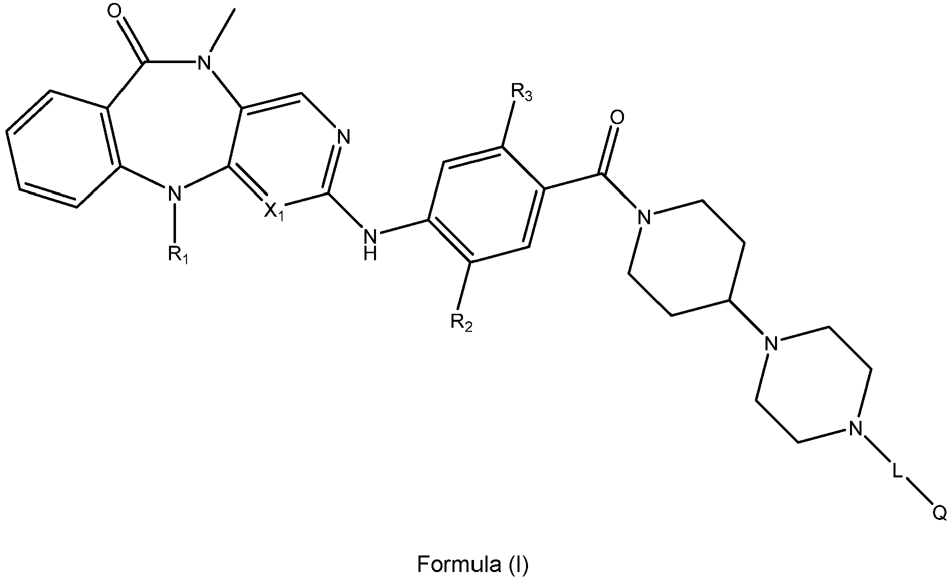 wherein
R₁ is -S(O)₂-(1-3C)alkyl;
R₂ is (1-4C)alkoxy;
R₃ is selected from hydrogen, fluoro, methyl, methoxy, trifluoromethyl, or trifluoromethoxy;
X₁ is N or CH;
L is a linker having the formula: 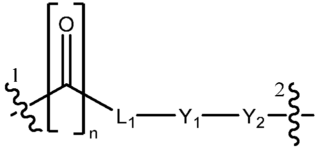 wherein: 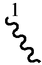
   denotes the point of attachment to the N atom of the piperazine ring; 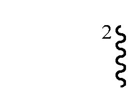
   denotes the point of attachment to Q;
   n is 0 or 1;
   L₁ is a (2-10C)alkylene linker or a PEG chain having the formula

      -[CH₂CH₂-o]₁₋₃-[CH₂]₁₋₃-;
   Y₁ and Y₂ together form:
      an ethynylene group (-C≡C-);
      an ethylene group (-CH=CH-);

         -CH₂-O-;

         -CH₂-NH-;

         or

         -CH₂-CH₂-;
Q is selected from:
   (i) 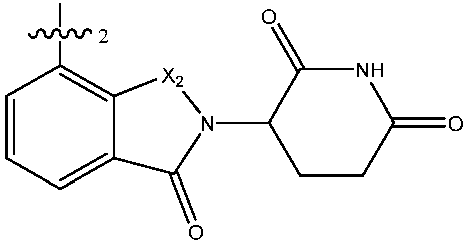
   (ii) 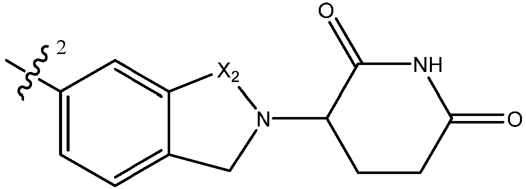
   (iii) 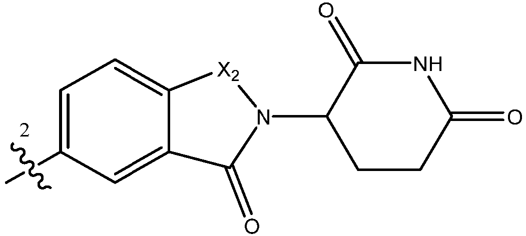 wherein: 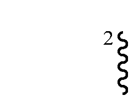
      denotes the point of attachment to Y₂ or group L;
      X₂ is selected from -CH₂- or -C(O)-; with the proviso that X₂ is not -C(O)- when Y₁and
      Y₂ together form an ethynylene group (-C≡C-).

Particular compounds of the invention include, for example, compounds of the Formula (I), or pharmaceutically acceptable salts and/or solvates thereof, wherein, unless otherwise stated, each of R₁, R₂, R₃, L, Q, and any associated substituent groups has any of the meanings defined hereinbefore or in any of paragraphs (1) to (55) hereinafter:-
(1) R₁ is -S(O)₂-CH₃.
(2) R₂ is (1-3C)alkoxy.
(3) R₂ is ethoxy.
(4) R₃ is selected from hydrogen, fluoro or methyl.
(5) R₃ is hydrogen.
(6) X₁ is N.
(7) L is a linker having the formula: 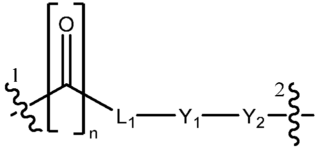 wherein: 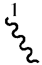
   denotes the point of attachment to the N atom of the piperazine ring; 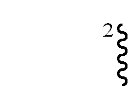
   denotes the point of attachment to Q;
   n is 0 or 1;
   L₁ is a (2-8C)alkylene linker or a PEG chain having the formula

      -[CH₂CH₂-O]₁₋₃-[CH₂]₁₋₂-;

      and
   Y₁ and Y₂ together form:
      an ethynylene group (-C≡C-);

      -CH₂-O-;

      -CH₂-NH-;

      or

      -CH₂-CH₂-.
(8) L is a linker having the formula: 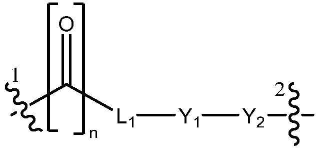 wherein: 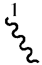
   denotes the point of attachment to the N atom of the piperazine ring; 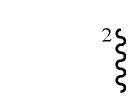
   denotes the point of attachment to Q;
   n is 0 or 1;
   L₁ is a (2-8C)alkylene linker or a PEG chain having the formula

      -[CH₂CH₂-O]₁₋₂-[CH₂]₁₋₂-;

      and
   Y₁ and Y₂ together form:
      an ethynylene group (-C≡C-);

      -CH₂-O-;

      -CH₂-NH-;

      or

      -CH₂-CH₂-.
(9) L is a linker having the formula: 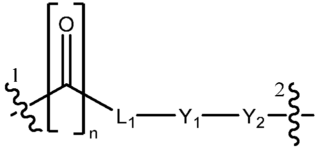 wherein: 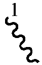
   denotes the point of attachment to the N atom of the piperazine ring; 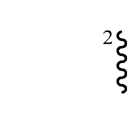
   denotes the point of attachment to Q;
   n is 0 or 1;
   L₁ is a (2-8C)alkylene linker; and
   Y₁ and Y₂ together form:
      an ethynylene group (-C≡C-);

      -CH₂-O-;

      -CH₂-NH-;

      or

      -CH₂-CH₂-.
(10) L is a linker having the formula: 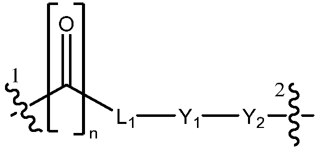 wherein: 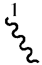
   denotes the point of attachment to the N atom of the piperazine ring; 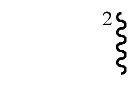
   denotes the point of attachment to Q;
   n is 0;
   L₁ is a (2-8C)alkylene linker or a PEG chain having the formula

      -[CH₂CH₂-O]₁₋₂-[CH₂]₁₋₂-;

      and
   Y₁ and Y₂ together form:
      an ethynylene group (-C≡C-);

      -CH₂-O-;

      -CH₂-NH-;

      or

      -CH₂-CH₂-.
(11) L is a linker having the formula: 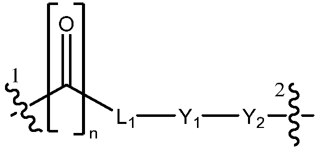 wherein: 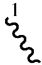
   denotes the point of attachment to the N atom of the piperazine ring; 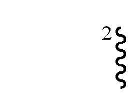
   denotes the point of attachment to Q;
   n is 0 or 1;
   L₁ is a (2-8C)alkylene linker or a PEG chain having the formula

      -[CH₂CH₂-O]₁₋₂-[CH₂]₁₋₂-;

      and
   Y₁ and Y₂ together form:
      an ethynylene group (-C≡C-).
(12) L is a linker having the formula: 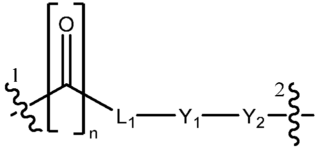 wherein: 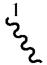
   denotes the point of attachment to the N atom of the piperazine ring; 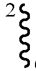
   denotes the point of attachment to Q;
   n is 0;
   L₁ is a (3-8C)alkylene linker; and
   Y₁ and Y₂ together form:
      an ethynylene group (-C≡C-);

      -CH₂-O-;

      -CH₂-NH-;

      or

      -CH₂-CH₂-.
(13) L is a linker having the formula: 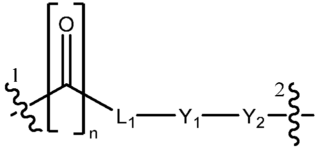 wherein: 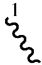
   denotes the point of attachment to the N atom of the piperazine ring; 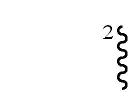
   denotes the point of attachment to Q;
   n is 0;
   L₁ is a (3-8C)alkylene linker; and
   Y₁ and Y₂ together form:
      an ethynylene group (-C≡C-).
(14) L is a linker having the formula: 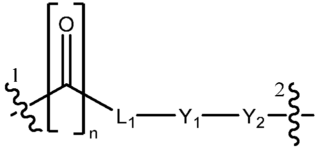 wherein: 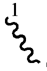
   denotes the point of attachment to the N atom of the piperazine ring; 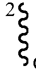
   denotes the point of attachment to Q;
   n is 0;
   L₁ is a (5-8C)alkylene linker; and
   Y₁ and Y₂ together form:
      an ethynylene group (-C≡C-).
(15) L is a linker having a formula selected from: 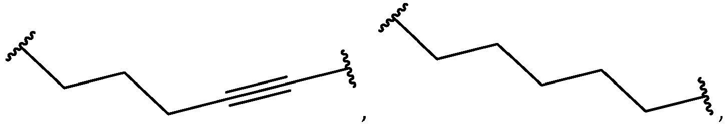 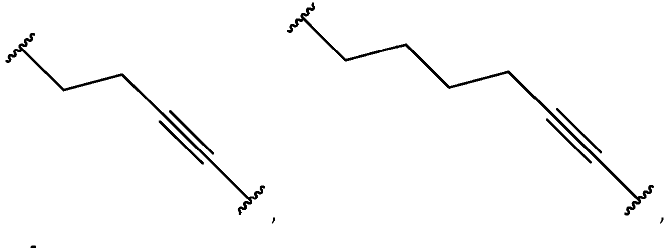 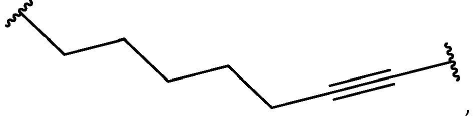 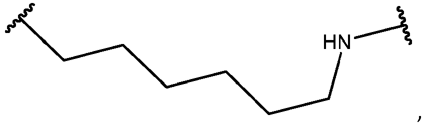 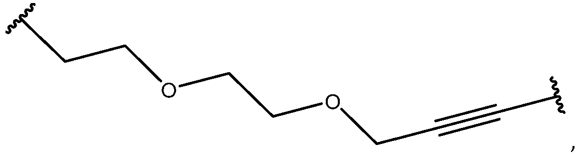 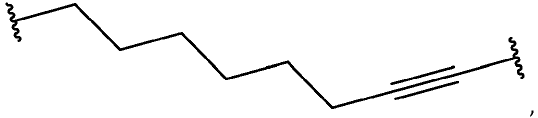 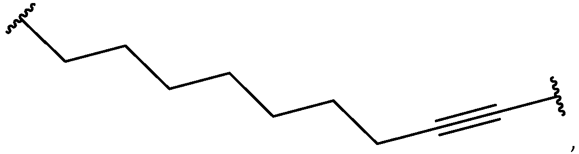 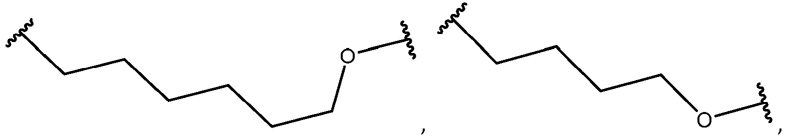 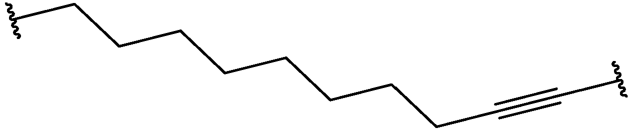 or 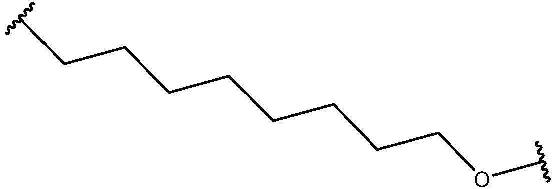
(16) Q is selected from:
   (i) 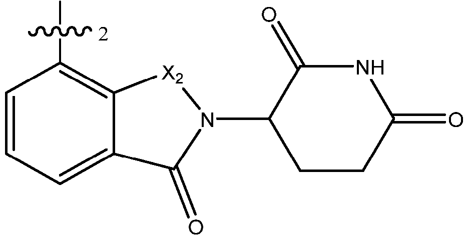 or 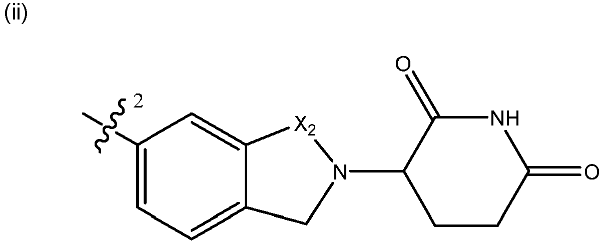 wherein X₂ is as defined herein.
(17) Q is: 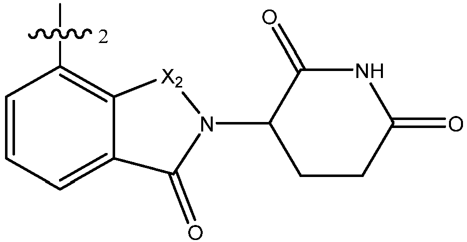 wherein X₂ is as defined herein.

Suitably, a heteroaryl or heterocyclyl group as defined herein is a monocyclic heteroaryl or heterocyclyl group comprising one, two or three heteroatoms selected from N, O or S.

Suitably, a heteroaryl is a 5- or 6-membered heteroaryl ring comprising one, two or three heteroatoms selected from N, O or S.

Suitably, a heterocyclyl group is a 4-, 5- or 6-membered heterocyclyl ring comprising one, two or three heteroatoms selected from N, O or S. Most suitably, a heterocyclyl group is a 5- or 6-membered ring comprising one, two or three heteroatoms selected from N, O or S [e.g. morpholinyl (e.g. 4-morpholinyl), oxetane, methyloxetane (e.g. 3-methyloxetane), pyrrolidinone (e.g. pyrrolidin-2-one)].

Suitably an aryl group is phenyl.

Suitably, R₁ is as defined in paragraph (1), i.e. R₁ is -S(O)₂-CH₃.

Suitably, R₂ is as defined as in paragraphs (2) and (3) above. Most suitably R₂ is as defined in paragraph (3), i.e. R₂ is ethoxy.

Suitably, R₃ is as defined as in paragraphs (4) and (5) above. Most suitably R₃ is as defined in paragraph (5), i.e. R₃ is hydrogen.

Suitably X₁ is as defined in paragraph (6), i.e. X₁ is N.

Suitably, L is as defined as in any one of paragraphs (7) to (15) above. More suitably, L is as defined as in any one of paragraphs (10) to (15). Most suitably, L is as defined in paragraph (14) or (15).

Suitably, Q is as defined in paragraph (16) or (17). Most suitably, Q is as defined in paragraph (17).

In a particular group of compounds of the invention, R₁ is as defined in paragraph (1), R₂ is as defined in paragraph (3), R₃ is as defined in paragraph (5) and X₁ is as defined in paragraph (6); i.e. the compounds have the structural formula (Ia) (a sub-definition of Formula (I)) shown below: 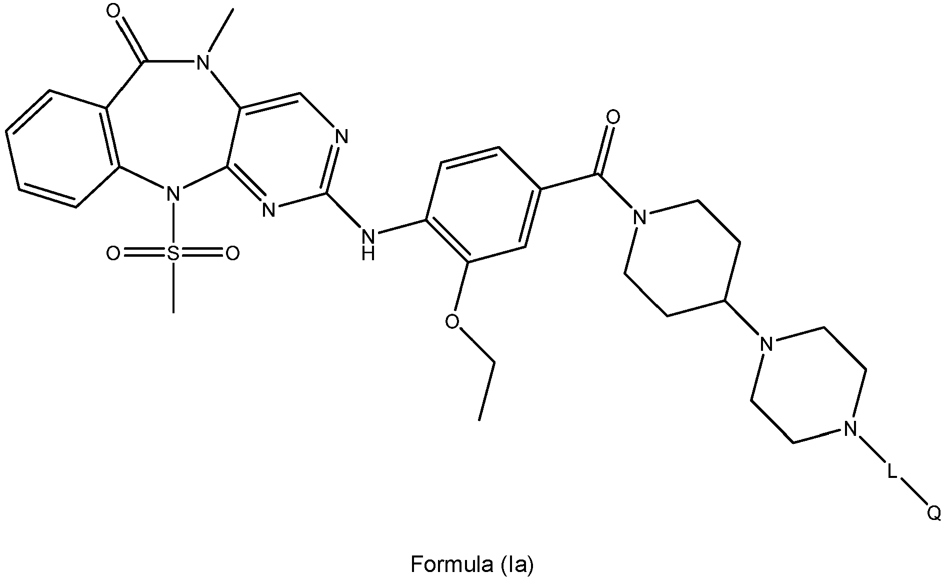 wherein L and Q are as defined herein.

In an embodiment of the compounds of formula (la):
L is as defined in any one of paragraphs (7) to (15) above;
Q is as defined in paragraph (16) or (17) above.

In an embodiment of the compounds of formula (la):
L is as defined in any one of paragraphs (10) to (15) above;
Q is as defined in paragraph (16) or (17) above.

In an embodiment of the compounds of formula (la):
L is as defined in any one of paragraphs (10) to (15) above;
Q is as defined in paragraph (17) above.

In an embodiment of the compounds of formula (la):
L is as defined in paragraph (14) or (15) above;
Q is as defined in paragraph (17) above.

Particular compounds of the present invention include any of the compounds exemplified in the present application, or a pharmaceutically acceptable salt or solvate thereof, and, in particular, any of the following:
2-(2,6-Dioxopiperidin-3-yl)-4-(5-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pentyl)isoindoline-1,3-dione;
3-(4-(5-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pent-1-yn-1-yl)-1-oxoisoindolin-2-yl)piperidine-2,6-dione;
3-(4-(4-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)but-1-yn-1-yl)-1-oxoisoindolin-2-yl)piperidine-2,6-dione;
3-(4-(6-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hex-1-yn-1-yl)-1-oxoisoindolin-2-yl)piperidine-2,6-dione;
3-(4-(7-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hept-1-yn-1-yl)-1-oxoisoindolin-2-yl)piperidine-2,6-dione;
2-(2,6-dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)amino)isoindoline-1,3-dione;
3-(4-(3-(2-(2-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)ethoxy)ethoxy)prop-1-yn-1-yl)-1-oxoisoindolin-2-yl)piperidine-2,6-dione;
3-(4-(8-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)oct-1-yn-1-yl)-1-oxoisoindolin-2-yl)piperidine-2,6-dione;
3-(4-(9-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)non-1-yn-1-yl)-1-oxoisoindolin-2-yl)piperidine-2,6-dione;
2-(2,6-Dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)oxy)isoindoline-1,3-dione;
2-(2,6-Dioxopiperidin-3-yl)-4-(4-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)butoxy)isoindoline-1,3-dione;
3-(4-(10-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)dec-1-yn-1-yl)-1-oxoisoindolin-2-yl)piperidine-2,6-dione;
2-(2,6-Dioxopiperidin-3-yl)-4-((8-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)octyl)oxy)isoindoline-1,3-dione;
or a pharmaceutically acceptable salt or solvate thereof.

The various functional groups and substituents making up the compounds of the Formula (I) are typically chosen such that the molecular weight of the compound of the Formula (I) does not exceed 1000. More usually, the molecular weight of the compound will be less than 900, for example less than 800, or less than 750, or less than 700, or less than 650. More preferably, the molecular weight is less than 600 and, for example, is 550 or less.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulfuric, phosphoric, trifluoroacetic, formic, citric methane sulfonate or maleic acid. In addition, a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a pharmaceutically acceptable cation, for example a salt with methylamine, dimethylamine, trimethylamine, piperidine, morpholine or tris-(2-hydroxyethyl)amine.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of this invention may possess one or more asymmetric centers; such compounds can therefore be produced as individual (R)- or (S)-stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the invention may have geometric isomeric centres (E- and Z- isomers). It is to be understood that the present invention encompasses all optical, diastereoisomers and geometric isomers and mixtures thereof that possess antiproliferative activity.

The present invention also encompasses compounds of the invention as defined herein which comprise one or more isotopic substitutions. For example, H may be in any isotopic form, including ¹H, ²H(D), and ³H (T); C may be in any isotopic form, including ¹²C, ¹³C, and ¹⁴C; and O may be in any isotopic form, including ¹⁶O and ¹⁸O; and the like.

It is also to be understood that certain compounds of the Formula (I) may exist in solvated as well as unsolvated forms such as, for example, hydrated forms. It is to be understood that the invention encompasses all such solvated forms that possess antiproliferative activity.

It is also to be understood that certain compounds of the Formula (I) may exhibit polymorphism, and that the invention encompasses all such forms that possess antiproliferative activity.

Compounds of the Formula (I) may exist in a number of different tautomeric forms and references to compounds of the Formula (I) include all such forms. For the avoidance of doubt, where a compound can exist in one of several tautomeric forms, and only one is specifically described or shown, all others are nevertheless embraced by Formula (I). Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

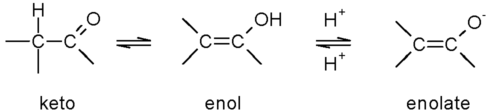

Compounds of the Formula (I) containing an amine function may also form N-oxides. A reference herein to a compound of the Formula (I) that contains an amine function also includes the N-oxide. Where a compound contains several amine functions, one or more than one nitrogen atom may be oxidised to form an N-oxide. Particular examples of N-oxides are the N-oxides of a tertiary amine or a nitrogen atom of a nitrogen-containing heterocycle. N-Oxides can be formed by treatment of the corresponding amine with an oxidizing agent such as hydrogen peroxide or a per-acid (e.g. a peroxycarboxylic acid), see for example Advanced Organic Chemistry, by Jerry March, 4th Edition, Wiley Interscience, pages. More particularly, N-oxides can be made by the procedure of L. W. Deady (Syn. Comm. 1977, 7, 509-514) in which the amine compound is reacted with *m*-chloroperoxybenzoic acid (*m*CPBA), for example, in an inert solvent such as dichloromethane.

Also disclosed herein, the compounds of Formula (I) may be administered in the form of a pro-drug which is broken down in the human or animal body to release a compound of the invention. A pro-drug may be used to alter the physical properties and/or the pharmacokinetic properties of a compound of the invention. A pro-drug can be formed when the compound of the invention contains a suitable group or substituent to which a property-modifying group can be attached. Examples of pro-drugs include *in vivo* cleavable ester derivatives that may be formed at a carboxy group or a hydroxy group in a compound of the Formula (I) and *in-vivo* cleavable amide derivatives that may be formed at a carboxy group or an amino group in a compound of the Formula (I).

Accordingly, the present invention includes those compounds of the Formula (I) as defined hereinbefore when made available by organic synthesis and when made available within the human or animal body by way of cleavage of a pro-drug thereof. Accordingly, the present invention includes those compounds of the Formula (I) that are produced by organic synthetic means and also such compounds that are produced in the human or animal body by way of metabolism of a precursor compound, that is a compound of the Formula (I) may be a synthetically-produced compound or a metabolically-produced compound.

A suitable pharmaceutically acceptable pro-drug of a compound of the Formula (I) is one that is based on reasonable medical judgement as being suitable for administration to the human or animal body without undesirable pharmacological activities and without undue toxicity.

Various forms of pro-drug have been described, for example in the following documents :-
a) Methods in Enzymology, Vol. 42, p. 309-396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988);
f) N. Kakeya, et al., Chem. Pharm. Bull., 32, 692 (1984);
g) T. Higuchi and V. Stella, "Pro-Drugs as Novel Delivery Systems", A.C.S. Symposium Series, Volume 14; and
h) E. Roche (editor), "Bioreversible Carriers in Drug Design", Pergamon Press, 1987.

A suitable pharmaceutically acceptable pro-drug of a compound of the Formula (I) that possesses a carboxy group is, for example, an *in vivo* cleavable ester thereof. An *in vivo* cleavable ester of a compound of the Formula (I) containing a carboxy group is, for example, a pharmaceutically acceptable ester which is cleaved in the human or animal body to produce the parent acid. Suitable pharmaceutically acceptable esters for carboxy include C₁₋₆alkyl esters such as methyl, ethyl and *tert-*butyl*,* C₁₋₆alkoxymethyl esters such as methoxymethyl esters, C₁₋₆alkanoyloxymethyl esters such as pivaloyloxymethyl esters, 3-phthalidyl esters, C₃₋₈cycloalkylcarbonyloxy- C₁₋₆alkyl esters such as cyclopentylcarbonyloxymethyl and 1-cyclohexylcarbonyloxyethyl esters, 2-oxo-1,3-dioxolenylmethyl esters such as 5-methyl-2-oxo-1,3-dioxolen-4-ylmethyl esters and C₁₋₆alkoxycarbonyloxy- C₁₋₆alkyl esters such as methoxycarbonyloxymethyl and 1-methoxycarbonyloxyethyl esters.

A suitable pharmaceutically acceptable pro-drug of a compound of the Formula (I) that possesses a hydroxy group is, for example, an *in vivo* cleavable ester or ether thereof. An *in vivo* cleavable ester or ether of a compound of the Formula (I) containing a hydroxy group is, for example, a pharmaceutically acceptable ester or ether which is cleaved in the human or animal body to produce the parent hydroxy compound. Suitable pharmaceutically acceptable ester forming groups for a hydroxy group include inorganic esters such as phosphate esters (including phosphoramidic cyclic esters). Further suitable pharmaceutically acceptable ester forming groups for a hydroxy group include C₁₋₁₀alkanoyl groups such as acetyl, benzoyl, phenylacetyl and substituted benzoyl and phenylacetyl groups, C₁₋₁₀alkoxycarbonyl groups such as ethoxycarbonyl, *N,N* -(C₁₋₆)₂carbamoyl, 2-dialkylaminoacetyl and 2-carboxyacetyl groups. Examples of ring substituents on the phenylacetyl and benzoyl groups include aminomethyl, *N*-alkylaminomethyl, *N,N*-dialkylaminomethyl, morpholinomethyl, piperazin-1-ylmethyl and 4-(C₁₋₄alkyl)piperazin-1-ylmethyl. Suitable pharmaceutically acceptable ether forming groups for a hydroxy group include α-acyloxyalkyl groups such as acetoxymethyl and pivaloyloxymethyl groups.

A suitable pharmaceutically acceptable pro-drug of a compound of the Formula (I) that possesses a carboxy group is, for example, an *in vivo* cleavable amide thereof, for example an amide formed with an amine such as ammonia, a C₁₋₄alkylamine such as methylamine, a (C₁₋₄alkyl)₂amine such as dimethylamine, *N*-ethyl-*N*-methylamine or diethylamine, a C₁₋₄alkoxy- C₂₋₄alkylamine such as 2-methoxyethylamine, a phenyl-C₁₋₄alkylamine such as benzylamine and amino acids such as glycine or an ester thereof.

A suitable pharmaceutically acceptable pro-drug of a compound of the Formula (I) that possesses an amino group is, for example, an *in vivo* cleavable amide derivative thereof. Suitable pharmaceutically acceptable amides from an amino group include, for example an amide formed with C₁₋₁₀alkanoyl groups such as an acetyl, benzoyl, phenylacetyl and substituted benzoyl and phenylacetyl groups. Examples of ring substituents on the phenylacetyl and benzoyl groups include aminomethyl, *N*-alkylaminomethyl, *N,N-*dialkylaminomethyl, morpholinomethyl, piperazin-1-ylmethyl and 4-(C₁₋₄alkyl)piperazin-1-ylmethyl.

The *in vivo* effects of a compound of the Formula (I) may be exerted in part by one or more metabolites that are formed within the human or animal body after administration of a compound of the Formula (I). As stated hereinbefore, the *in vivo* effects of a compound of the Formula (I) may also be exerted by way of metabolism of a precursor compound (a pro-drug).

Though the present invention may relate to any compound or particular group of compounds defined herein by way of optional, preferred or suitable features or otherwise in terms of particular embodiments, the present invention may also relate to any compound or particular group of compounds that specifically excludes said optional, preferred or suitable features or particular embodiments.

Suitably, the present invention excludes any individual compounds not possessing the biological activity defined herein.

### Synthesis

The compounds of the present invention can be prepared by any suitable technique known in the art. Particular processes for the preparation of these compounds are described further in the accompanying examples.

In the description of the synthetic methods described herein and in any referenced synthetic methods that are used to prepare the starting materials, it is to be understood that all proposed reaction conditions, including choice of solvent, reaction atmosphere, reaction temperature, duration of the experiment and workup procedures, can be selected by a person skilled in the art.

It is understood by one skilled in the art of organic synthesis that the functionality present on various portions of the molecule must be compatible with the reagents and reaction conditions utilised.

It will be appreciated that during the synthesis of the compounds of the invention in the processes defined herein, or during the synthesis of certain starting materials, it may be desirable to protect certain substituent groups to prevent their undesired reaction. The skilled chemist will appreciate when such protection is required, and how such protecting groups may be put in place, and later removed.

For examples of protecting groups see one of the many general texts on the subject, for example, 'Protective Groups in Organic Synthesis' by Theodora Green (publisher: John Wiley & Sons). Protecting groups may be removed by any convenient method described in the literature or known to the skilled chemist as appropriate for the removal of the protecting group in question, such methods being chosen so as to effect removal of the protecting group with the minimum disturbance of groups elsewhere in the molecule.

Thus, if reactants include, for example, groups such as amino, carboxy or hydroxy it may be desirable to protect the group in some of the reactions mentioned herein.

By way of example, a suitable protecting group for an amino or alkylamino group is, for example, an acyl group, for example an alkanoyl group such as acetyl, an alkoxycarbonyl group, for example a methoxycarbonyl, ethoxycarbonyl or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively an acyl group such as a tert-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium, sodium hydroxide or ammonia. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a t-butyl group which may be removed, for example, by treatment with an acid, for example an organic acid such as trifluoroacetic acid, or for example a benzyl group which may be removed, for example, by hydrogenation over a catalyst such as palladium-on-carbon.

Resins may also be used as a protecting group.

The methodology employed to synthesise a compound of formula (I) will vary depending on the nature of R₁, R₂, R₃ X₁, L and Q, and any substituent groups associated therewith. Suitable processes for their preparation are described further in the accompanying Examples.

Once a compound of Formula (I) has been synthesised by any one of the processes defined herein, the processes may then further comprise the additional steps of:
(i) removing any protecting groups present;
(ii) converting the compound Formula (I) into another compound of Formula (I);
(iii) forming a pharmaceutically acceptable salt, hydrate or solvate thereof; and/or
(iv) forming a prodrug thereof.

An example of (ii) above is when a compound of Formula (I) is synthesised and then one or more of the groups of R₁, R₂, R₃ X₁, L and Q, may be further reacted to change the nature of the group and provide an alternative compound of Formula (I). For example, the compound can be reacted to covert R₃ into a substituent group other than hydrogen.

The resultant compounds of Formula (I) can be isolated and purified using techniques well known in the art.

### Biological Activity

The assays described in accompanying Biological Example section may be used to measure the pharmacological effects of the compounds of the present invention. In reference to the accompanying Figures:
Figure 1 shows western blot data demonstrating the ERK5 levels in MDA-MB-468s at different concentrations of OS1. Graphs show quantification of protein levels relative to β-actin. The left hand graph shows ERK5 and the right hand graph shows BRD4 levels at different concentrations of OS1.
Figure 2 shows ERK5 western blot and graphical quantification of ERK5 levels relative to β-actin in MDA_MB-468s at different concentrations of CE207, OS3 and OS4.
Figure 3 shows ERK5 western blot and graphical quantification of ERK5 levels relative to β-actin in MDA-MB-468s at different concentrations of OS6 and OS7.
Figure 4 shows ERK5 western blot and graphical quantification of ERK5 levels relative to β-actin in MDA-MB-468s at different concentrations of OS9, OS10 and OS11.
Figure 5 shows ERK5 western blot and graphical quantification of ERK5 levels relative to β-actin in MDA_MB-468s at different concentrations of OS12, OS13 and OS14.

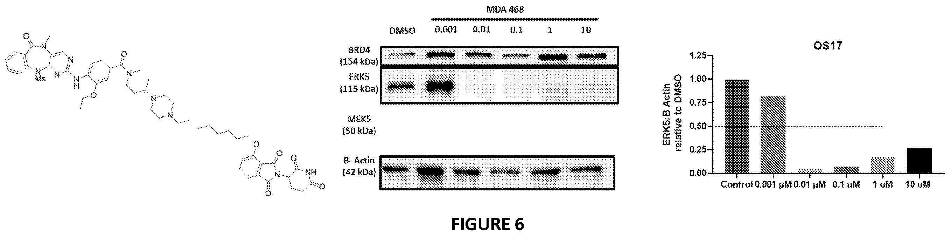

Figure 6 shows western blots for BRD4, ERK5 and β-actin in triple-negative breast cancer cell lines MDA-MB-468, in response to different concentrations of OS17.
Relative protein levels of ERK5 normalised to β-actin, are graphically represented in the right hand side graphs.

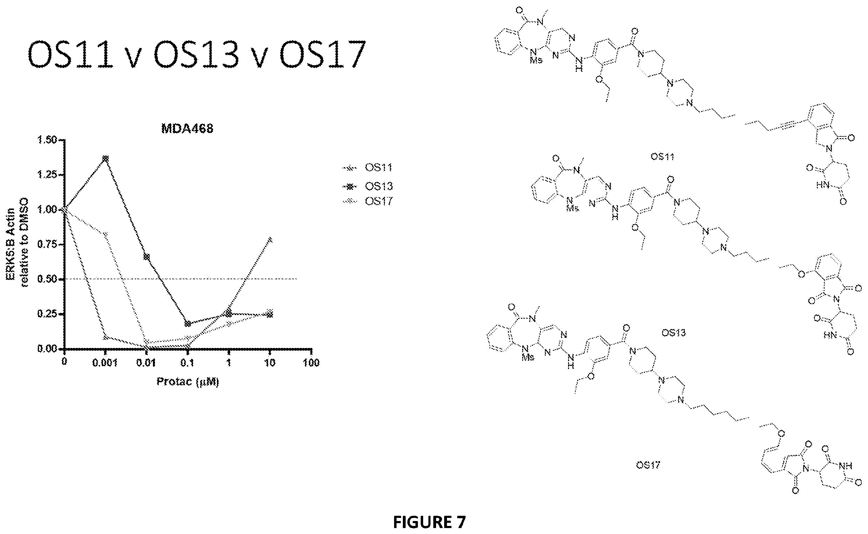

Figure 7 shows the relative protein levels of ERK5 relative to β-actin, at different concentrations of OS11, OS13 and OS 17.
Figure 8 shows western blots for ERK5, the phosphorylated form of ERK5, the upstream activator of ERK5, MEK5 as well as BRD4, ERK1/2 and β-actin in three triple-negative breast cancer cell lines: MDA-MB-231, MDA-MB-468 and SUM159, in response to different concentrations of OS1. Relative protein levels, normalised to β-actin, are graphically represented in the right hand side graphs.
Figure 9 shows western blots for ERK5, BRD4, MEK5 and β-actin in three triple-negative breast cancer cell lines: MDA-MB-231, MDA-MB-468 and SUM159 in response to either 1µM OS1 or 2.5µM AX15836 (ERK5 kinase inhibitor 5,11-Dihydro-2-[[2-ethoxy-4-[[4-(4-methyl-1-piperazinyl)-1-piperidinyl]carbonyl]phenyl]amino]-5-methyl,11-(methylsulfonyl)-6*H*-pyrimido[4,5-*b*][1,4]benzodiazepin-6-one) shows the duration of the effect of a single does of OS1 and the effect of repeated addition of OS1, every 48h, for up to 2 weeks. The ERK5 kinase inhibitor AX15836 is used as comparison.
Figure 10 shows immunofluorescence images of the the effect of 1µM OS1 or 2.5µM AX15836 on ERK5 levels and the morphology of three triple-negative breast cancer cell lines: MDA-MB-231, MDA-MB-468 and SUM159.
Figure 11 shows relative mRNA levels of EMT, angiogenesis and inflammatory regulators in response to either 0.1 or 1µM OS1 treatment for 24h in three triple-negative breast cancer cell lines: MDA-MB-231, MDA-MB-468 and SUM159. Results are normalised to the internal loading controls PGK1 or ACTB.
Figures 12 and 13 show a comparison of OS1 and OS11, respectively, to Example 7 of WO2021061894.
Figure 14.A shows the effective knockdown of ERK5 by OS11 *in vivo* both in the tumour compartment and in PBMCs. Figure 14.B shows that OS11 has an anti-tumour effect equal to that of PD1-inhibition and that combinatorial treatment of OS11 with PD1 inhibition has no further effect beyond that of either agent alone.

Although the pharmacological properties of the compounds of Formula (I) vary with structural change, as expected, the compounds of the invention were found to be active in these assays.

### Pharmaceutical Compositions

According to a further aspect of the invention there is provided a pharmaceutical composition which comprises a compound of the invention as defined hereinbefore, or a pharmaceutically acceptable salt, hydrate or solvate thereof, in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

An effective amount of a compound of the present invention for use in therapy is an amount sufficient to treat or prevent a proliferative condition referred to herein, slow its progression and/or reduce the symptoms associated with the condition.

The amount of active ingredient that is combined with one or more excipients to produce a single dosage form will necessarily vary depending upon the individual treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula (I) will naturally vary according to the nature and severity of the conditions, the age and sex of the animal or patient and the route of administration, according to well known principles of medicine.

In using a compound of the invention for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a compound of this invention.

### Therapeutic Uses and Applications

The present invention provides compounds that function as degraders of ERK5.

Also disclosed herein is a method of degrading ERK5 enzyme activity *in vitro* or *in vivo,* said method comprising contacting a cell with an effective amount of a compound, or a pharmaceutically acceptable salt, hydrate or solvate thereof, as defined herein.

Also disclosed herein is a method of treating a disease or disorder in which ERK5 activity is implicated in a patient in need of such treatment, said method comprising administering to said patient a therapeutically effective amount of a compound, or a pharmaceutically acceptable salt, hydrate or solvate thereof, or a pharmaceutical composition as defined herein.

Also disclosed herein is a method of inhibiting cell proliferation, *in vitro* or *in vivo,* said method comprising contacting a cell with an effective amount of a compound, or a pharmaceutically acceptable salt, hydrate or solvate thereof, as defined herein.

Also disclosed herein is a method of treating cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin- sensitive rhinosinusitis, cystic fibrosis, multiple sclerosis, kidney diseases, diabetes, pain, fibrotic diseases, for example impaired wound healing and pulmonary fibrosis; and cancer pain.

Also disclosed herein is a method of treating:
a) a fibrotic disease, e.g. impaired wound healing, pulmonary fibrosis,
b) an Inflammatory disease e.g. psoriasis, asthma,
c) a proliferative condition, e.g. cancers such as triple-negative breast cancer, mesothelioma, primary bone cancer, glioblastoma, lung cancer, squamous cell carcinoma,
d) diabetes,
e) pain, or
f) a central nervous system (CNS) disorder e.g. Parkinsons disease, dementia;
in a patient in need of such treatment, said method comprising administering to said patient a therapeutically effective amount of a compound, or a pharmaceutically acceptable salt, hydrate or solvate thereof, or a pharmaceutical composition as defined herein.

Also disclosed herein is a method of treating a proliferative disorder in a patient in need of such treatment, said method comprising administering to said patient a therapeutically effective amount of a compound, or a pharmaceutically acceptable salt, hydrate or solvate thereof, or a pharmaceutical composition as defined herein.

Also disclosed herein is a method of treating cancer in a patient in need of such treatment, said method comprising administering to said patient a therapeutically effective amount of a compound, or a pharmaceutically acceptable salt, hydrate or solvate thereof, or a pharmaceutical composition as defined herein. Suitably, the cancer is selected from triple-negative breast cancer, mesothelioma, primary bone cancer, glioblastoma, lung cancer or squamous cell carcinoma.

Also disclosed herein is a method of treating a central nervous system (CNS) disorder in a patient in need of such treatment, said method comprising administering to said patient a therapeutically effective amount of a compound, or a pharmaceutically acceptable salt, hydrate or solvate thereof, or a pharmaceutical composition as defined herein. Suitably, the central nervous system (CNS) disorder is Parkinsons disease or dementia.

The present invention provides a compound, or a pharmaceutically acceptable salt, hydrate or solvate thereof, or a pharmaceutical composition as defined herein for use in therapy.

The present invention provides a compound, or a pharmaceutically acceptable salt, hydrate or solvate thereof, or a pharmaceutical composition as defined herein for use in the treatment of a proliferative condition.

The present invention provides a compound as defined herein, or a pharmaceutically acceptable salt or solvate thereof, or a pharmaceutical composition as defined herein, for use in the treatment of cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin-sensitive rhinosinusitis, cystic fibrosis, multiple sclerosis, kidney diseases, diabetes, pain, fibrotic diseases, for example impaired wound healing and pulmonary fibrosis; and cancer pain.

The present invention provides a compound, or a pharmaceutically acceptable salt, hydrate or solvate thereof, or a pharmaceutical composition as defined herein for use in the treatment of:
a) a fibrotic disease, e.g. impaired wound healing, pulmonary fibrosis,
b) an Inflammatory disease e.g. psoriasis, asthma,
c) a proliferative condition, e.g. cancers such as triple-negative breast cancer, mesothelioma, primary bone cancer, glioblastoma, lung cancer, squamous cell carcinoma,
d) diabetes,
e) pain, or
f) a central nervous system (CNS) disorder e.g. Parkinsons disease, dementia.

The present invention provides a compound, or a pharmaceutically acceptable salt, hydrate or solvate thereof, or a pharmaceutical composition as defined herein for use in the treatment of cancer. In a particular embodiment, the cancer is human cancer. In a particular embodiment, the cancer is selected from triple-negative breast cancer, mesothelioma, primary bone cancer, glioblastoma, lung cancer or squamous cell carcinoma.

The present invention provides a compound as defined herein, or a pharmaceutically acceptable salt or solvate thereof, or a pharmaceutical composition as defined herein, for use in the treatment of a central nervous system (CNS) disorder. In a particular embodiment, the central nervous system (CNS) disorder is Parkinsons disease or dementia.

The present invention provides a compound, or a pharmaceutically acceptable salt, hydrate or solvate thereof, as defined herein for use in the degreadation of ERK5 enzyme activity.

The present invention provides a compound, or a pharmaceutically acceptable salt, hydrate or solvate thereof, as defined herein, for use in the treatment of a disease or disorder in which ERK5 activity is implicated.

Diseases or disorders in which ERK5 is implicated include cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin- sensitive rhinosinusitis, cystic fibrosis, multiple sclerosis, kidney diseases, diabetes, pain, fibrotic diseases, for example impaired wound healing and pulmonary fibrosis; and cancer pain.

Also disclosed herein is a use of a compound, or a pharmaceutically acceptable salt, hydrate or solvate thereof, as defined herein in the manufacture of a medicament for the treatment of a proliferative condition.

Also disclosed herein is a use of a compound, or a pharmaceutically acceptable salt, hydrate or solvate thereof, as defined herein in the manufacture of a medicament for the treatment of cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin-sensitive rhinosinusitis, cystic fibrosis, multiple sclerosis, kidney diseases, diabetes, pain, fibrotic diseases, for example impaired wound healing and pulmonary fibrosis; and cancer pain.

Also disclosed herein is a use of a compound, or a pharmaceutically acceptable salt, hydrate or solvate thereof, as defined herein in the manufacture of a medicament for the treatment of:
a) a fibrotic disease, e.g. impaired wound healing, pulmonary fibrosis,
b) an inflammatory disease e.g. psoriasis, asthma,
c) a proliferative condition, e.g. cancers such as triple-negative breast cancer, mesothelioma, primary bone cancer, glioblastoma, lung cancer, squamous cell carcinoma,
d) diabetes,
e) pain, or
f) a central nervous system (CNS) disorder e.g. Parkinsons disease, dementia.

Also disclosed herein is a use of a compound, or a pharmaceutically acceptable salt, hydrate or solvate thereof, as defined herein in the manufacture of a medicament for the treatment of cancer. Suitably, the medicament is for use in the treatment of human cancers. More suitably, the cancer is selected from triple-negative breast cancer, mesothelioma, primary bone cancer, glioblastoma, lung cancer or squamous cell carcinoma.

Also disclosed herein is a use of a compound, or a pharmaceutically acceptable salt, hydrate or solvate thereof, as defined herein in the manufacture of a medicament for the treatment of a central nervous system (CNS) disorder. Suitably, the medicament is for use in the treatment of Parkinsons disease or dementia.

Also disclosed herein is a use of a compound, or a pharmaceutically acceptable salt, hydrate or solvate thereof, as defined herein in the manufacture of a medicament for the reduction of ERK5 enzyme activity.

Also disclosed herein is a use of a compound, or a pharmaceutically acceptable salt, hydrate or solvate thereof, as defined herein in the manufacture of a medicament for the treatment of a disease or disorder in which ERK5 activity is implicated.

The term "proliferative disorder" are used interchangeably herein and pertain to an unwanted or uncontrolled cellular proliferation of excessive or abnormal cells which is undesired, such as, neoplastic or hyperplastic growth, whether *in vitro* or *in vivo.* Examples of proliferative conditions include, but are not limited to, pre-malignant and malignant cellular proliferation, including but not limited to, malignant neoplasms and tumours, cancers, leukemias, psoriasis, bone diseases, fibroproliferative disorders (e.g., of connective tissues), and atherosclerosis. Any type of cell may be treated, including but not limited to, lung, colon, breast, ovarian, prostate, liver, pancreas, brain, and skin.

The anti-proliferative effects of the compounds of the present invention have particular application in the treatment of human cancers (by virtue of their reduction of ERK5 enzyme activity via degradation of the ERK5 enzyme).

The anti-cancer effect may arise through one or more mechanisms, including but not limited to, the regulation of cell proliferation, the inhibition of angiogenesis (the formation of new blood vessels), the inhibition of metastasis (the spread of a tumour from its origin), the inhibition of invasion (the spread of tumour cells into neighbouring normal structures), or the promotion of apoptosis (programmed cell death).

In a particular embodiment of the invention, the proliferative condition to be treated is cancer. In a particular embodiment, the cancer is selected from triple-negative breast cancer, mesothelioma, primary bone cancer, glioblastoma, lung cancer or squamous cell carcinoma.

In a particular embodiment of the invention, the fibrotic disease to be treated is impaired wound healing or pulmonary fibrosis.

In a particular embodiment of the invention, the inflammatory disease to be treated is psoriasis or asthma.

In a particular embodiment of the invention, the central nervous system (CNS) disorder to be treated is Parkinsons disease or dementia.

In a particular embodiment of the invention, the disease or disorder to be treated is selected from cancer for example triple-negative breast cancer, mesothelioma, primary bone cancer, glioblastoma, lung cancer and squamous cell carcinoma; inflammatory diseases, for example systemic lupus erythematosus, autoimmune mediated glomerulonephritis, rheumatoid arthritis, psoriasis, inflammatory bowel disease, autoimmune diabetes mellitus, Eczema hypersensitivity reactions, asthma, COPD, rhinitis, and upper respiratory tract disease; cardiovascular diseases for example cardiac hypertrophy, restenosis, atherosclerosis; neurodegenerative disorders, for example Alzheimer's disease, dementia, AIDS-related dementia, Parkinson's disease, amyotropic lateral sclerosis, retinitis pigmentosa, spinal muscular atropy and cerebellar degeneration; glomerulonephritis; myelodysplasia syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin-sensitive rhinosinusitis, cystic fibrosis, multiple sclerosis, kidney diseases, diabetes, pain, fibrotic diseases, for example impaired wound healing and pulmonary fibrosis; and cancer pain.

### Routes of Administration

The compounds of the invention or pharmaceutical compositions comprising these compounds may be administered to a subject by any convenient route of administration, whether systemically/ peripherally or topically (i.e., at the site of desired action).

Routes of administration include, but are not limited to, oral (e.g, by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

### Combination Therapies

The anti proliferative treatment defined hereinbefore may be applied as a sole therapy or may involve, in addition to the compound of the invention, conventional surgery or radiotherapy or chemotherapy. Such chemotherapy may include one or more of the following categories of anti-tumour agents:-
(i) other antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) anti-invasion agents [for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline (AZD0530; International Patent Application WO 01/94341), N-(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825; J. Med. Chem., 2004, 47, 6658-6661) and bosutinib (SKI-606), and metalloproteinase inhibitors like marimastat, inhibitors of urokinase plasminogen activator receptor function or antibodies to Heparanase];
(iv) inhibitors of growth factor function: for example such inhibitors include growth factor antibodies and growth factor receptor antibodies (for example the anti-erbB2 antibody trastuzumab [Herceptin^{™}], the anti-EGFR antibody panitumumab, the anti-erbB1 antibody cetuximab [Erbitux, C225] and any growth factor or growth factor receptor antibodies disclosed by Stern et al. (Critical reviews in oncology/haematology, 2005, Vol. 54, pp11-29); such inhibitors also include tyrosine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, ZD1839), *N-*(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)-quinazolin-4-amine (Cl 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib (BAY 43-9006), tipifarnib (R115777) and lonafarnib (SCH66336)), inhibitors of cell signalling through MEK and/or AKT kinases, c-kit inhibitors, abl kinase inhibitors, PI3 kinase inhibitors, PIt3 kinase inhibitors, CSF-1R kinase inhibitors, IGF receptor (insulin-like growth factor) kinase inhibitors; aurora kinase inhibitors (for example AZD1152, PH739358, VX-680, MLN8054, R763, MP235, MP529, VX-528 AND AX39459) and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin^{™}) and for example, a VEGF receptor tyrosine kinase inhibitor such as vandetanib (ZD6474), vatalanib (PTK787), sunitinib (SU11248), axitinib (AG-013736), pazopanib (GW 786034) and 4-(4-fluoro-2-methylindol-5-yloxy)-6-methoxy-7-(3-pyrrolidin-1-ylpropoxy)quinazoline (AZD2171; Example 240 within WO 00/47212), compounds such as those disclosed in International Patent Applications WO97/22596, WO 97/30035, WO 97/32856 and WO 98/13354 and compounds that work by other mechanisms (for example linomide, inhibitors of integrin αvβ3 function and angiostatin)];
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) an endothelin receptor antagonist, for example zibotentan (ZD4054) or atrasentan;
(viii) antisense therapies, for example those which are directed to the targets listed above, such as ISIS 2503, an anti-ras antisense;
(ix) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(x) immunotherapy approaches, including for example *ex-vivo* and *in-vivo* approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In a particular embodiment, the anti proliferative treatment defined hereinbefore may involve, in addition to the compound of the invention, conventional surgery or radiotherapy or chemotherapy.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a combination for use in the treatment of a cancer (for example a cancer involving a solid tumour) comprising a compound of the invention as defined hereinbefore, or a pharmaceutically acceptable salt, hydrate or solvate thereof, and another anti-tumour agent.

According to this aspect of the invention there is provided a combination for use in the treatment of a proliferative condition, such as cancer (for example a cancer involving a solid tumour), comprising a compound of the invention as defined hereinbefore, or a pharmaceutically acceptable salt, hydrate or solvate thereof, and any one of the anti-tumour agents listed herein above.

In a further aspect of the invention there is provided a compound of the invention or a pharmaceutically acceptable salt, hydrate or solvate thereof, for use in the treatment of cancer in combination with another anti-tumour agent, optionally selected from one listed herein above.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

According to a further aspect of the invention there is provided a pharmaceutical composition which comprises a compound of the invention, or a pharmaceutically acceptable salt, hydrate or solvate thereof, in combination with an anti-tumour agent (optionally selected from one listed herein above), in association with a pharmaceutically acceptable diluent or carrier.

### EXAMPLES

### Chemical Synthesis

### 4-Bromo-2-(2,6-dioxopiperidin-3-yl)isoindoline-1,3-dione

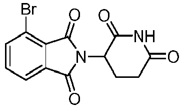

3-Bromophthalic anhydride (840 mg and 3.70 mmol), 3-aminopiperidine-2,6-dione hydrochloride (667 mg and 4.07 mmol) and sodium acetate (364 mg and 4.44 mmol) were suspended in (glacial) acetic acid (30 mL) and heated at 140 °C overnight . Reaction was cooled to 25 °C, concentrated *in vacuo* and water (30 mL) was added the crude. The solid was filtered off to give 4-bromo-2-(2,6-dioxopiperidin-3-yl)isoindoline-1,3-dione as off white solid (730 mg, 59 %).¹ mp: > 300°C. ¹H NMR (400 MHz, DMSO-*d*₆) δ 11.16 (s, 1H), 8.07 (d, *J* = 8.2 Hz, 1H), 7.94 (d, *J* = 7.3 Hz, 1H), 7.79 (*app.* t*, J* = 7.7 Hz, 1H), 5.18 (dd, *J* = 12.8, 5.4 Hz, 1H), 2.95-2.86 (m, 1H,), 2.66 - 2.57 (m, 2H), 2.11-2.05 (m, 1H). *m*/*z* (ES⁺) = 336.7.

### ((Pent-4-yn-1-yloxy)methyl)benzene

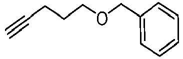

NaH (1.9 g, 47.6 mmol) was suspended in THF (95 mL), 4-pentynol (2.0 g, 23.8 mmol) was added dropwise at 0 °C. Benzyl bromide (2.6 mL, 21.9 mmol) was then added dropwise and allowed to stir overnight. Sat. aq. ammonium chloride solution (50 mL) was added and the mixture extracted with EtOAc (3 × 50 mL), the organics were dried with MgSO₄ and concentrated *in vacuo.* The crude was purified via flash column chromatography (20:1, Hexane:EtOAc) the clean fractions were concentrated *in vacuo* to give ((pent-4-yn-1-yloxy)methyl)benzene as a yellow oil (2.0 g, 52%). ¹H NMR (400 MHz, Chloroform-d) δ 7.32 - 7.07 (m, 5H), 4.42 (s, 2H), 3.49 (t, *J* = 6.2 Hz, 2H), 2.23 (td, *J* = 7.1, 2.6 Hz, 2H), 1.85 (t, *J* = 2.6 Hz, 1H), 1.78 - 1.69 (m, 2H).

### 4-(5-(Benzyloxy)pent-1-yn-1-yl)-2-(2,6-dioxopiperidin-3-yl)isoindoline-1,3-dione

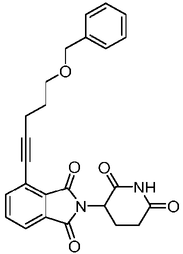

4-Bromo-2-(2,6-dioxopiperidin-3-yl)isoindoline-1,3-dione (1000 mg and 2.97 mmol), pent-4-yn-1-yloxy)methyl)benzene (1030 mg and 5.95 mmol), copper iodide (66 mg and 0.35 mmol) and Pd(PPh₃)₂Cl₂ (126 mg and 0.18 mmol) were suspended in DMF (10 mL). The reaction was purged with argon and evacuated 3 times before NEt₃ (10 mL) was added purged and evacuated 3 more times and heated to 60 °C overnight. The reaction mixture was diluted with water (50 mL), extracted with EtOAc (3 × 50 mL), dried with MgSO₄ and concentrated. The crude was purified via flash column chromatography (3:1, Hexane: EtOAc) the clean fraction were taken concentrated *in vacuo* to give 4-(5-(benzyloxy)pent-1-yn-1-yl)-2-(2,6-dioxopiperidin-3-yl)isoindoline-1,3-dione as a brown solid (850 mg, 66%). ¹H NMR (400 MHz, Chloroform-d) δ 8.01 (*br.*s*,* 1H), 7.71 (m, 1H), 7.62 - 7.52 (m, 1H), 7.31 - 7.20 (m, 5H), 4.90 (dd, *J* = 12.3, 5.3 Hz, 1H), 4.48 (s, 2H), 3.62 (t, *J* = 6.1 Hz, 2H), 2.90 - 2.64 (m, 3H), 2.59 (t, *J* = 7.0 Hz, 2H), 2.12 - 2.01 (m, 1H), 1.96 - 1.86 (m, 2H). *m*/*z* (ES⁺) = 431.3.

### 2-(2,6-Dioxopiperidin-3-yl)-4-(5-hydroxypentyl)isoindoline-1,3-dione

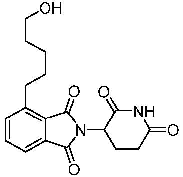

4-(5-(Benzyloxy)pent-1-yn-1-yl)-2-(2,6-dioxopiperidin-3-yl)isoindoline-1,3-dione (126 mg, 0.21 mmol) was suspended in EtOH (10 mL). Palladium on carbon (10% w/w, 20 mg and 0.023 mmol) was added under argon before a hydrogen balloon was fitted and stirred overnight at 25 °C. Reaction was diluted with EtOH (40 mL), filtered through celite and the filtrate was concentrated *in vacuo* to give 2-(2,6-dioxopiperidin-3-yl)-4-(5-hydroxypentyl)isoindoline-1,3-dione as a white solid (50 mg, 49%). mp: 176-178 °C. ¹H NMR (400 MHz, Methanol-*d*₄) δ 7.67 - 7.50 (m, 3H), 5.03 (dd, *J* = 12.5, 5.5 Hz, 1H), 3.45 (t, *J* = 6.6 Hz, 2H), 3.05 - 2.98 (m, 2H), 2.84 - 2.57 (m, 3H), 2.10 - 1.98 (m, 1H), 1.91 (s, 1H), 1.67 - 1.53 (m, 2H), 1.52 - 1.44 (m, 2H), 1.41 - 1.33 (m, 2H). *m*/*z* (ES⁺) = 345.1.

### 5-(2-(2,6-Dioxopiperidin-3-yl)-1,3-dioxoisoindolin-4-yl)pentanal

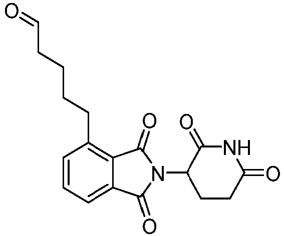

2-(2,6-Dioxopiperidin-3-yl)-4-(5-hydroxypentyl)isoindoline-1,3-dione (623 mg, 1.80 mmol) was suspended in DCM (20 mL) , Pyridinium chlorochromate (582 mg, 2.70 mmol) was added in one portion and allowed to stir for 3 h at 25 °C. Fluorosil was then added to the reaction mixture and allowed to stir for a further hour before being filtered through a pad of celite, the celite pad was washed with DCM (50 mL) and the organics were concentrated to leave 5-(2-(2,6-dioxopiperidin-3-yl)-1,3-dioxoisoindolin-4-yl)pentanal as a grey solid. *m*/*z* (ES⁺) = 343.3

### tert-Butyl 4-(4-(5-(2-(2,6-dioxopiperidin-3-yl)-1,3-dioxoisoindolin-4-yl)pentyl)piperazin-1-yl)piperidine-1-carboxylate

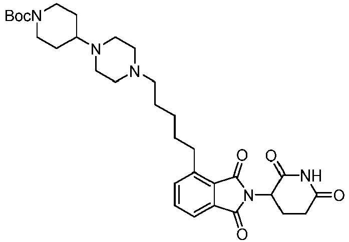

1-(1-Boc-piperdin-4-yl)-piperazine (39 mg, 0.13 mmol) was suspended in anhydrous DCM (10 mL) over 4 Å molecular sieves and stirred for 10 mins. 5-(2-(2,6-Dioxopiperidin-3-yl)-1,3-dioxoisoindolin-4-yl)pentanal (42 mg, 0.13 mmol) was added and stirred for 3h. Sodium triacetoxyborohydride (82 mg, 0.39 mmol) was added and stirred for 3h at 25 °C. Water (20 mL) was added and extracted with DCM (2 × 30 mL). The organics were dried with MgSO₄ and concentrated *in vacuo* to give *tert*-butyl 4-(4-(5-(2-(2,6-dioxopiperidin-3-yl)-1,3-dioxoisoindolin-4-yl)pentyl)piperazin-1-yl)piperidine-1-carboxylate as a colourless oil (40 mg, 54% (2 steps)). ¹H NMR (400 MHz, Chloroform-d) δ 7.65 (d, *J* = 7.5 Hz, 1H), 7.56 (*app*. t*, J = 7.5* Hz, 1H), 7.45 (d, *J* = 7.5 Hz, 1H), 4.94 - 4.85 (m, 1H), 4.13 - 3.99 (m, 2H), 3.00 (td, *J* = 7.4, 4.2 Hz, 2H), 2.88 - 2.21 (m, 14H), 2.12 - 2.04 (m, 1H), 1.80 - 1.42 (m, 9H), 1.38-1.28 (m, 12H).. *m*/*z* (ES⁺) = 596.0.

### 4-(4-(5-(2-(2,6-Dioxopiperidin-3-yl)-1,3-dioxoisoindolin-4-yl)pentyl)piperazin-1-yl)piperidin-1-ium hydrochloride

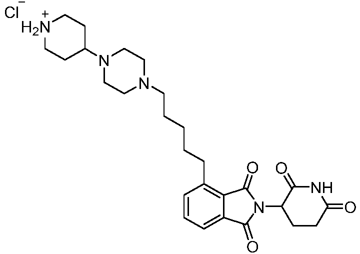

*Tert-*butyl 4-(4-(5-(2-(2,6-dioxopiperidin-3-yl)-1,3-dioxoisoindolin-4-yl)pentyl)piperazin-1-yl)piperidine-1-carboxylate (40 mg, 0.067 mmol) was suspended in THF (5 mL). HCl in dioxane (4N, 3 mL) was added and stirred at 25 °C overnight. The reaction was concentrated under reduced pressure to leave 4-(4-(5-(2-(2,6-dioxopiperidin-3-yl)-1,3-dioxoisoindolin-4-yl)pentyl)piperazin-1-yl)piperidin-1-ium hydrochloride as a white solid (32 mg, 92%). ¹H NMR (400 MHz, DMSO-*d*₆) δ 11.11 (s, 1H, NH(imide)), 7.84 - 7.65 (m, 3H), 5.15 (dd, *J* = 13.2, 6.4 Hz, 1H), 3.64 (t, *J =* 6.6 Hz, 2H), 3.40 (t, *J* = 5.7 Hz, 2H), 3.16 - 3.00 (m, 4H), 2.99 - 2.78 (m, 4H), 2.68 - 2.53 (m, 2H), 2.40 - 1.83 (m, 7), 1.82 - 1.69 (m, 4H), 1.63 (h, *J* = 6.8, 5.8 Hz, 2H), 1.56 - 1.46 (m, 2H). 2 aliphatic protons missing underneath DMSO peak. *m*/*z* (ES⁺) = 495.9.

### 5-(Methylamino)pyrimidine-2,4-(1H,3H)-dione

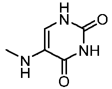

5-Bromo uracil (19.10 g, 100.00 mmol) was suspended in 40% aq. methylamine (160 mL) and heated at 80 ^{o}C overnight. Reaction was cooled to RT, acidified with 3M aq. HCl and a white precipitate formed which was filtered and washed with water to give 5-(methylamino)pyrimidine-2,4(1H,3H)-dione as a white solid (9.3 g, 66%).² mp: 199-202 °C. ¹H NMR (400 MHz, DMSO-*d*₆) δ 11.09 (s, 1H), 10.14 (s, 1H), 6.19 (d, *J* = 5.0 Hz, 1H), 4.46 (q, *J* = 5.5 Hz, 1H) (Me peak under DMSO peak). *m*/*z* (ES⁺)= 141.1.

### N-(2,4-Dioxo-1,2,3,4-tetrahydropyrimidin-5-yl)-N-methyl-2-nitrobenzamide

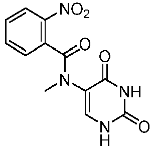

5-(Methylamino)pyrimidine-2,4(1*H*,3*H*)-dione (2.00 g, 14.80 mmol) was suspended in THF (10 mL) and cooled at 0 °C, aq. NaOH (2 M, 20mL) was added followed by 2-nitrobenzoyl chloride (2.30 mL, 17.8 mmol) dropwise. Following the addition reaction was warmed to 25 °C and stirred for 48 h. Reaction was acidified to pH 4 with 4M aq. HCl and left to stand to 48 h. The solid was filtered and washed with water to give N-(2,4-dioxo-1,2,3,4-tetrahydropyrimidin-5-yl)-N-methyl-2-nitrobenzamide as a white solid (2.60 g, 61%).² mp: 259 - 261 °C (degraded). ¹H NMR (400 MHz, DMSO-*d*₆) δ 11.35 (s, 1H), 10.88 (d, *J* = 3.8 Hz, 1H), 8.10 (dd, *J* = 8.2, 1.2 Hz, 1H), 7.75 (td, *J* = 7.5, 1.2 Hz, 1H), 7.64 (ddd, *J* = 8.2, 7.5, 1.2 Hz, 1H), 7.43 - 7.33 (m, 2H), 3.15 (s, 3H)

### N-(2, 4-Dichloropyrimidin-5-yl)-N-methyl-2-nitrobenzamide

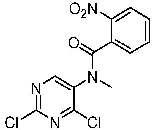

*N*-(2,4-Dioxo-1,2,3,4-tetrahydropyrimidin-5-yl)-N-methyl-2-nitrobenzamide (9.00 g, 27.6 mmol) was suspended phosphorous oxychloride (50 mL) and N,N-dimethylaniline (2.5 mL). Reaction was heated to 100 °C for 18 h before being cooled to 25 °C. Concentrated *in vauco* and the crude was taken straight into the next step (reduction and cyclisation).²

### 2-Chloro-5-methyl-5,11-dihydro-6H-benzo[e]pyrimido[5,4-b][1,4]diazepin-6-one

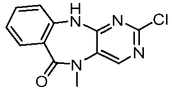

The crude of *N*-(2,4-dichloropyrimidin-5-yl)-*N*-methyl-2-nitrobenzamide (assumed 27.6 mmol) was suspended in acetic acid (100 mL) and Fe powder (9.1 g) was added and heated to 60 °C for 4 h. Reaction was cooled to 25 °C, the acetic acid was removed *in vacuo,* the crude was solublised in DCM/MeOH (50 mL/20 mL), filtered through celite and concentrated to leave a white solid. Cold EtOH was added, and the solid was collected via filtration to give 2-chloro-5-methyl-5,11-dihydro-6*H*-benzo[e]pyrimido[5,4-b][1,4]diazepin-6-one as a white solid (5.8 g, 82% (2 steps)).² mp: 196 - 201 °C. ¹H NMR (400 MHz, Chloroform-*d*) δ 8.22 (s, 1H), 7.98 (dd, *J* = 8.2, 1.6 Hz, 1H), 7.45 - 7.33 (m, 1H), 7.13 (ddd, *J* = 8.2, 7.3, 1.0 Hz, 1H), 6.81 (dd, *J* = 8.2, 1.0 Hz, 1H), 6.66 (s, 1H), 3.51 (s, 3H). m/z= 261.2 (³⁵Cl), 263.2 (³⁷Cl).

### 2-Chloro-5-methyl-11-(methylsulfonyl)-5,11-dihydro-6H-benzo[e]pyrimido[5,4-b][1,4]diazepin-6-one

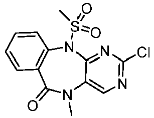

2-Chloro-5-methyl-5,11-dihydro-6*H*-benzo[e]pyrimido[5,4-b][1,4]diazepin-6-one (200 mg, 0.77 mmol) was suspended in THF (10 mL). Sodium hydride (60% suspension in mineral oil 61 mg, 1.53 mmol) was added at 0 °C and stirred for 1 h. Methane sulfonyl chloride (0.13mL and 1.53 mmol) was added dropwise and stirred for 1 h. Reaction was quenched with water (10 mL) and extracted with EtOAc (3 × 20 mL), the organics were dried with MgSO₄ and concentrated *in vacuo.* The crude was purified via flash column chromatography (1:1, Hexane:EtOAc) the clean fractions were taken concentrated *in vacuo* to afford 2-chloro-5-methyl-11-(methylsulfonyl)-5,11-dihydro-6*H*-benzo[e]pyrimido[5,4-b][1,4]diazepin-6-one as a white solid (151 mg, 61 %).² mp: 101 -104 °C. ¹H NMR (400 MHz, DMSO-*d*₆) δ 9.06 (s, 1H), 7.87 - 7.79 (m, 1H), 7.72 - 7.67 (m, 2H), 7.54 (ddd, *J* = 8.2, 5.9, 2.5 Hz, 1H), 3.78 (s, 3H), 3.56 (s, 3H). *m*/*z* (ES⁺) = 339.2 (³⁵Cl), 341.2 (³⁷Cl).

### Ethyl 3-ethoxy-4-nitrobenzoate

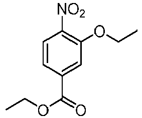

3-Hydroxy-4-nitrobenzonic acid (1.00 g, 5.46 mmol), potassium carbonate (3.00 g, 16.45 mmol) were suspended in DMF (20 mL). Iodoethane (1.3 mL, 21.74 mmol) was added dropwise and stirred at 25 °C for 18 h. Water (50 mL) was added to reaction mixture and extracted with EtOAc (3 × 50 mL) the organics were washed with brine (30 mL) and dried with MgSO₄ and concentrated *in vacuo* to leave ethyl 3-ethoxy-4-nitrobenzoate as an orange oil (1.01 g, 83%).^{2 1}H NMR (400 MHz, Chloroform-d) δ 7.81 (d, *J =* 8.3 Hz, 1H), 7.75 (*app* s, 1H), 7.69 (dd, *J* = 8.3, 1.5 Hz, 1H), 4.48 - 4.39 (q, *J* = 6.9 Hz, 2H), 4.31 - 4.23 (q, *J* = 7.1 Hz, 2H), 1.50 (t, *J* = 6.9 Hz, 3H), 1.44 (t, *J* = 7.1 Hz, 3H).

### Ethyl 4-amino-3-ethoxybenzoate

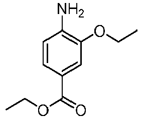

Ethyl 3-ethoxy-4-nitrobenzoate (570 mg 2.39 mmol) was suspended in EtOH (10 mL) and THF (10 mL). Palladium on carbon (10% w/w, 20 mg, 0.23 mmol) was added under Argon and then reaction mix placed in hydrogen atmosphere and stirred for 18 h. Reaction was diluted with EtOH (20 mL) filtered through celite and concentrated *in vacuo* to give ethyl 4-amino-3-ethoxybenzoate as a brown solid (500 mg, 100%).² mp: 86 - 88 °C. ¹H NMR (400 MHz, Chloroform-d) δ 7.73 (d, *J* = 8.3 Hz, 1H), 7.66 (d, *J* = 1.6 Hz, 1H), 7.60 (dd, *J* = 8.3, 1.6 Hz, 1H), 4.35 (q, *J* = 7.1 Hz, 2H), 4.18 (q, *J* = 7.0 Hz, 2H), 1.42 (t, *J =* 7.0 Hz, 3H), 1.35 (t, *J* = 7.1 Hz, 3H). *m*/*z* (ES⁺)= 210.0.

### Ethyl 3-ethoxy-4-((5-methyl-11-(methylsulfonyl)-6-oxo-6,11-dihydro-5H-benzo[e]pyrimido[5,4-b][1,4]diazepin-2-yl)amino)benzoate

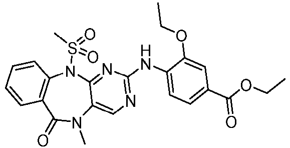

2-Chloro-5-methyl-11-(methylsulfonyl)-5,11-dihydro-6*H*-benzo[e]pyrimido[5,4-b][1,4]diazepin-6-one (270 mg, 1.04 mmol), ethyl 4-amino-3-ethoxybenzoate (261 mg, 1.25 mmol), K₂CO₃ (828 mg, 6.00 mmol) and tBuXPhos Gen 3 pre catalyst (65 mg, 0.08 mmol) were suspended in tBuOH (15 mL) evacuated and purged with Argon 3 times and then heated at 100 °C for 18 h. Water (50 mL) was added and extracted with EtOAc (3 × 30 mL), dried with MgSO₄ and concentrated *in vacuo.* The crude was purified via flash column chromatography (10:1, Toluene:EtOAc) the clean fractions were taken concentrated under reduced pressure to give ethyl 3-ethoxy-4-((5-methyl-11-(methylsulfonyl)-6-oxo-6,11-dihydro-5H-benzo[e]pyrimido[5,4-b][1,4]diazepin-2-yl)amino)benzoate as an orange oil (110 mg, 23 %). ¹H NMR (400 MHz, Chloroform-d) δ 8.51 (d, *J* = 1.5 Hz, 1H), 8.46 (dd, *J* = 8.5, 1.4 Hz, 1H), 8.01 (s, 1H), 7.94 (dt, *J* = 7.8, 1.6 Hz, 1H), 7.73 (dt, *J* = 8.5, 1.7 Hz, 1H), 7.60 - 7.54 (m, 2H), 7.53 - 7.43 (m, 2H), 4.37 (q, *J* = 7.0 Hz, 2H), 4.25 (q, *J* = 7.2 Hz, 2H), 3.62 (s, 3H), 3.49 (s, 3H, N-Me), 1.51 (t, *J* = 7.0 Hz, 3H), 1.40 (t, *J =* 7.2, 3H). *m*/*z=* 511.1.

### 3-Ethoxy-4-((5-methyl-11-(methylsulfonyl)-6-oxo-6,11-dihydro-5H-benzo[e]pyrimido[5,4-b][1,4]diazepin-2-yl)amino)benzoic acid

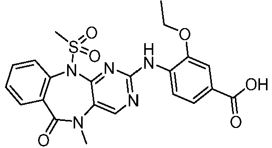

3-Ethoxy-4-((5-methyl-11-(methylsulfonyl)-6-oxo-6,11-dihydro-5H-benzo[e]pyrimido[5,4-b][1,4]diazepin-2-yl)amino)benzoic acid (40 mg, 0.08 mmol) and LiOH (10 mg, 0.43 mmol) were suspended in THF (5 mL), H₂O (1 mL) and MeOH (1 mL) and stirred at 25 °C for 19 h. The reaction was concentrated under reduced pressure the crude was dissolved in DCM (50 mL) and washed with 1 M aqueous hydrochloric acid (2 x 30 mL). The organics were dried with MgSO₄ and concentrated *in vacuo* to give 3-ethoxy-4-((5-methyl-11-(methylsulfonyl)-6-oxo-6,11-dihydro-5*H*-benzo[e]pyrimido[5,4-b][1,4]diazepin-2-yl)amino)benzoic acid as a white solid (30 mg, 77%). mp: 265 - 266 °C. ¹H NMR (400 MHz, DMSO-*d*₆) δ 12.81 (s, 1H), 8.98 (s, 1H), 8.79 (s, 1H), 7.99 (d, *J* = 8.3 Hz, 1H), 7.78 (dd, *J =* 7.9, 1.5 Hz, 1H), 7.68 - 7.56 (m, 2H) 7.59 (dd, *J* = 8.3, 4.0 Hz, 1H), 7.54 - 7.44 (m, 2H), 4.16 (app qt, *J* = 7.0 Hz, 2H), 3.78 (s, 3H, N-Me), 3.50 (s, 3H), 1.32 (t, *J* = 7.0 Hz, 3H). *m*/*z* (ES⁺)= 484.3.

### Method A

Alkyne alcohol (1 equiv.), TsCl (1.2 equiv.), cat. DMAP and NEt₃ (3 equiv.) was solubilised in DCM (0.1 mM), were stirred at 25 °C for 17 h. Saturated aq. NaHCO₃ (20 mL) was added, the crude was extracted with DCM (2 x 20 mL), the organics were combined and dried with MgSO₄, filtered and concentrated *in vacuo.* The crude material was taken into the next step without further purification.

### But-3-yn-1-yl 4-methylbenzenesulfonate

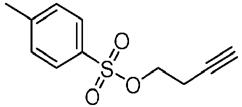

Synthesised using Method A. 553 mg, 44%. ¹H NMR (500 MHz, Chloroform-d) δ 7.83 (d, *J* = 8.1 Hz, 2H), 7.37 (d, *J* = 8.1 Hz, 2H), 4.13 (t, *J* = 7.0 Hz, 2H), 2.58 (td, *J* = 7.0, 2.7 Hz, 2H), 2.48 (s, 3H), 1.99 (t, *J* = 2.7 Hz, 1H).

### Pent-4-yn-1-yl 4-methylbenzenesulfonate

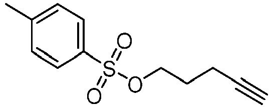

Synthesised using Method A. 600 mg, 57 % ¹H NMR (400 MHz, Chloroform-d) δ 7.79 (d, *J* = 8.3 Hz, 2H), 7.35 (d, *J* = 8.1 Hz, 2H), 4.05 (t, *J* = 6.3 Hz, 2H), 2.45 (s, 3H), 2.16 (td, *J* = 7.0, 2.7 Hz, 2H), 1.92 (t, *J* = 2.7 Hz, 1H), 1.82 - 1.72 (m, 2H), 1.61 - 1.51 (m, 2H).

### Hex-5-yn-1-yl 4-methylbenzenesulfonate

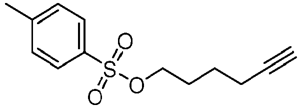

Synthesised using Method A. 1.01 g, 62% ¹H NMR (500 MHz, Chloroform-d) δ 7.75 - 7.68 (m, 2H), 7.28 (d, *J =* 8.0 Hz, 2H), 3.96 (t, *J =* 6.5 Hz, 2H), 2.38 (s, 3H), 2.08 (td, *J* = 6.8, 2.7 Hz, 2H), 1.86 (t, *J =* 2.7 Hz, 1H), 1.64 - 1.54 (m, 2H), 1.46 - 1.29 (m, 4H).

### Hep-6-yn-1-yl 4-methylbenzenesulfonate

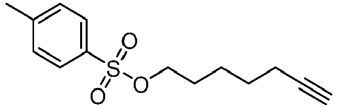

Synthesised using Method A. Material taken straight into next step.

### Oct-7-yn-1-yl 4-methylbenzenesulfonate

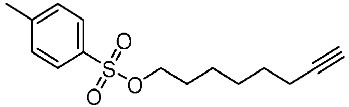

Synthesised using Method A. Material taken straight into next step.

### Non-8-yn-1-yl 4-methylbenzenesulfonate

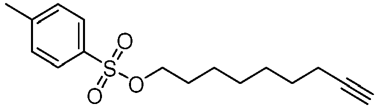

Synthesised using Method A. 2.5 g, 65% ¹H NMR (500 MHz, Chloroform-*d*) δ 7.72 (d, *J* = 8.2 Hz, 2H), 7.28 (d, *J* = 8.0 Hz, 2H), 3.95 (t, *J* = 6.5 Hz, 2H), 2.38 (s, 3H), 2.09 (td, *J* = 7.1, 2.7 Hz, 2H), 1.86 (t, *J* = 2.6 Hz, 1H), 1.63 - 1.53 (m, 2H), 1.44 - 1.38 (m, 2H), 1.33 - 1.13 (m, 6H).

### Dec-9-yn-1-yl 4-methylbenzenesulfonate

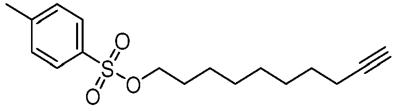

Synthesised using Method A. 500 mg, 61%.
¹H NMR (400 MHz, Chloroform-*d*) δ 7.84 - 7.74 (m, 2H), 7.34 (d, *J* = 8.0 Hz, 2H), 4.01 (t, *J =* 6.5 Hz, 2H), 2.43 (d, *J* = 14.4 Hz, 3H), 2.16 (td, *J* = 7.1, 2.6 Hz, 2H), 1.93 (t, *J* = 2.7 Hz, 1H), 1.63 (d, *J =* 14.3 Hz, 4H), 1.50 (dq, *J* = 14.6, 6.9 Hz, 2H), 1.43 - 1.19 (m, 11H).

### Methyl 3-iodo-2-methylbenzoate

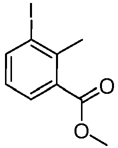

3-lodo-2-methylbenzonic acid (1.00 g, 3.81 mmol) was suspended in MeOH (40 mL). SOCl₂ (0.28 mL, 3.81 mmol) was added dropwise at 0 °C and heated to 80 °C for 2 h. The reaction mix was concentrated *in vacuo.* The residue was quenched with saturated aq. NaHCO₃ (40 mL) and was extracted with EtOAc (3 x 20 mL), the organics were dried with MgSO₄, filtered and concentrated to leave a Methyl 3-iodo-2-methylbenzoate (0.85 g, 80%). ¹H NMR (500 MHz, Chloroform-*d*) δ 7.98 (dd, *J* = 7.8, 1.3 Hz, 1H), 7.73 (dd, *J* = 7.8, 1.3 Hz, 1H), 6.92 (*app.* t, *J* = 7.8 Hz, 1H), 3.90 (s, 3H), 2.66 (s, 3H).

### 3-(4-Iodo-1-oxoisoindolin-2-yl)piperidine-2,6-dione

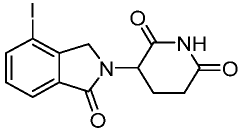

Methyl 3-iodo-2-methylbenzoate (850 mg, 3.07 mmol) was suspended in diethylcarbonate (8 mL), NBS (653 mg, 3.69 mmol) and AIBN (101 mg, 0.616 mmol) was added in one portion and stirred at 80 °C for 17 h. The reaction mix was concentrated *in vacuo* and then suspended in MeCN (10 mL), 3-amino piperidone hydrochloride (595mg, 3.64 mmol) and NEt₃ (0.66 mL, 4.66 mmol) were added and heated at 80 °C for 17 h. The reaction mix was cooled and concentrated *in vacuo,* water (25 mL) was added and the resulting solid was filtered to leave 3-(4-iodo-1-oxoisoindolin-2-yl)piperidine-2,6-dione as a purple solid. ¹H NMR (500 MHz, DMSO-*d*₆) δ 11.03 (s, 1H), 8.05 (d, *J* = 7.8 Hz, 1H), 7.79 (d, *J* = 7.5 Hz, 1H), 7.37 (app. t, *J* = 7.6 Hz, 1H), 5.17 (dd, *J* = 13.3, 5.1 Hz, 1H), 4.37 - 4.10 (m, 2H), 2.98- 2.88 (m, 1H), 2.68 - 2.44 (m, 3H), 2.10 - 1.99 (m, 1H).

### Method B

Alkyne tosylate (1 equiv.), 4-piperzin-1-yl-piperidine-1-carbooxylic acid *tert* butyl ester (2 equiv.), Cs₂CO₃ (3 equiv.) and Nal (catalytic) were solubilised in MeCN (0.1 mM). The reaction mix was heated at 80 °C for 17 h. The reaction mix was concentrated *in vacuo,* the crude was diluted with EtOAc (30 mL washed with saturated aq. NaHCO₃ (2 x 20 mL). The organics were dried with MgSO₄, filtered and concentrated *in vacuo.* The crude mix was taken into the next step.

3-(4-Iodo-1-oxoisoindolin-2-yl)piperidine-2,6-dione (1 equiv.), Alkyne (2 equiv.), Pd(PPh₃)₂Cl₂ (0.06 equiv.), Cul (0.03 equiv.) were solubilised DMF (0.1 mM), purged with Argon, NEt₃ (10 equiv.) was added and was heated to 80 °C for 2 h. The reaction was cooled to RT, H₂O (20 mL) was added and extracted with EtOAc (3 x 20 mL). The organics were dried onto celite and purified via acidic reverse phase, the clean fraction were taken and concentrated *in vacuo.*

### tert-Butyl 4-(4-(4-(2-(2,6-dioxopiperidin-3-yl)-1-oxoisoindolin-4-yl)but-3-yn-1-yl)piperazin-1-yl)piperidine-1-carboxylate (Intermediate for OS3)

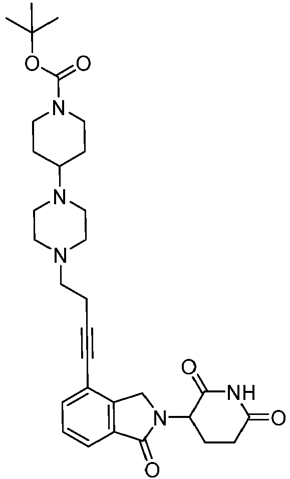

Synthesised using Method B. 61 mg, 10%. *m*/*z (ES⁺)* = 564.4,

### tert-Butyl 4-(4-(5-(2-(2,6-dioxopiperidin-3-yl)-1-oxoisoindolin-4-yl)pent-4-yn-1-yl)piperazin-1-yl)piperidine-1-carboxylate (Intermediate for CE207)

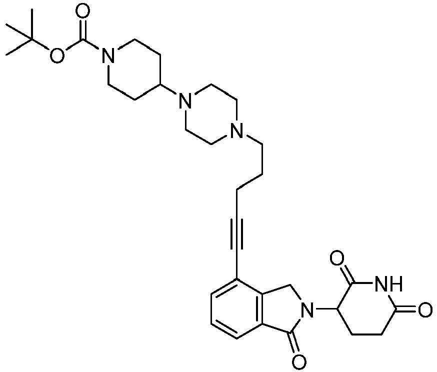

Synthesised using Method B. 46 mg, 64%. *m*/*z (ES⁺)* = 578.1

### tert-butyl 4-(4-(5-(2-(2,6-dioxopiperidin-3-yl)-1-oxoisoindolin-4-yl)hex-5-yn-1-yl)piperazin-1-yl)piperidine-1-carboxylate (Intermediate for OS4)

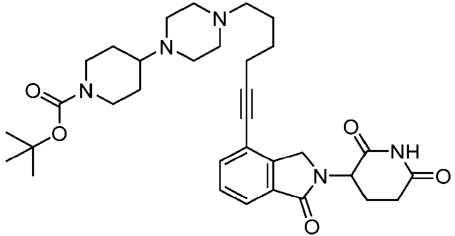

Synthesised using Method B. 90 mg, 33%. *m*/*z (ES⁺)* = 592.5

### tert-butyl 4-(4-(5-(2-(2,6-dioxopiperidin-3-yl)-1-oxoisoindolin-4-yl)hept-6-yn-1-yl)piperazin-1-yl)piperidine-1-carboxylate (Intermediate for OS6)

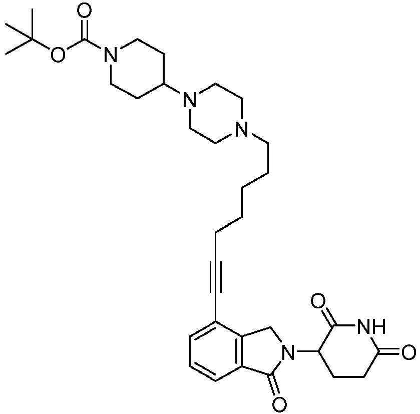

Synthesised using Method B. 40mg, 41% *m*/*z (ES⁺)* = 626.5

### tert-butyl 4-(4-(2-(2-((3-(2-(2,6-dioxopiperidin-3-yl)-1-oxoisoindolin-4-yl)prop-2-yn-1-yl)oxy)ethoxy)ethyl)piperazin-1-yl)piperidine-1-carboxylate (Intermediate for OS9)

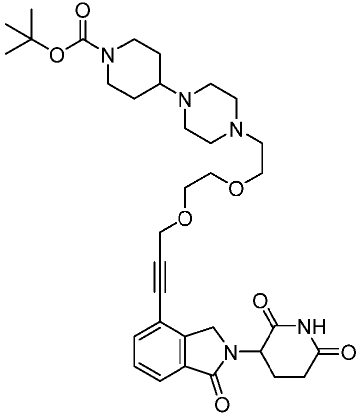

Synthesised using Method B. 120 mg, 35%. *m*/*z (ES⁺)* = 638.5

### tert-butyl 4-(4-(5-(2-(2,6-dioxopiperidin-3-yl)-1-oxoisoindolin-4-yl)oct-7-yn-1-yl)piperazin-1-yl)piperidine-1-carboxylate (Intermediate for OS10)

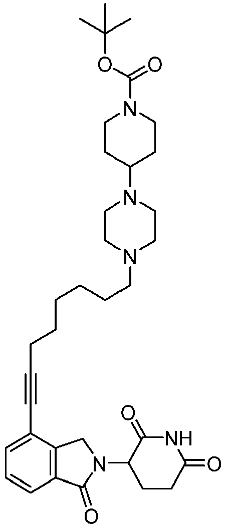

Synthesised using Method B. 40 mg 22 %. *m*/*z (ES⁺)* = 620.4

### tert-butyl 4-(4-(5-(2-(2,6-dioxopiperidin-3-yl)-1-oxoisoindolin-4-yl)non-8-yn-1-yl)piperazin-1-yl)piperidine-1-carboxylate (Intermediate for OS11)

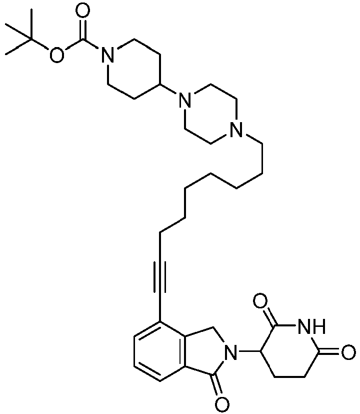

Synthesised using Method B. 121 mg, 36%. *m*/*z (ES⁺)* = 634.1

### tert-butyl 4-(4-(10-(2-(2,6-dioxopiperidin-3-yl)-1-oxoisoindolin-4-yl)dec-9-yn-1-yl)piperazin-1-yl)piperidine-1-carboxylate (Intermediate for OS14)

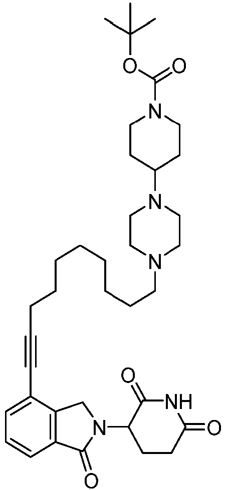

Synthesised using Method B. 100 mg, 32%. *m*/*z (ES⁺)* = 648.4

### Method C

Phthalic anhydride (1 equiv.) 3-aminopiperidine-2,6-dione hydrochloride (1.2 equiv.) and sodium acetate (1.2 equiv.) were suspended in acetic acid (glacial, 0.1 mmol) and heated at 140 °C overnight . Reaction was cooled to 25 °C, concentrated *in vacuo* and diluted with water (30 mL) and the solid was filtered to afford the desired product. These products do not ionise well on MS.

### 4-Fluoro-2-(2,6-dioxopiperidin-3-yl)isoindoline-1,3-dione

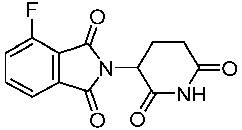

Synthesised by method C. 800 mg, 48 %

### 2-(2,6-Dioxopiperidin-3-yl)-5-fluoroisoindoline-1,3-dione

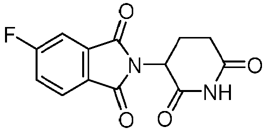

Synthesised by method C. 800 mg, 48 %

### 2-(2,6-dioxopiperidin-3-yl)-4-((6-hydroxyhexyl)amino)isoindoline-1,3-dione

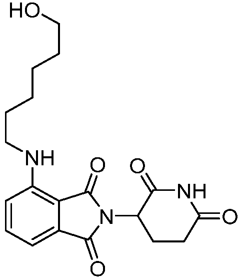

4-Fluoro-2-(2,6-dioxopiperidin-3-yl)isoindoline-1,3-dione (200 mg, 0.72 mmol), 6-aminohexanol (84 mg, 0.72 mmol) and DIPEA (0.38 mL, 2.1 mmol) were suspended in DMF (5 mL) and heated at 80 °C for 17 h. The reaction was cooled and diluted with H₂O (30 mL) and this was extracted with EtOAc (3 x 10 mL), the organics were combined dried with MgSO₄, filtered and concentrated *in vacuo,* the material was purified via acidic reverse phase to leave 2-(2,6-dioxopiperidin-3-yl)-4-((6-hydroxyhexyl)amino)isoindoline-1,3-dione as a brown oil (30 mg, 11%). *m*/*z (ES⁺)* = 374.3

### 6-((2-(2,6-dioxopiperidin-3-yl)-1,3-dioxoisoindolin-4-yl)amino)hexyl 4-methylbenzenesulfonate

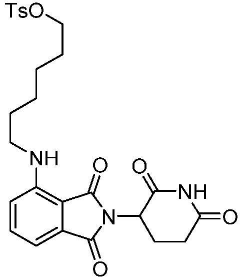

2-(2,6-Dioxopiperidin-3-yl)-4-((6-hydroxyhexyl)amino)isoindoline-1,3-dione (30 mg, 0.080 mmol), TsCl (17 mg, 0.088 mmol) and NEt₃ (0.02 mL, 0.12 mmol) were suspended in DCM (5 mL) and a catalytic amount DMAP was added and allowed to stir at 25 °C for 17 h. The reaction mix was quenched with saturated aq. sodium bicarbonate (15 mL) and extracted with DCM (3 x 10 mL). The organics were combined, dried with MgSO₄ filtered and concentrated *in vacuo.* The material was taken straight into the next step.

### tert-Butyl 4-(4-(6-((2-(2,6-dioxopiperidin-3-yl)-1,3-dioxoisoindolin-4-yl)amino)hexyl)piperazin-1-yl)piperidine-1-carboxylate

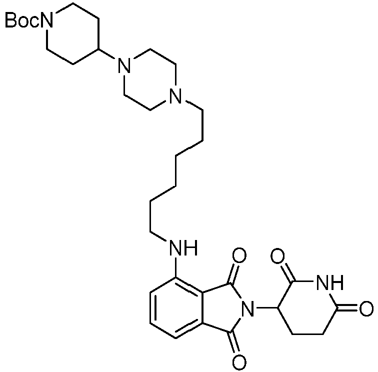

The material from a previous step was taken up in MeCN (5 mL), 4-piperzin-1-yl-piperidine-1-carbooxylic acid *tert* butyl ester (58 mg, 0.19 mmol), Cs₂CO₃ (61 mg, 0.19 mmol) and Nal (3 mg, 0.03 mmol) were added and heated to 50 °C for 17 h. The material was then concentrated *in vacuo,* the crude was taken in saturated aq. sodium bicarbonate (10 mL) and extracted with EtOAc (3 x 10 mL), the organics were combined, dried with MgSO₄, filtered and concentrated *in vacuo.* The material was purified via acidic reverse phase and the clean fraction were taken and concentrated *in vacuo* to leave tert-butyl 4-(4-(6-((2-(2,6-dioxopiperidin-3-yl)-1,3-dioxoisoindolin-4-yl)amino)hexyl)piperazin-1-yl)piperidine-1-carboxylate (11 mg, 34%). *m*/*z (ES⁺)* = 625.2

### 2-(2,6-Dioxopiperidin-3-yl)-5-((6-hydroxyhexyl)amino)isoindoline-1,3-dione

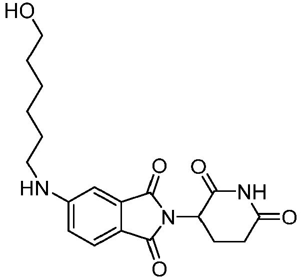

4-Fluoro-2-(2,6-dioxopiperidin-3-yl)isoindoline-1,3-dione (200 mg, 0.72 mmol), 6-aminohexanol (252 mg, 2.1 mmol) and DIPEA (0.38 mL, 2.1 mmol) were suspended in NMP (5 mL) and heated at 80 °C for 17 h. The reaction was cooled and diluted with H₂O (30 mL) and this was extracted with EtOAc (3 x 10 mL), the organics were combined dried with MgSO₄, filtered and concentrated with *in vacuo,* the material was purified via acidic reverse phase to leave 2-(2,6-dioxopiperidin-3-yl)-4-((6-hydroxyhexyl)amino)isoindoline-1,3-dione (60 mg, *22%). m*/*z (ES⁺)* = 374.3

### 6-((2-(2,6-Dioxopiperidin-3-yl)-1,3-dioxoisoindolin-5-yl)amino)hexyl 4-methylbenzenesulfonate

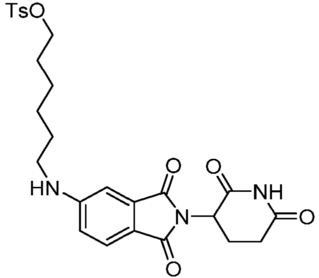

2-(2,6-Dioxopiperidin-3-yl)-4-((6-hydroxyhexyl)amino)isoindoline-1,3-dione (60 mg, 0.16 mmol), TsCl (36 mg, 0.19 mmol) and NEt₃ (0.07 mL, 0.48 mmol) were suspended in DCM (3 mL) and a catalytic amount DMAP was added and allowed to stir at 25 °C for 17 h. The reaction mix was quenched with saturated aq. sodium bicarbonate (15 mL) and extracted with DCM (3 x 10 mL). The organics were combined, dried with MgSO₄ filtered and concentrated *in vacuo.* The material was taken straight into the next step.

### tert-Butyl 4-(4-(6-((2-(2,6-dioxopiperidin-3-yl)-1,3-dioxoisoindolin-5-yl)amino)hexyl)piperazin-1-yl)piperidine-1-carboxylate

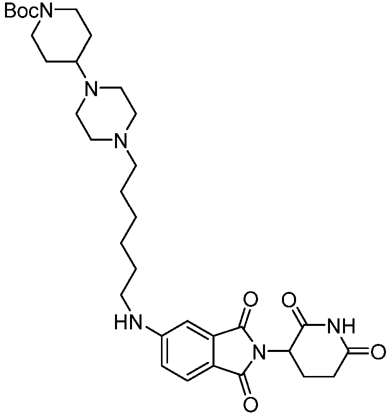

The material from a previous step was taken up in MeCN (5 mL), 4-piperzin-1-yl-piperidine-1-carbooxylic acid *tert* butyl ester (58 mg, 0.19 mmol), Cs₂CO₃ (61 mg, 0.19 mmol) and Nal (3 mg, 0.03 mmol) were added and heated to 50 °C for 17 h. The material was then concentrated *in vacuo,* the crude was taken in saturated aq. sodium bicarbonate (10 mL) and extracted with EtOAc (3 x 10 mL), the organics were combined, dried with MgSO₄, filtered and concentrated *in vacuo.* The material was purified via acidic reverse phase and the clean fraction were taken and concentrated *in vacuo* to leave tert-butyl 4-(4-(6-((2-(2,6-dioxopiperidin-3-yl)-1,3-dioxoisoindolin-4-yl)amino)hexyl)piperazin-1-yl)piperidine-1-carboxylate (11 mg, 34%). *m*/*z (ES⁺)* = 625.3

### 2-(2,6-Dioxopiperidin-3-yl)-4-hydroxyisoindoline-1,3-dione

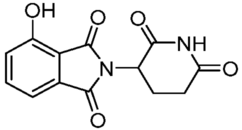

Synthesised by method C. 2.5 g, 56 %. *m*/*z (ES⁺)* = 273.1

### 2-(2,6-Dioxopiperidin-3-yl)-4-((6-hydroxyhexyl)oxy)isoindoline-1,3-dione

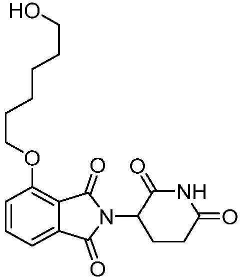

1,6-Hexanediol (212 mg, 2.16 mmol) and triphenylphosphine (561 mg, 2.16 mmol) were suspended in THF (5 mL), DIAD (0.42 mL, 2.16 mmol) was added dropwise to solution and allowed to stir at 25 °C for 30 mins. 2-(2,6-Dioxopiperidin-3-yl)-4-hydroxyisoindoline-1,3-dione (500 mg, 1.80 mmol) was added in one portion and allowed to stir at 25 °C for 3 h. The reaction mix was then concentrated *in vacuo,* water (30 mL) was added to the crude and then extracted with EtOAc (3 x 20 mL), the organics were dried with MgSO₄, filtered and concentrated *in vacuo.* The crude was then taken up in EtOH (15 mL) and ZnCl₂ (700 mg, 5.49 mmol) was added in one portion and allowed to stir at 25 °C for a further 17 h. The mixture was then filtered to leave 2-(2,6-dioxopiperidin-3-yl)-4-((8-hydroxyoctyl)oxy)isoindoline-1,3-dione (600 mg, 40%). *m*/*z (ES⁺)* = 375.3

### 6-((2-(2,6-Dioxopiperidin-3-yl)-1,3-dioxoisoindolin-4-yl)oxy)hexyl 4-methylbenzenesulfonate

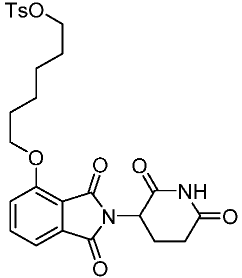

2-(2,6-dioxopiperidin-3-yl)-4-((6-hydroxyhexyl)oxy)isoindoline-1,3-dione (80 mg, 0.21 mmol), TsCl (50 mg, 0.25 mmol), NEt₃ (0.09 mL, 0.63 mmol) and cat. DMAP were suspended in DCM (5 mL) and stirred at 25 °C for 17 h. The reaction was diluted with sat. aq. sodium bicarbonate (3 x 10 mL) and extracted with EtOAc (3 x 10 mL), the organics were combined dried with MgSO4, filtered and concentrated *in vacuo* to leave 6-((2-(2,6-dioxopiperidin-3-yl)-1,3-dioxoisoindolin-4-yl)oxy)hexyl 4-methylbenzenesulfonate (75 mg, 68%).

### tert-Butyl 4-(4-(6-((2-(2,6-dioxopiperidin-3-yl)-1,3-dioxoisoindolin-4-yl)oxy)hexyl)piperazin-1-yl)piperidine-1-carboxylate

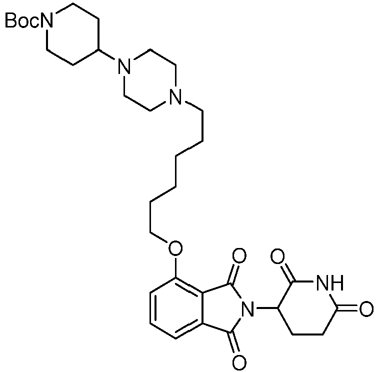

6-((2-(2,6-dioxopiperidin-3-yl)-1,3-dioxoisoindolin-4-yl)oxy)hexyl 4-methylbenzenesulfonate (75 mg, 0.14 mmol), *tert-butyl* 4-(piperazin-1-yl)piperidine-1-carboxylate hydrochloride (86 mg, 0.28 mmol), Cs₂CO₃ (136 mg, 0.42 mmol) and cat. Nal were suspended in MeCN (5 mL) and heated to 80 °C for 17 h. The reaction was cooled and concentrated *in vacuo,* diluted with H₂O (10 mL), and extracted with EtOAc (3 x 10 mL). The crude was purified via acidic reverse phase and the clean fractions were taken and concentrated *in vacuo* to leave *tert-butyl* 4-(4-(6-((2-(2,6-dioxopiperidin-3-yl)-1,3-dioxoisoindolin-4-yl)oxy)hexyl)piperazin-1-yl)piperidine-1-carboxylate (50 mg, 57%). *m*/*z (ES⁺)* = 626.4

### 2-(2,6-Dioxopiperidin-3-yl)-4-((8-hydroxyoctyl)oxy)isoindoline-1,3-dione

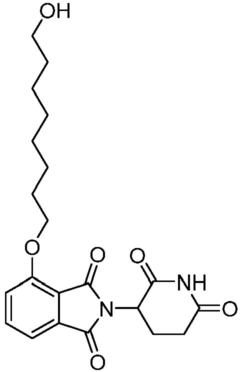

1,8 Octandiol (1.6 g, 10.99 mmol) and triphenylphosphine (1.15 g, 4.36 mmol) were suspended in THF (40 mL), DIAD (0.8 mL, 4.36 mmol) was added dropwise to solution and allowed to stir at 25 °C for 30 mins. 2-(2,6-dioxopiperidin-3-yl)-4-hydroxyisoindoline-1,3-dione (1.00 g, 3.66 mmol) was added in one portion and allowed to stir at 25 °C for 3 h. The reaction mix was then concentrated *in vacuo,* water (30 mL) was added to the crude and then extracted with EtOAc (3 x 20 mL), the organics were dried with MgSO₄, filtered and concentrated *in vacuo.* Ther crude was then taken up in EtOH (30 mL) and ZnCl₂ (1.36 g, 10.99 mmol) was added in one portion and allowed to stir at 25 °C for a further 17 h. The mixture was then filtered to leave 2-(2,6-dioxopiperidin-3-yl)-4-((8-hydroxyoctyl)oxy)isoindoline-1,3-dione (600 mg, 40%). *m*/*z (ES⁺)* = 403.1

### 4-((8-Bromooctyl)oxy)-2-(2,6-dioxopiperidin-3-yl)isoindoline-1,3-dione

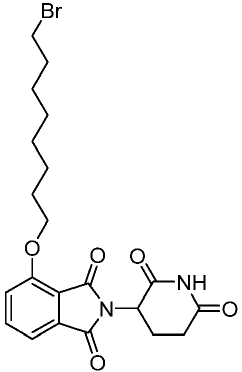

2-(2,6-Dioxopiperidin-3-yl)-4-((8-hydroxyoctyl)oxy)isoindoline-1,3-dione (600 mg, 1.48 mmol), carbon tetrabromide (738 mg, 2.23 mmol) and PPh₃ (596 mg, 2.29 mmol) were suspended in THF (20 mL) and heated to 50 °C for 17 h. Reaction was concentrated *in vacuo,* diluted with H₂O (30 mL) and extracted with EtOAc (3 x 30 mL). The organics were dried with MgSO₄, filtered and concentrated *in vacuo.* The crude was then taken up in EtOH (30 mL) and ZnCl₂ (600 mg, 4.41 mmol) was added in one portion and allowed to stir at 25 °C for a further 17 h. The mixture was then filtered, the filtrate was concentrated and purified via flash column chromatography (hexane/EtOAc(1:5)). The clean fractions were taken concentrated *in vacuo* to leave 4-((8-bromooctyl)oxy)-2-(2,6-dioxopiperidin-3-yl)isoindoline-1,3-dione (70 mg, 10%).

### tert-butyl 4-(4-(8-((2-(2,6-dioxopiperidin-3-yl)-1,3-dioxoisoindolin-4-yl)oxy)octyl)piperazin-1-yl)piperidine-1-carboxylate

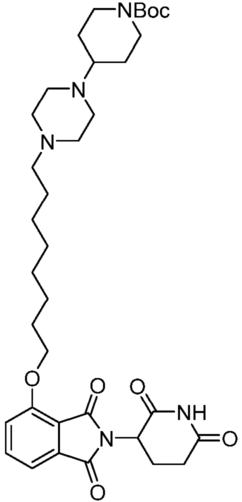

4-((8-Bromooctyl)oxy)-2-(2,6-dioxopiperidin-3-yl)isoindoline-1,3-dione (70 mg, 0.15 mmol), *tert-butyl* 4-(piperazin-1-yl)piperidine-1-carboxylate hydrochloride (45 mg, 0.15 mmol), Cs₂CO₃ (146 mg, 0.45 mmol) and cat. Nal was suspended in MeCN (5 mL) and heated to 80 °C for 17 h. The reaction mix was cooled and concentrated *in vacuo,* the crude was quenched with sat. aq. sodium bicarbonate (15 mL) and extracted with EtOAc (3 x 20 mL) to leave *tert*-butyl 4-(4-(8-((2-(2,6-dioxopiperidin-3-yl)-1,3-dioxoisoindolin-4-yl)oxy)octyl)piperazin-1-yl)piperidine-1-carboxylate (70 mg, 71 %). *m*/*z (ES⁺)* = 654.1.

### tert-butyl 4-(4-(5-(((2S)-1-((4R)-4-hydroxy-2-((4-(4-methylthiazol-5-yl)benzyl)carbamoyl)pyrrolidin-1-yl)-3,3-dimethyl-1-oxobutan-2-yl)amino)-5-oxopentanoyl)piperazin-1-yl)piperidine-1-carboxylate

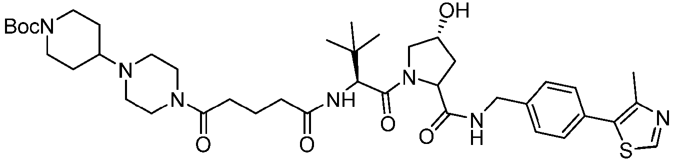

VHL tocris (20 mg, 0.036 mmol), *tert*-butyl 4-(piperazin-1-yl)piperidine-1-carboxylate hydrochloride (12 mg, 0.039 mmol) and HATU (16 mg, 0.043 mmol) were suspended in DMF (1 mL) and DIPEA was added (12.5 µL, 0.072 mmol) and allowed to stir for 1 h at 25 °C. Diluted with EtOAc (10 mL) and washed with brine (3 x 10 mL), dried with MgSO₄, filtered and concentrated *in vacuo.* The crude was purified via basic reverse phase chromatography, and the clean fraction were taken, combined and concentrated *in vacuo* to leave a colourless oil (22 mg, 77%). *m*/*z (ES⁺)* = 810.5

### Method D

Ligase binder (1 equiv.) was solubilised in DCM, TFA (half the amount of DCM) was added slowly and stirred at 25 °C for 2 h. The reaction mix was concentrated *in vacuo.* To this crude material, 3-ethoxy-4-((5-methyl-11-(methylsulfonyl)-6-oxo-6,11-dihydro-5H-benzo[e]pyrimido[5,4-b][1,4]diazepin-2-yl)amino)benzoic (1 equiv.), HATU (3 equiv.) and DIPEA (5 equiv.) were added and suspended in DMF. The reaction mix was stirred for 17 h. Water (10 mL) was added and the extracted with EtOAc (3 x 10 mL), dried onto celite and purified via acidic reverse phase, the clean fractions were combined to leave the desired final compound.

### Example 1: 2-(2,6-Dioxopiperidin-3-yl)-4-(5-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pentyl)isoindoline-1,3-dione (OS1)

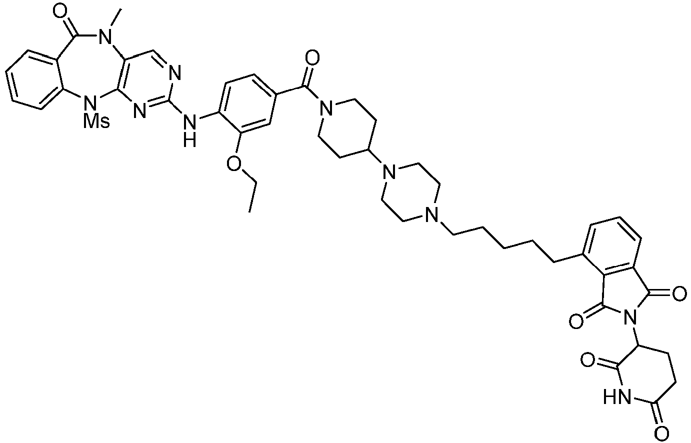

Synthesised using method D. (7 mg, 23 %). mp: 190 - 192 °C (degradation). ¹H NMR (400 MHz, DMSO-*d*₆) δ 11.13 (s, 1H), 9.01 (s, 1H), 8.72 (s, 1H), 7.85 - 7.67 (m, 5H), 7.66 - 7.59 (m, 2H), 7.47 (ddd, *J* = 8.2, 6.3, 2.1 Hz, 1H), 7.06 - 7.03 (m, 1H), 6.97 (dd, *J =* 8.0, 1.7 Hz, 1H), 5.13 (dd, *J* = 12.7, 5.4 Hz, 1H), 4.17 - 3.99 (m, 2H), 3.73 (s, 3H), 3.48 (s, 3H), 3.04 (t, *J* = 7.7 Hz, 3H), 2.89 (ddd, *J* = 16.7, 13.7, 5.3 Hz, 3H), 2.69 - 2.52 (m, 4H), 2.30 (d, *J* = 5.9 Hz, 2H), 2.11-2.01 (m, 1H), 1.80 (s, 2H), 1.68 - 1.57 (m, 2H), 1.45 - 1.29 (m, 4H), 1.24 (t, *J* = 6.8 Hz, 3H). 9 aliphatic protons missing under DMSO. *m*/*z (ES⁺)* = 961.4

### Example 2: 3-(4-(5-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pent-1-yn-1-yl)-1-oxoisoindolin-2-yl)piperidine-2,6-dione (CE207)

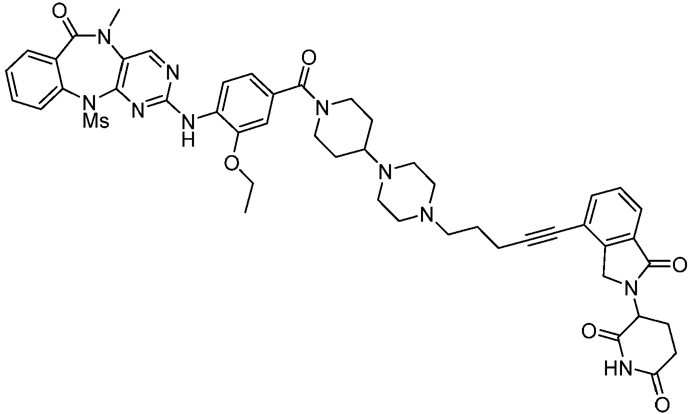

Synthesised using Method D. 10 mg, 14% *m*/*z (ES⁺)* = 941.1
¹H NMR (500 MHz, DMSO-*d*₆) δ 11.03 (s, 1H), 8.99 (s, 1H), 8.73 (s, 1H), 7.83 - 7.68 (m, 3H), 7.55 - 7.42 (m, 2H), 7.04 (s, 1H), 6.98 (d, *J =* 8.1 Hz, 1H), 5.17 (dd, *J* = 13.3, 5.2 Hz, 1H), 4.50 - 4.27 (m, 3H), 4.16 - 4.03 (m, 2H), 3.74 (s, 3H), 3.18 (d, *J* = 4.5 Hz, 1H), 2.93 (ddd, *J* = 17.9, 13.7, 5.5 Hz, 2H), 2.46 - 2.33 (m, 5H), 2.06 - 1.98 (m, 1H), 1.78 - 1.70 (m, 2H), 1.43 - 1.31 (m, 2H), 1.25 (t, *J* = 6.9 Hz, 3H).

### Example 3: 3-(4-(4-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)but-1-yn-1-yl)-1-oxoisoindolin-2-yl)piperidine-2,6-dione (OS3)

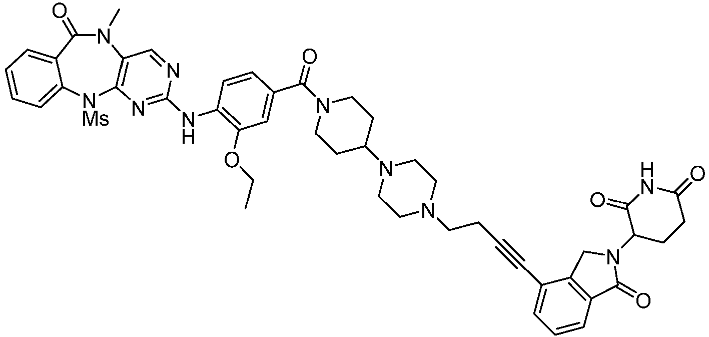

Synthesised using Method D. 24 mg, 23 %. *m*/*z (ES⁺)* = 929.3
¹H NMR (500 MHz, DMSO-*d*₆) δ 11.09 (s, 1H), 9.06 (s, 1H), 8.79 (s, 1H), 8.20 (s, 1H), 7.87 - 7.77 (m, 3H), 7.72 - 7.64 (m, 3H), 7.59 (t, *J* = 7.6 Hz, 1H), 7.53 (ddd, *J* = 8.2, 5.8, 2.7 Hz, 1H), 7.11 (d, *J =* 1.7 Hz, 1H), 7.05 (dd, *J* = 8.1, 1.7 Hz, 1H), 5.23 (dd, *J* = 13.3, 5.1 Hz, 1H), 4.56 - 4.32 (m, 2H), 4.22 - 4.09 (m, 2H), 3.80 (s, 3H), 3.55 (s, 3H), 2.73 - 2.59 (m, 2H), 2.53 - 2.41 (m, 1H), 2.13 (s, 2H), 2.13 - 1.95 (m, 3H), 1.56 (d, *J* = 12.8 Hz, 2H), 1.32 (t, *J* = 6.9 Hz, 3H).

### Example 4: 3-(4-(6-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hex-1-yn-1-yl)-1-oxoisoindolin-2-yl)piperidine-2,6-dione (OS4)

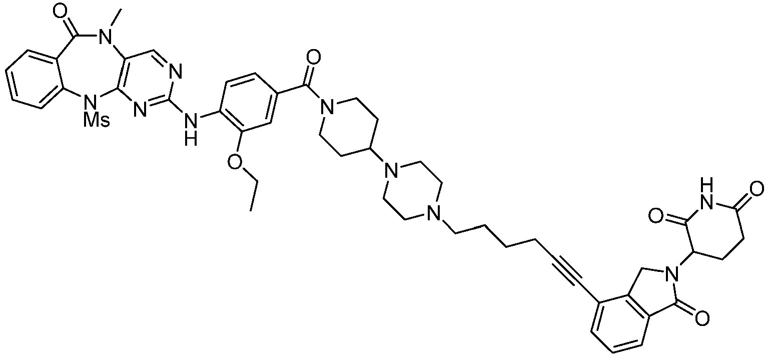

Synthesised using Method D. 7 mg, 18 %. *m*/*z (ES⁺)* = 957.3 ¹H NMR (500 MHz, DMSO-*d*₆) δ 11.02 (s, 1H), 9.01 (s, 1H), 8.73 (s, 1H), 7.81 - 7.71 (m, 2H), 7.67 - 7.60 (m, 2H), 7.54 (t, *J* = 7.6 Hz, 1H), 7.51 - 7.46 (m, 1H), 7.05 (d, *J =* 1.7 Hz, 1H), 6.99 (d, *J =* 8.1 Hz, 1H), 5.17 (dd, *J* = 13.3, 5.1 Hz, 1H), 4.58 - 4.26 (m, 3H), 4.16 - 4.04 (m, 2H), 3.74 (s, 3H), 3.49 (s, 3H), 3.00 - 2.88 (m, 3H), 2.64 - 2.53 (m, 3H), 2.47 - 2.33 (m, 2H), 2.11 - 1.95 (m, 2H), 1.66 - 1.57 (m, 2H), 1.26 (t, *J =* 6.9 Hz, 3H).

### Example 5: 3-(4-(7-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hept-1-yn-1-yl)-1-oxoisoindolin-2-yl)piperidine-2,6-dione (OS6)

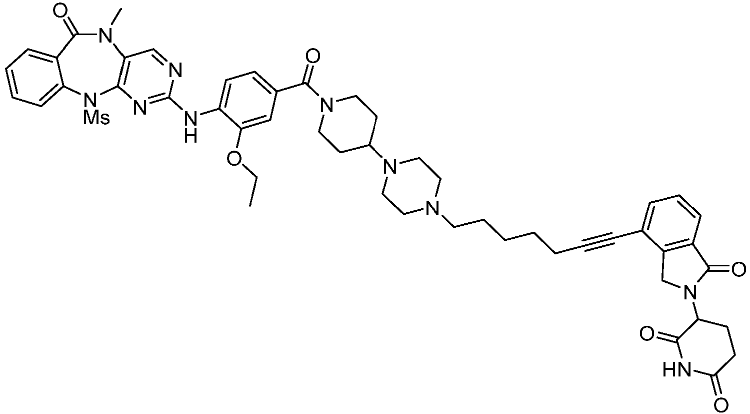

Synthesised using Method D. 4 mg, 10%. *m*/*z (ES⁺)* =971.4. ¹H NMR (500 MHz, DMSO-*d*₆) δ 9.00 (s, 1H), 8.57 (s, 1H), 8.45 (s, 1H), 7.91 (dd, *J* = 27.5, 8.6 Hz, 2H), 7.84 - 7.76 (m, 1H), 7.50 - 7.46 (m, 1H), 7.46 - 7.31 (m, 3H), 7.18 - 7.01 (m, 1H), 5.16 - 5.08 (m, 1H), 4.97 - 4.83 (m, 1H), 4.59 - 4.33 (m, 4H), 4.30 - 4.16 (m, 2H), 3.90 - 3.75 (m, 1H), 3.72 - 3.60 (m, 2H), 3.51 - 3.39 (m, 4H), 2.80 - 2.64 (m, 3H), 2.45 - 2.24 (m, 6H), 2.18 - 1.88 (m, 6H), 1.81 - 1.68 (m, 2H), 1.55 - 1.36 (m, 4H).

### Example 6: 2-(2,6-dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)amino)isoindoline-1,3-dione (OS7)

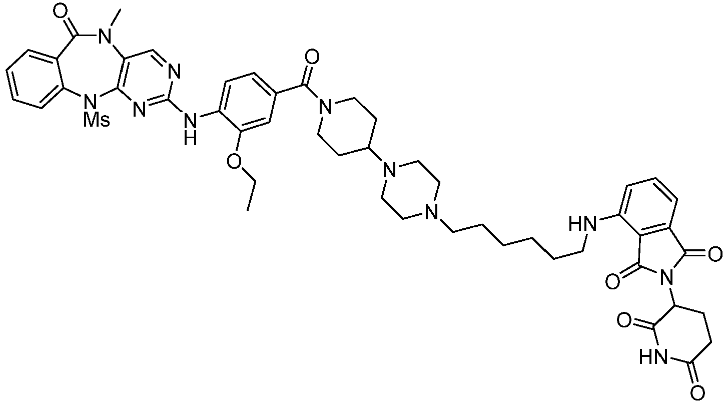

Synthesised using Method D. 11 mg, 63% *m*/*z (ES⁺)* = 990.3
¹H NMR (400 MHz, DMSO-*d*₆) δ 11.10 (s, 1H), 9.00 (s, 1H), 8.73 (s, 1H), 7.80 - 7.55 (m, 6H), 7.51 - 7.44 (m, 1H), 7.13 - 6.94 (m, 4H), 6.54 (t, *J* = 6.0 Hz, 1H), 5.06 (dd, *J* = 12.7, 5.4 Hz, 1H), 4.17 - 4.03 (m, 2H), 3.73 (s, 4H), 3.49 (s, 4H), 3.11 - 2.76 (m, 8H), 1.59 (d, *J =* 7.8 Hz, 4H), 1.44 - 1.28 (m, 9H), 1.26 (t, *J* = 6.9 Hz, 3H).

### Example 7: 3-(4-(3-(2-(2-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)ethoxy)ethoxy)prop-1-yn-1-yl)-1-oxoisoindolin-2-yl)piperidine-2,6-dione (OS9)

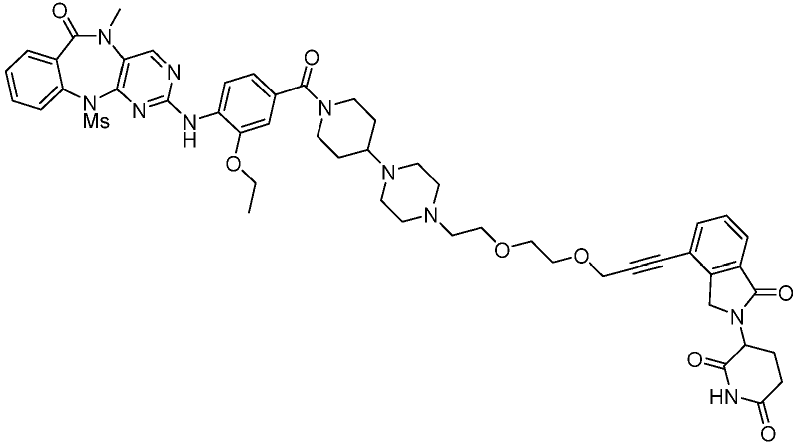

Synthesised using Method D. 9 mg, 10%. *m*/*z (ES⁺)* = 1003.3
¹H NMR (400 MHz, DMSO-*d*₆) δ 11.04 (s, 1H), 9.01 (s, 1H), 8.72 (s, 1H), 8.19 (s, 1H), 7.74 (dd, *J* = 16.5, 7.7 Hz, 3H), 7.65 - 7.42 (m, 4H), 7.04 - 6.93 (m, 2H), 5.15 (dd, *J* = 13.3, 5.1 Hz, 1H), 4.53 - 4.28 (m, 5H), 4.17 - 4.01 (m, 3H), 3.78 - 3.44 (m, 14H), 3.00 - 2.52 (m, 7H), 2.05 - 1.61 (m, 5H), 1.40 - 1.09 (m, 8H).

### Example 8: 3-(4-(8-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)oct-1-yn-1-yl)-1-oxoisoindolin-2-yl)piperidine-2,6-dione (OS10)

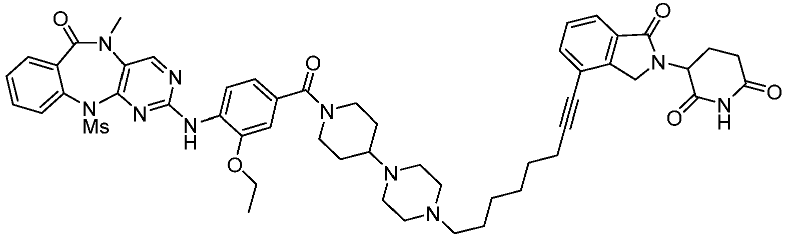

Synthesised using Method D. 5 mg, 8%. *m*/*z (ES⁺)* = 985.4,
¹H NMR (500 MHz, DMSO-*d*₆) δ 11.03 (s, 1H), 9.02 (s, 1H), 8.73 (s, 1H), 8.14 (d, *J =* 8.0 Hz, 1H), 7.96 (s, 1H), 7.81 - 7.69 (m, 3H), 7.66 - 7.59 (m, 3H), 7.56 - 7.45 (m, 2H), 7.04 (d, *J* = 1.7 Hz, 1H), 6.98 (dd, *J* = 8.0, 1.7 Hz, 1H), 5.17 (dd, *J* = 13.4, 5.1 Hz, 1H), 4.49 - 4.27 (m, 3H), 4.16 - 4.05 (m, 2H), 3.74 (s, 3H), 3.49 (s, 3H), 2.90 (s, 4H), 2.74 (s, 3H), 2.65 - 2.55 (m, 2H), 2.47 - 2.31 (m, 2H), 2.10 - 1.99 (m, 2H), 1.83 (s, 3H), 1.63 - 1.52 (m, 4H), 1.50 - 1.29 (m, 6H), 1.25 (t, *J* = 6.9 Hz, 3H).

### Example 9: 3-(4-(9-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)non-1-yn-1-yl)-1-oxoisoindolin-2-yl)piperidine-2,6-dione (OS11)

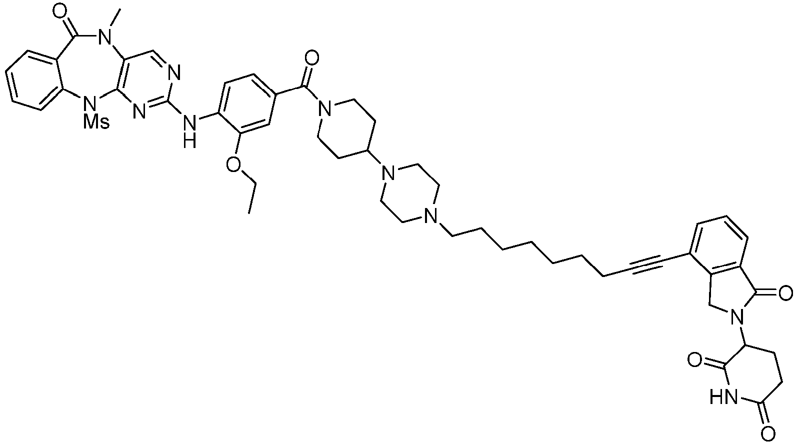

Synthesised using Method D. 40 mg, 60%. *m*/*z (ES⁺)* = 999.3
¹H NMR (500 MHz, DMSO-*d*₆) δ 11.08 (s, 1H), 9.01 (s, 1H), 8.75 (s, 1H), 7.81 - 7.71 (m, 3H), 7.67 - 7.60 (m, 3H), 7.57 - 7.47 (m, 2H), 7.05 (d, *J* = 1.7 Hz, 1H), 6.99 (dd, *J* = 8.1, 1.7 Hz, 1H), 5.17 (dd, *J =* 13.3, 5.1 Hz, 1H), 4.54 - 4.24 (m, 3H), 4.20 - 4.03 (m, 2H), 3.76 (s, 3H), 3.51 (s, 3H), 2.98 - 2.89 (m, 2H), 2.68 - 2.58 (m, 2H), 2.07 - 1.99 (m, 1H), 1.62 - 1.55 (m, 2H), 1.49 - 1.24 (m, 12H).

### Example 10: 2-(2,6-Dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)oxy)isoindoline-1,3-dione (OS12)

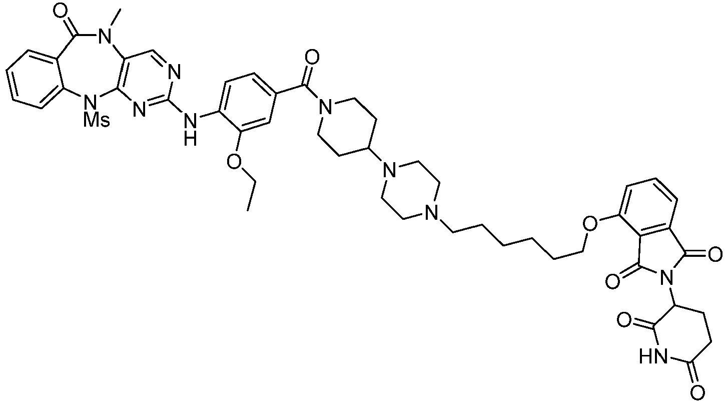

Synthesised using Method D. 3mg. 4% *m*/*z (ES⁺)* = 990.1
¹H NMR (500 MHz, DMSO-*d*₆) δ 9.03 (s, 1H), 8.75 (s, 1H), 8.27 (s, 1H), 7.88 - 7.71 (m, 1H), 7.68 - 7.55 (m, 1H), 7.49 (t, *J* = 7.4 Hz, 1H), 7.23 - 6.91 (m, 3H), 5.07 (dd, *J* = 12.8, 5.5 Hz, 1H), 4.17 - 3.99 (m, 2H), 3.76 (s, 1H), 3.51 (s, 2H), 3.09 - 2.63 (m, 4H), 2.44 - 1.70 (m, 7H), 1.63 - 1.02 (m, 8H), 0.91 - 0.80 (m, 1H).

### Example 11: 2-(2,6-Dioxopiperidin-3-yl)-4-(4-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)butoxy)isoindoline-1,3-dione (OS13)

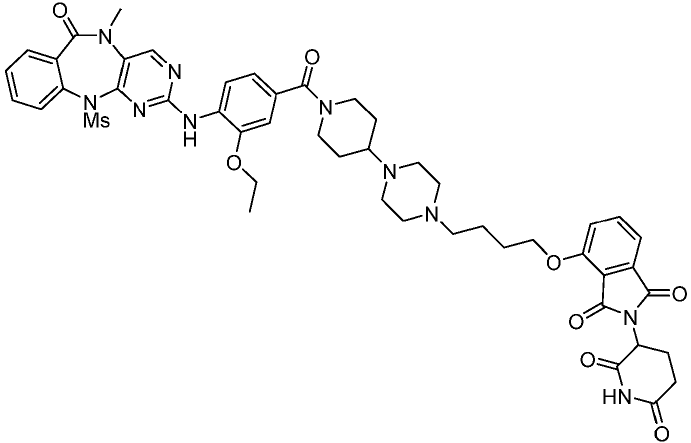

Synthesised using Method D. 7 mg, 11% *m*/*z (ES⁺)* = 991.3
¹H NMR (500 MHz, DMSO-*d*₆) δ 11.12 (s, 1H), 9.01 (s, 1H), 8.73 (d, *J =* 1.6 Hz, 1H), 7.86 - 7.72 (m, 2H), 7.62 (d, *J* = 5.6 Hz, 1H), 7.54 - 7.44 (m, 2H), 7.14 - 6.91 (m, 2H), 5.09 (dd, *J =* 13.0, 5.4 Hz, 1H), 4.22 (t, *J =* 5.8 Hz, 1H), 4.15 - 4.04 (m, 2H), 3.74 (s, 2H), 3.49 (d, *J =* 1.6 Hz, 3H), 3.14 - 2.84 (m, 5H), 2.08 - 1.99 (m, 1H), 1.84 - 1.72 (m, 3H), 1.66 (s, 2H), 1.51 (s, 2H), 1.46 - 1.32 (m, 3H), 1.31 - 1.22 (m, 3H).

### Example 12: 3-(4-(10-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)dec-1-yn-1-yl)-1-oxoisoindolin-2-yl)piperidine-2,6-dione (OS14)

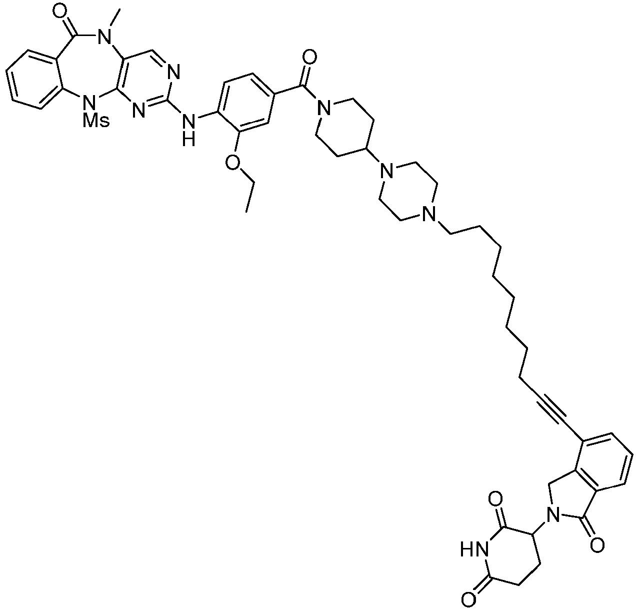

Synthesised using Method D. 12 mg, 15% *m*/*z (ES⁺)* = 1013.2

### Example 13: 2-(2,6-Dioxopiperidin-3-yl)-4-((8-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)octyl)oxy)isoindoline-1,3-dione (OS17)

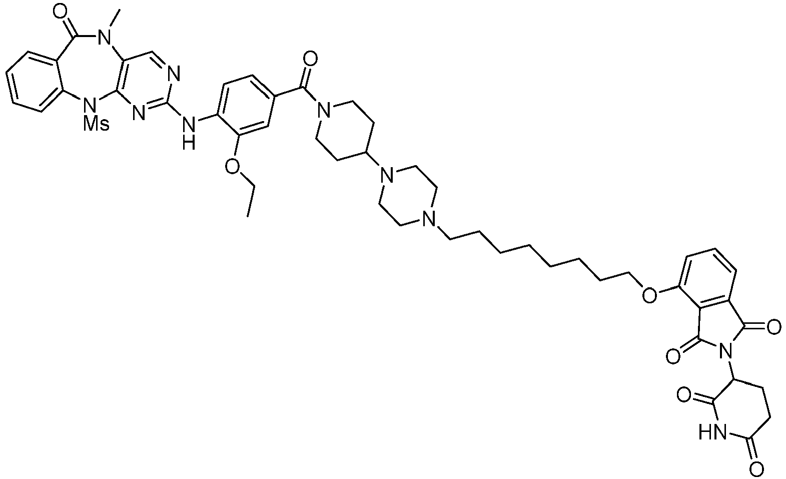

Synthesised using Method D. 40 mg, 39%. *m*/*z (ES⁺)* = 1019.2
¹H NMR (500 MHz, DMSO-*d*₆) δ 11.14 (s, 1H), 9.00 (s, 1H), 8.75 (s, 1H), 7.86 - 7.71 (m, 3H), 7.71 - 7.59 (m, 2H), 7.56 - 7.42 (m, 3H), 7.05 (d, *J* = 1.7 Hz, 1H), 6.99 (dd, *J =* 8.1, 1.7 Hz, 1H), 5.10 (dd, *J* = 12.9, 5.5 Hz, 1H), 4.22 (t, *J =* 6.4 Hz, 2H), 4.12 (dtt, *J* = 16.6, 9.6, 7.0 Hz, 2H), 3.76 (s, 3H), 3.51 (s, 3H), 3.44 - 3.29 (m, 3H), 2.90 (ddd, *J* = 16.9, 14.0, 5.5 Hz, 2H), 2.65 - 2.56 (m, 2H), 2.50 - 2.21 (m, 7H), 2.05 (dtd, *J* = 12.9, 5.2, 2.2 Hz, 1H), 1.77 (p, *J* = 6.5 Hz, 3H), 1.51 - 1.23 (m, 13H).

### BIOLOGICAL EXAMPLES

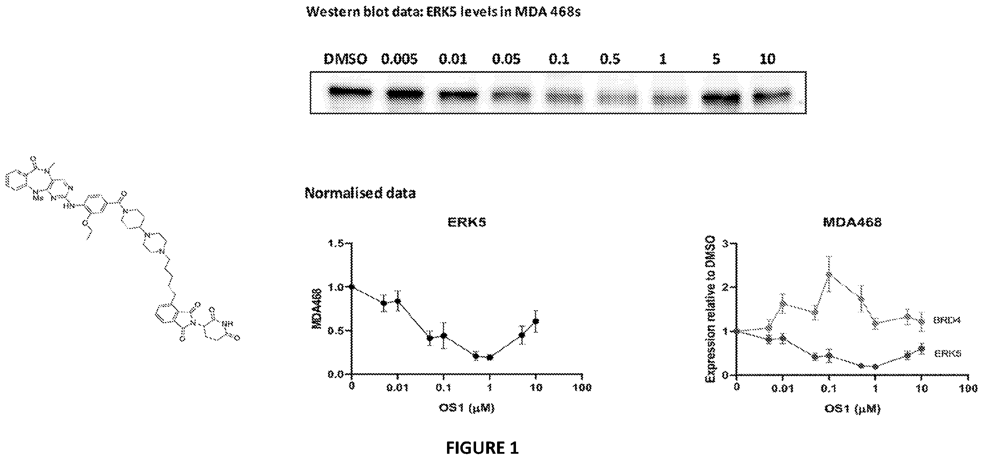

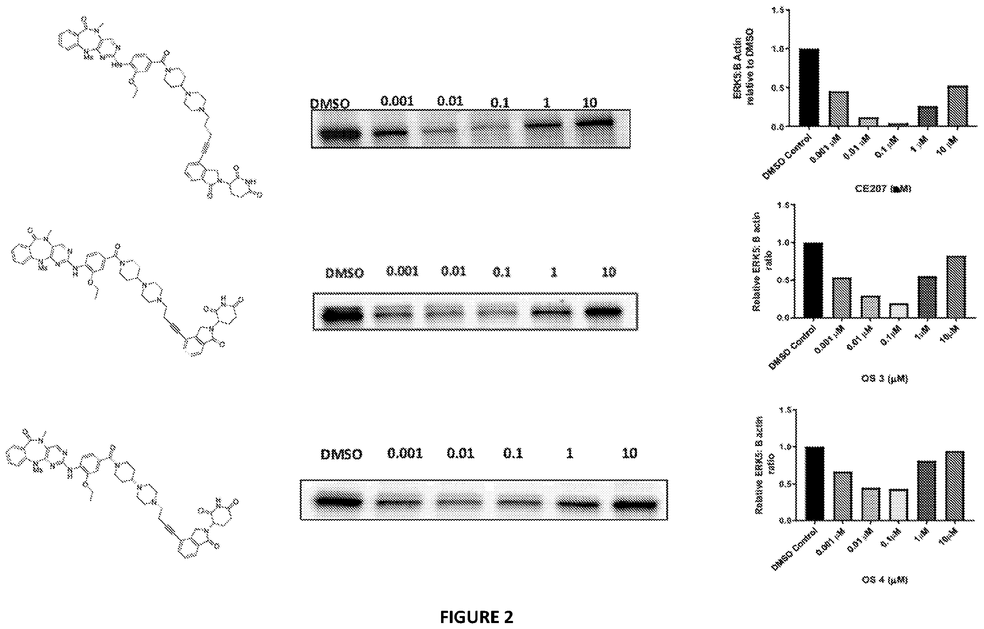

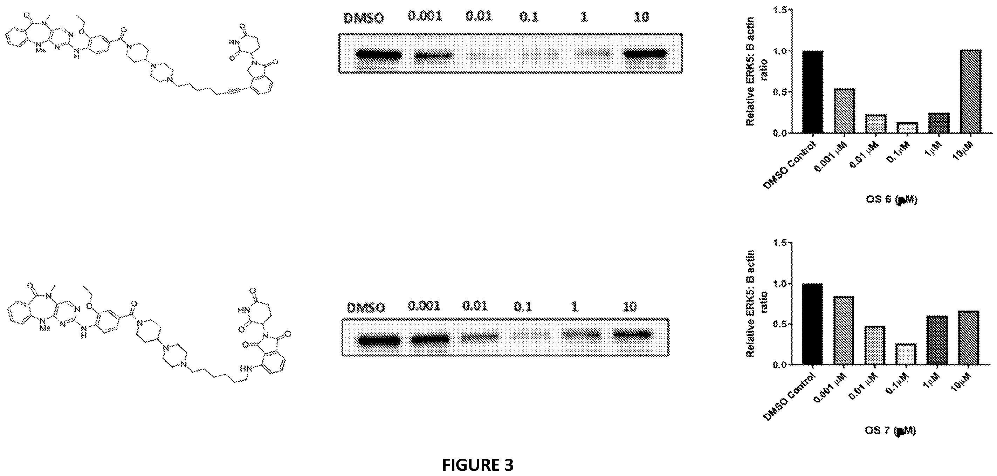

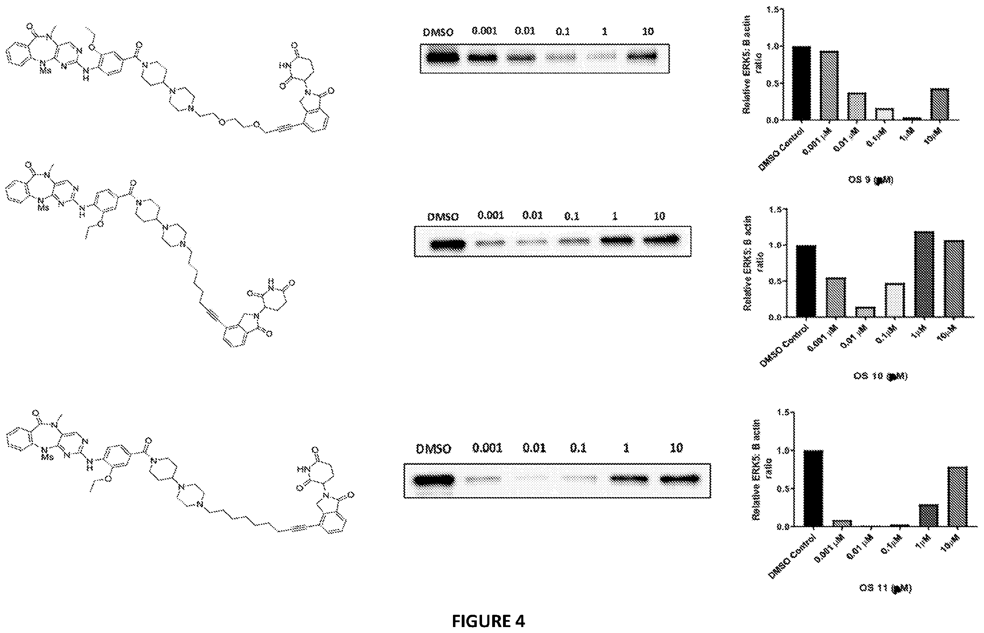

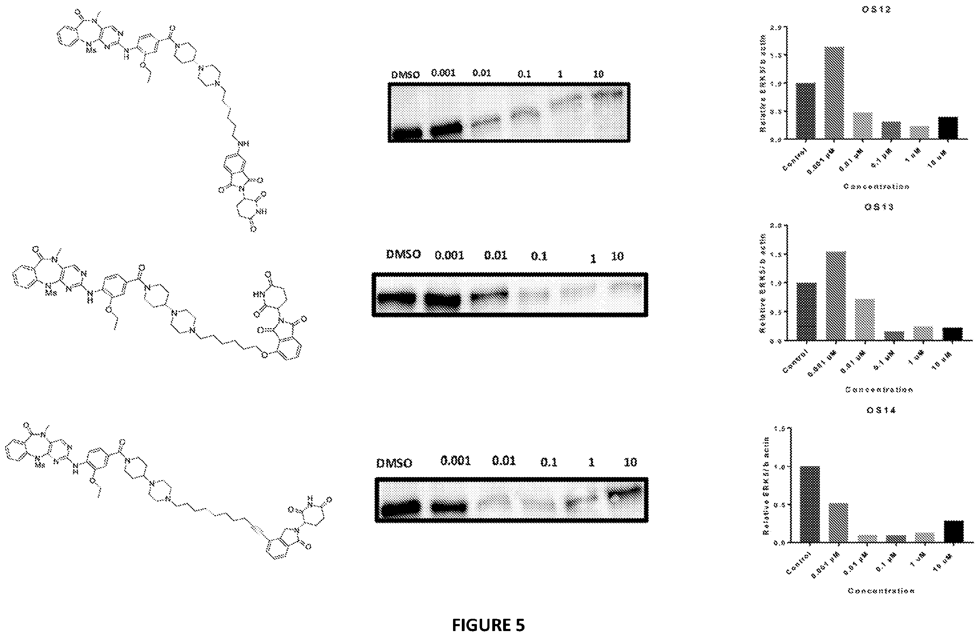

### Activity Data of Example Compounds (Figures 1 to 5)

### Immunoblotting.

Proteins were extracted in RIPA assay buffer containing protease and phosphatase inhibitors. Extracts (30 µg) were resolved by SDS/PAGE and electrophoretically transferred to an Immun-Blot^{®} PVDF membrane (Bio-Rad). Membranes were saturated in 3% non-fat dry milk or 3% BSA and probed overnight at 4 °C with antibodies (1:1,000 dilution unless otherwise indicated) to ERK5 , BRD4, p-ERK5, MEK5, ERK1/2 and β-actin, where indicated. We detected immunocomplexes by enhanced chemiluminescence with IgG coupled to horseradish peroxidase as the secondary anti-rabbit and anti-mouse antibodies (Abcam).

Western blot data showing ERK5 levels in MDA 468s at different concentrations of Examples 1 to 12 is shown in Figures 1 to 5. Graphical representations of this data is provided to demonstrate the expression relative to DMSO control. The right hand graph of Figure 1 also includes expression of ERK5 (bottom line) and BRD4 (upper line).

Graphical representation of immunoblot signals: densitometry of the bands from proteins of interest on immunoblot were measured using ImageJ. Protein levels were calculated relative to loading control (b-actin) and are presented as fold of control - vehicle treated.

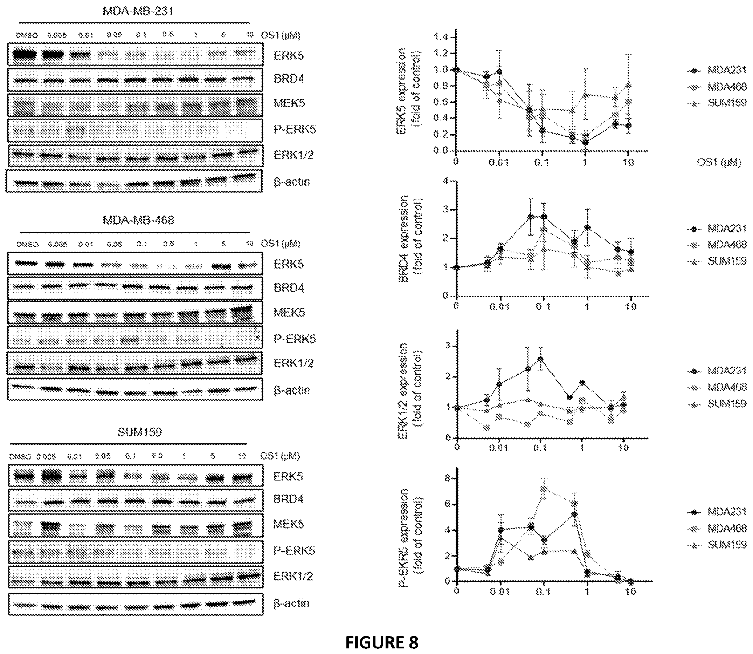

### Specificity Testing - 8 point dose response to OS1: 3 TNBC cell lines (Figure 8)

### Materials and methods

### Immunoblotting.

We extracted proteins in RIPA assay buffer containing protease and phosphatase inhibitors. We resolved extracts (30 µg) by SDS/PAGE and electrophoretically transferred to an Immun-Blot^{®} PVDF membrane (Bio-Rad). Membranes were saturated in 3% non-fat dry milk or 3% BSA and probed overnight at 4 °C with antibodies (1:1,000 dilution unless otherwise indicated) to ERK5, P-ERK5, ERK1/2, BRD4, MEK5 and β-actin. We detected immunocomplexes by enhanced chemiluminescence with IgG coupled to horseradish peroxidase as the secondary anti-rabbit and anti-mouse antibodies (Abcam).

### What the figure shows

Left hand side panels are Western blot images, showing the relative expression of proteins in response to a range of doses of OS1. Right hand side panels are graphs quantifying the western blot images to show ERK5, phospho-ERK5, ERK1/2 and BRD4 protein levels relative to the loading control β-actin.

The data in Figure 8 shows that OS1 is specific and degrades >90% of ERK5 in a range of TNBC cell lines. OS1 has no off target effects on structurally similar proteins (ERK1/2) or known off-targets of literature ERK5 inhibitors (BRD4). OS1 is most effective at 1uM, but doses of 10uM induce the hook effect.

At higher doses OS1 also has kinase inhibitor activity, exemplified by the reduction in p-ERK5 on the immunoblot. This is expected due to the ERK5-targeting warhead being based on ERK5 kinase inhibitors.

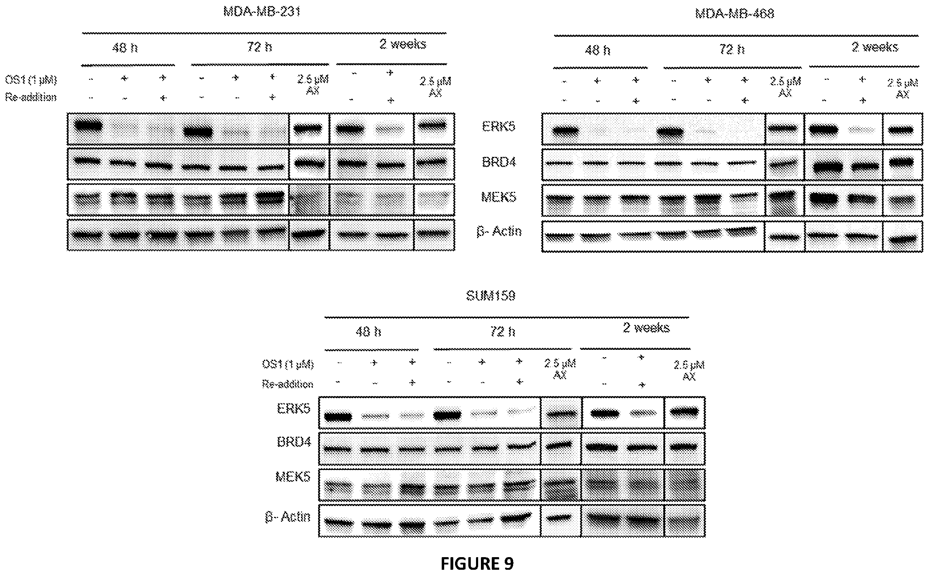

### Repeated dosing of OS1 up to 2 weeks (Figure 9)

### Materials and methods

### Immunoblotting.

We extracted proteins in RIPA assay buffer containing protease and phosphatase inhibitors. We resolved extracts (30 µg) by SDS/PAGE and electrophoretically transferred to an Immun-Blot^{®} PVDF membrane (Bio-Rad). Membranes were saturated in 3% non-fat dry milk or 3% BSA and probed overnight at 4 °C with antibodies (1:1,000 dilution unless otherwise indicated) to ERK5, BRD4, MEK5 and β-actin. We detected immunocomplexes by enhanced chemiluminescence with IgG coupled to horseradish peroxidase as the secondary anti-rabbit and anti-mouse antibodies (Abcam).

### What the figure shows

The data in Figure 9 shows that repeated dosing of OS1 every 48h does not affect the specificity of OS1.

Repeated dosing was performed for up to two weeks and this had no effect on either ERK1/2 (structurally similar kinase) or BRD4 (known off target of literature kinase inhibitors). MEK5 the upstream activator of ERK5 is also unaffected by repeated dosing of OS1.

We can conclude from this that all data using OS1 will be a result of specific loss of ERK5.

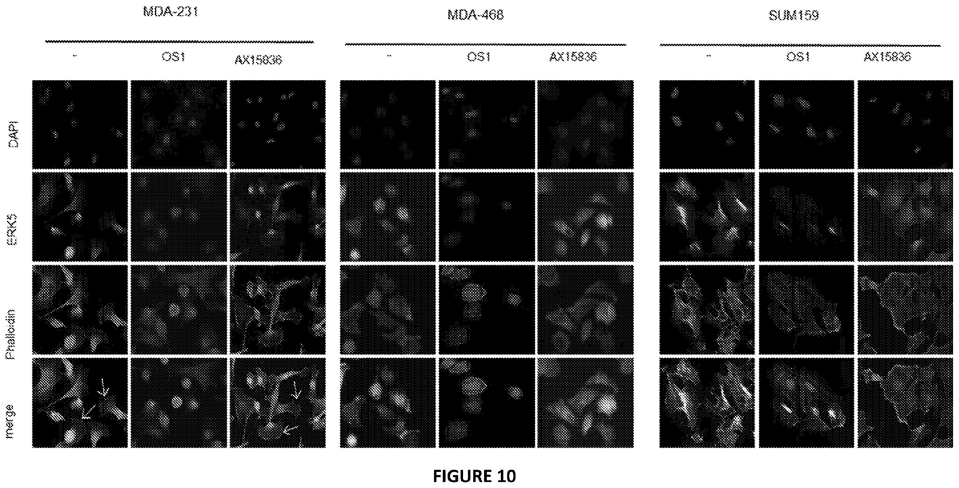

### Morphology of TNBC cells (Figure 10)

### Materials and methods

Cells were seeded and treated. Media was aspirated, cells were washed with PBS and subsequently fixed in 10% formalin for 10 minutes. Fixed cells were washed in PBS and then permeablised with 0.1% (v/v) Triton-100 in PBS for 10 minutes. Cells were washed with PBS and the non-specific binding sites were blocked by incubating with 1% BSA for 30 minutes. Targets of interested were identified by incubating with 100µl of primary antibody diluted in 1% BSA (see table) overnight at 4°C. After washing with PBS, samples were further incubated with species specific secondary antibody coupled to fluorescent tags (see antibody table) for 1 hour in the dark at room temperature. Some samples were further incubated with 100µl of 1:200 phalloidin (Invitrogen, A12381) in 1% BSA for 2 hours at room temperature to identify actin filament structures. The EZ-slides casing was dismantled as described in manufacturer's instruction and slides were washed in distilled H₂O. Samples were incubated with one drop of Mounting Medium with DAPI (abcam) for 5 minutes in the dark at room temperature to visualise the nuclei. A coverslip was added to each slide and allowed to dry at room temperature. Images were acquired on a 3D-Histech Pannoramic-250 microscope slidescanner using a 20x/ 0.80 Plan Apochromat objective (Zeiss) and the DAPI, FITC and TRITC filter sets. Snapshots of the slide-scans were taken using the Case Viewer software (3D-Histech). Images were then processed and analysed using ImageJ.

### What the figure shows

Immunoflorescence staining in three triple negative breast cancer cell lines: MDA-MB-231, MDA-MB-468 and SUM159.

Figure 10 shows that OS1 affects morphology of TNBC cells, whereas AX15836 (kinase inhibition does not). This data is congruent with data from shERK5 and CRISPR studies in TNBC, where loss of ERK5 leads to morphological changes. Morphological changes observed here are indicative of a loss of migratory and metastatic capacity.

Reaffirms that OS1 effectively degrades ERK5, as indicated by a loss of ERK5 staining (green) in OS1 treated cells.

Treatment with OS1 induces morphological changes in TNBC cell lines, which specific kinase inhibitors fail to recapitulate (AX15836).

These morphological changes are congruent with studies in TNBC where EKR5 is knocked down or eliminated via genetic and siRNA approaches.

The changes in morphology seen are consistent with a switch to less migratory, less metastatic phenotype in TNBC cells. Morphological changes akin to that seen here occur with siRNA/CRISPR knockdown ERK5 studies and in these studies they mapped directly to an anti-metastatic phenotype in in vivo pre-clinical models of TNBC. Therefore, we can extrapolate that OS1 will have anti-metastatic effects *in vivo.*

In contrast, specific kinase inhibitors cannot recapitulate this phenotype and are likely to be ineffective in vivo to prevent metastases of TNBC.

Therefore the degradation approach is the most appropriate way to target ERK5 for therapeutic gain in TNBC.

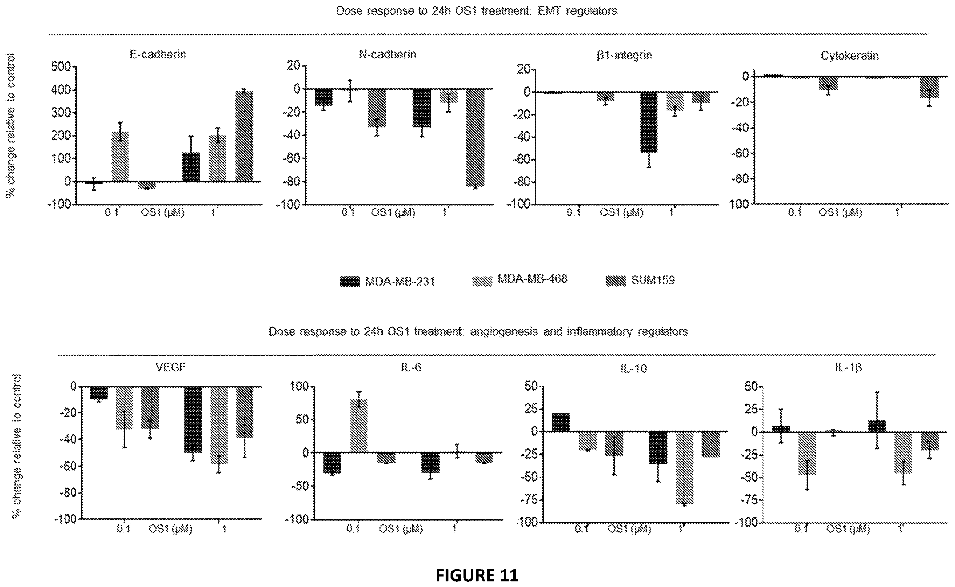

### qRT-PCR in response to OS1 treatment for 24h (Figure 11)

### Materials and methods

**qRT-PCR:** Total RNA was isolated from cells using TRIZOL and/or the miRNeasy mini kit (Qiagen). We carried out cDNA synthesis as previously described by Green, D., Eyre, H., Singh, A. et al (Targeting the MAPK7/MMP9 axis for metastasis in primary bone cancer. Oncogene 39, 5553-5569 (2020)). We performed qPCR using the SYBR Green I core kit (Eurogentec). PCR products were detected in the ABI PRISM^{®} 7700 sequence detection system (Thermo Fisher Scientific). We analysed results using the 2-ΔΔG method. Gene expression was normalised to *PGK1* or *ACTB.*

### What the figure shows

Relative mRNA levels, compared to internal control PGK1 or ACTB, in three triple negative breast cancer cell lines: MDA-MB-231, MDA-MB-468 and SUM159.

The data in Figure 11 shows that loss of ERK5, via OS1, leads to a loss of expression of genes that promote EMT, angiogenesis and pro-tumour inflammation

Treatment with OS1, but not a specific ERK5 kinase inhibitor (AX15836), induces a loss of expression of genes that promote EMT, migration and metastatic behaviour (N-cadherin, cytokeratin and B1-integrin), angiogenesis (VEGF) and pro-tumour inflammation (IL6, IL1b).

Conversely, ERK5 degradation (via OS1) causes an increase in expression of genes that block EMT, migration and metastatic behaviour (E-cadherin).

Doses of 1uM OS1 are most effective in affecting gene expression and therefore we conclude that >80% loss of ERK5 protein is needed to elicit maximal phenotypic effects in TNBC cell lines.

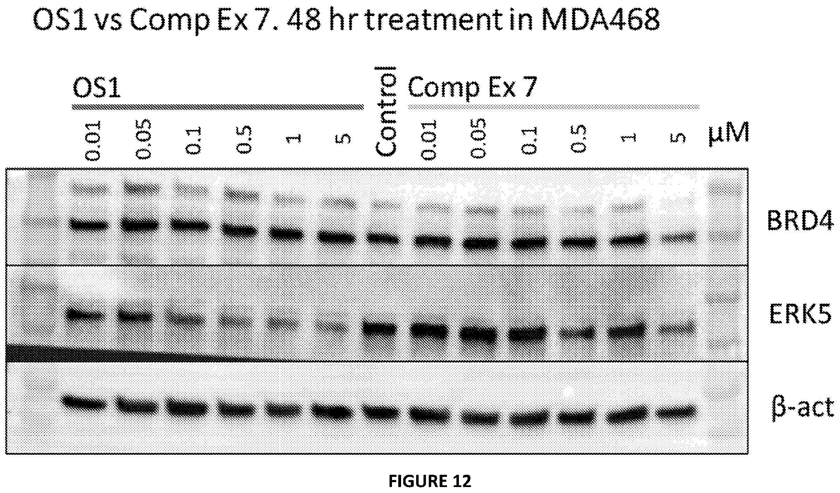

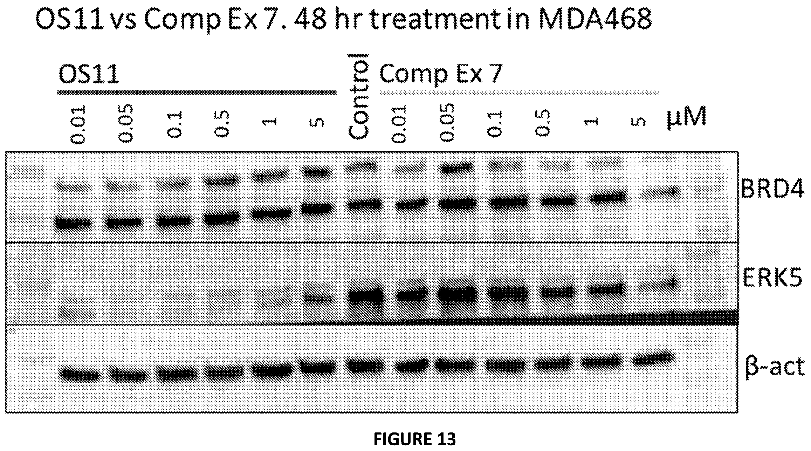

### Comparison of OS1 and OS11 to Example 7 of WO2021061894 (Figures 12 and 13)

### Materials and methods

### Immunoblotting

We extracted proteins in RIPA assay buffer containing protease and phosphatase inhibitors. We resolved extracts (30 µg) by SDS/PAGE and electrophoretically transferred to an Immun-Blot^{®} PVDF membrane (Bio-Rad). Membranes were saturated in 3% non-fat dry milk or 3% BSA and probed overnight at 4 °C with antibodies (1:1,000 dilution unless otherwise indicated) to ERK5, BRD4, MEK5 and β-actin. We detected immunocomplexes by enhanced chemiluminescence with IgG coupled to horseradish peroxidase as the secondary anti-rabbit and anti-mouse antibodies (Abcam).

### What the figure shows

The data in Figures 12 and 13 shows western blot of ERK5 and BRD4 levels, relative to b-actin, 48 hours after treatment with a single dose of the ERK5-PROTACs: OS1 (Figure 12), OS11 (Figure 13) and Example 7 of WO2021061894 (labelled hereinafter as "Comparative Example 7" (Comp Ex 7)) in MDA-MB-468 cells. OS1 and OS11 degrade EKR5 at lower concentrations than Comparative Example 7. OS1 is efficacious at degrading ERK5 at doses of 0.1µM and OS11 at 0.01 µM. In contrast, a dose of 5µM of Comparative Example 7 is required to achieve analogous ERK5 degradation to 0.1µM OS1 or 0.01 µM OS11. OS1 or OS11 do not cause degradation of the known off-target of ERK5-targeted agents: BRD4. However, Comparative Example 7 causes concurrent degradation of BRD4 at the dose required to degrade ERK5 (5 µM of Comparative Example 7). Therefore, both OS1 and OS11 are more potent and specific than ERK5-PROTAC compound Comparative Example 7.

### Conclusions

The biological examples provided herein demonstrate that an ERK5-PROTAC approach is viable. The compounds of the invention are specific and potent against ERK5. Furthermore, the data demonstrates:
- The compounds of the invention can induce an anti-migratory and anti-metastatic phenotype in TNBC cells, whereas kinase inhibition cannot.
- The compounds of the invention can prevent pro-tumour inflammatory signalling in TNBC cells, whereas kinase inhibition cannot.
- The compounds of the invention, unlike ERK5-kinase inhibitors, can recapitulate the anti-cancer effects seen from pre-clinical cancer studies that used genetic or siRNA approaches to target ERK5.
- The ERK5 degradation approach is the most appropriate way to target ERK5 for therapeutic gain in multiple cancers of unmet need.

### In vivo efficacy of OS11 in immune-competent models of TNBC

### Materials and methods

### In vivo efficacy testing

8-10 week old Balb/c mice were implanted orthotopically (mammary fat pad) with the murine TNBC cell line EMT6. Tumours were measured by caliper and once tumours reached 100-150mm³ mice were randomised into 4 treatment groups: Vehicle control, PD1-inhibitor alone, OS11 alone (20mg/kg) and Combination treatment. The experiment was conducted blind. Tumours were allowed to grow until end-point <1000mm³ unless adverse phenotype due to metastases was observed, which required earlier end-point. At end point terminal blood samples and tumours were collected for analysis.

### Immunoblotting

We extracted proteins from terminal blood samples and *ex vivo* biopsis of tumour in RIPA assay buffer containing protease and phosphatase inhibitors. We resolved extracts (30 µg) by SDS/PAGE and electrophoretically transferred to an Immun-Blot^{®} PVDF membrane (Bio-Rad). Membranes were saturated in 3% non-fat dry milk or 3% BSA and probed overnight at 4 °C with antibodies (1:1,000 dilution unless otherwise indicated) to ERK5 and β-actin. We detected immunocomplexes by enhanced chemiluminescence with IgG coupled to horseradish peroxidase as the secondary anti-rabbit and anti-mouse antibodies (Abcam).

### What the figure shows

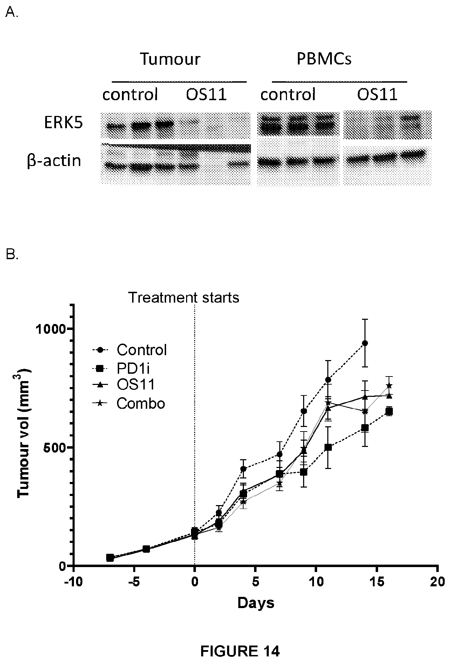

The data in Figure 14.A shows the effective knockdown of ERK5 by OS11 *in vivo* both in the tumour compartment and in PBMCs (derived from terminal blood samples). Data in Figure 14.B shows that OS11 has an anti-tumour effect equal to that of PD1-inhibition and that combinatorial treatment of OS11 with PD1 inhibition has no further effect beyond that of either agent alone.

### Conclusions

The compounds of the invention are effective at degrading ERK5 *in vivo,* both in the tumour compartment and in peripheral blood and are as effective as an anti-cancer agent as PD1-inhibitors, in orthotopic, immune-competent models of TNBC.

## Claims

1. A compound, or a pharmaceutically acceptable salt, hydrate or solvate thereof, having the structural Formula (I), shown below: 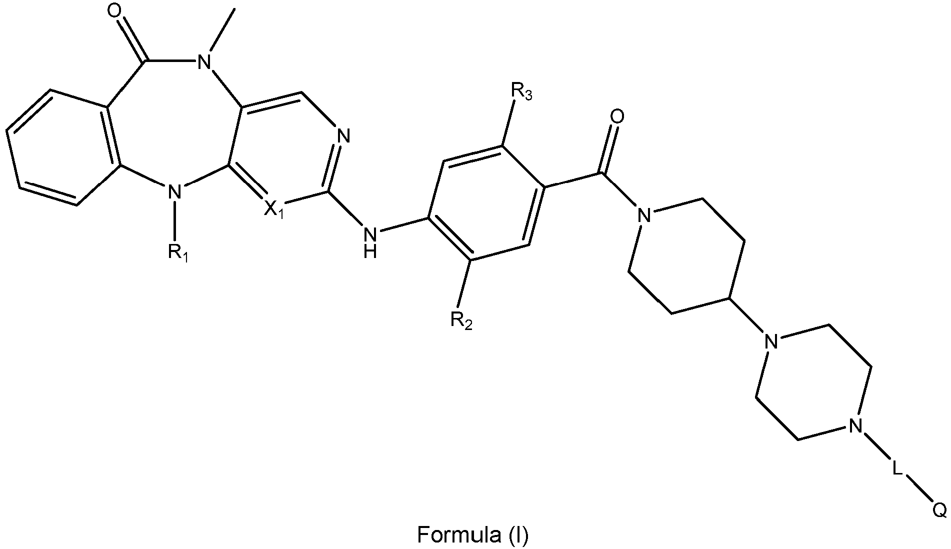 wherein
R₁ is -S(O)₂-(1-3C)alkyl;
R₂ is (1-4C)alkoxy;
R₃ is selected from hydrogen, fluoro, methyl, methoxy, trifluoromethyl, or trifluoromethoxy;
X₁ is N or CH;
L is a linker having the formula: 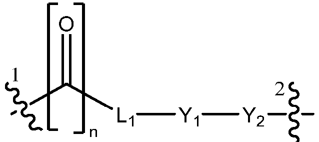 wherein: 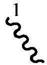
denotes the point of attachment to the N atom of the piperazine ring; 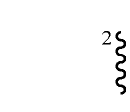
denotes the point of attachment to Q;
n is 0 or 1;
L₁ is a (2-10C)alkylene linker or a PEG chain having the formula
-[CH₂CH₂-O]₁₋₃-[CH₂]₁₋₃-;
Y₁ and Y₂ together form:
an ethynylene group (-C≡C-);
an ethylene group (-CH=CH-);
-CH₂-O-;
-CH₂-NH-;
or
-CH₂-CH₂-;
Q is selected from:
(i) 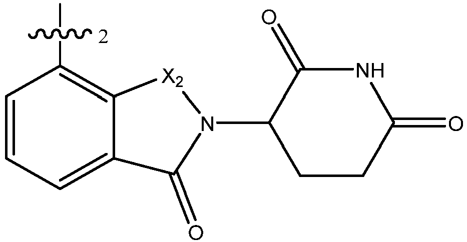
(ii) 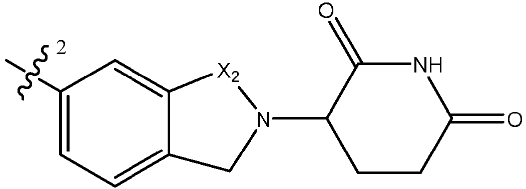
(iii) 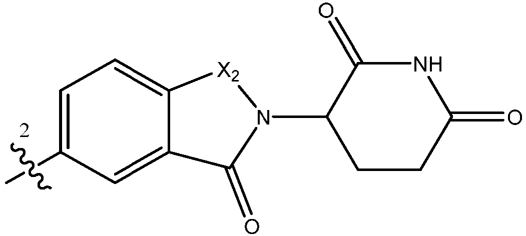 wherein: 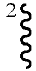
denotes the point of attachment to Y₂ or group L;
X₂ is selected from -CH₂- or -C(O)-; with the proviso that X₂ is not -C(O)- when Y₁and
Y₂ together form an ethynylene group (-C≡C-).

2. A compound, or a pharmaceutically acceptable salt, hydrate or solvate thereof, according to claim 1, wherein R₁ is -S(O)₂-CH₃.

3. A compound, or a pharmaceutically acceptable salt, hydrate or solvate thereof, according to claim 1 or claim 2, wherein any one or both of the following statements (A)-(B) applies:
(A) R₂ is (1-3C)alkoxy;
(B) R₂ is ethoxy.

4. A compound, or a pharmaceutically acceptable salt, hydrate or solvate thereof, according to any one of claims 1 to 3, wherein any one or both of the following statements (A)-(B) applies:
(A) R₃ is selected from hydrogen, fluoro or methyl;
(B) R₃ is hydrogen.

5. A compound, or a pharmaceutically acceptable salt, hydrate or solvate thereof, according to any one of claims 1 to 4, wherein X₁ is N.

6. A compound, or a pharmaceutically acceptable salt, hydrate or solvate thereof, according to any one of the preceding claims, having the structural formula (la) shown below: 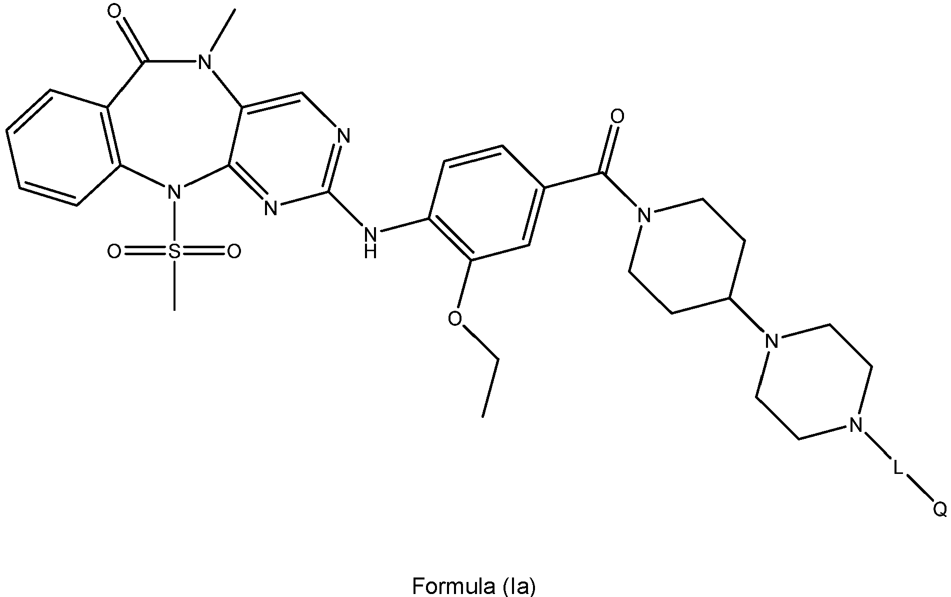 wherein L and Q are as defined in claim 1.

7. A compound, or a pharmaceutically acceptable salt, hydrate or solvate thereof, according to any one of claims 1 to 6, wherein any one or more of the following statements (A)-(H) applies:
(A) L is a linker having the formula: 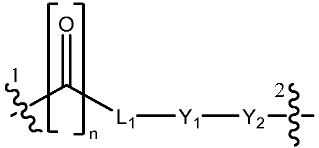 wherein: 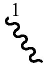
denotes the point of attachment to the N atom of the piperazine ring; 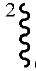
denotes the point of attachment to Q;
n is 0 or 1;
L₁ is a (2-8C)alkylene linker or a PEG chain having the formula
-[CH₂CH₂-O]₁₋₃-[CH₂]₁₋₂-;
and
Y₁ and Y₂ together form:
an ethynylene group (-C≡C-);
-CH₂-O-;
-CH₂-NH-;
or
-CH₂-CH₂-;
(B) L is a linker having the formula: 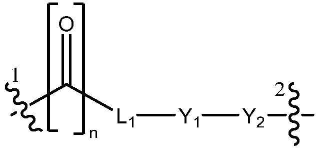 wherein: 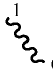
denotes the point of attachment to the N atom of the piperazine ring; 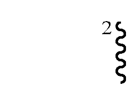
denotes the point of attachment to Q;
n is 0 or 1;
L₁ is a (2-8C)alkylene linker or a PEG chain having the formula
-[CH₂CH₂-O]₁₋₂-[CH₂]₁₋₂-;
and
Y₁ and Y₂ together form:
an ethynylene group (-C≡C-);
-CH₂-O-;
-CH₂-NH-;
or
-CH₂-CH₂-;
(C) L is a linker having the formula: 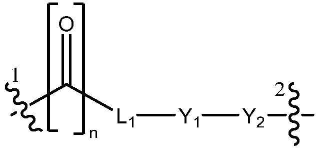 wherein: 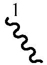
denotes the point of attachment to the N atom of the piperazine ring; 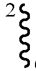
denotes the point of attachment to Q;
n is 0 or 1;
L₁ is a (2-8C)alkylene linker; and
Y₁ and Y₂ together form:
an ethynylene group (-C≡C-);
-CH₂-O-;
-CH₂-NH-;
or
-CH₂-CH₂-;
(D) L is a linker having the formula: 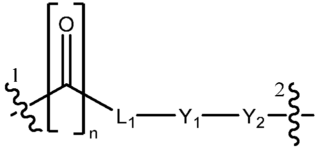 wherein: 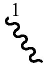
denotes the point of attachment to the N atom of the piperazine ring; 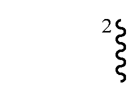
denotes the point of attachment to Q;
n is 0;
L₁ is a (2-8C)alkylene linker or a PEG chain having the formula
-[CH₂CH₂-O]₁₋₂-[CH₂]₁₋₂-;
and
Y₁ and Y₂ together form:
an ethynylene group (-C≡C-);
-CH₂-O-;
-CH₂-NH-;
or
-CH₂-CH₂-;
(E) L is a linker having the formula: 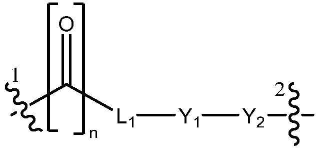 wherein: 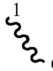
denotes the point of attachment to the N atom of the piperazine ring; 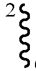
denotes the point of attachment to Q;
n is 0 or 1;
L₁ is a (2-8C)alkylene linker or a PEG chain having the formula
-[CH₂CH₂-O]₁₋₂-[CH₂]₁₋₂-;
and
Y₁ and Y₂ together form:
an ethynylene group (-C≡C-);
(F) L is a linker having the formula: 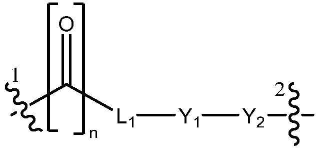 wherein: 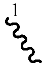
denotes the point of attachment to the N atom of the piperazine ring; 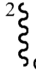
denotes the point of attachment to Q;
n is 0;
L₁ is a (3-8C)alkylene linker; and
Y₁ and Y₂ together form:
an ethynylene group (-C≡C-);
-CH₂-O-;
-CH₂-NH-;
or
-CH₂-CH₂-;
(G) L is a linker having the formula: 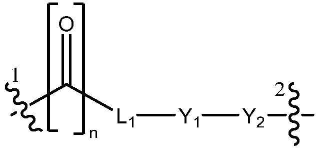 wherein: 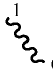
denotes the point of attachment to the N atom of the piperazine ring; 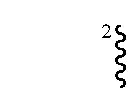
denotes the point of attachment to Q;
n is 0;
L₁ is a (3-8C)alkylene linker; and
Y₁ and Y₂ together form:
an ethynylene group (-C≡C-);
(H) L is a linker having the formula: 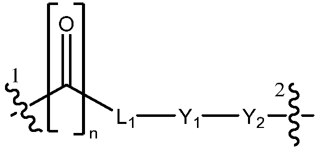 wherein: 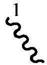
denotes the point of attachment to the N atom of the piperazine ring; 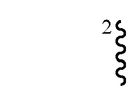
denotes the point of attachment to Q;
n is 0;
L₁ is a (5-8C)alkylene linker; and
Y₁ and Y₂ together form:
an ethynylene group (-C≡C-).

8. A compound, or a pharmaceutically acceptable salt, hydrate or solvate thereof, according to any one of claims 1 to 7, wherein L is a linker having a formula selected from: 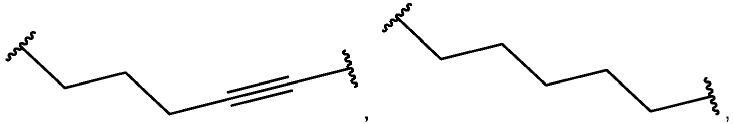 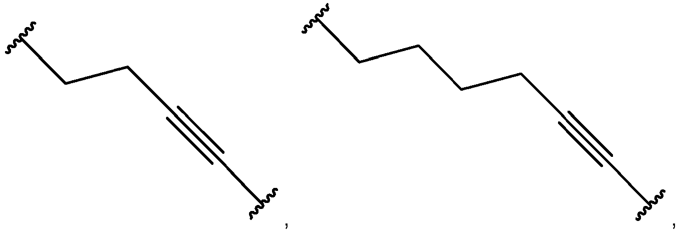 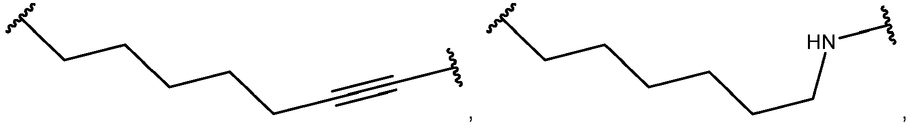 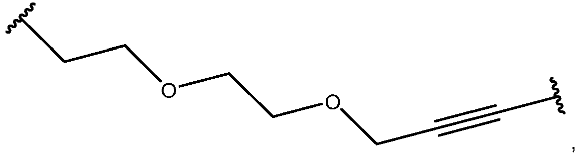 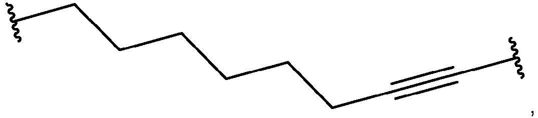 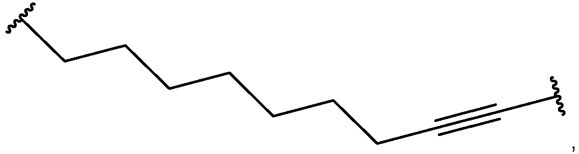 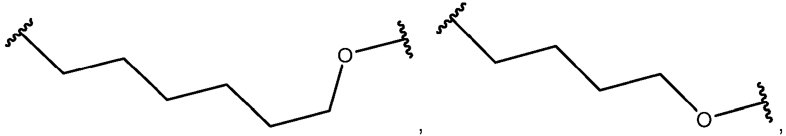 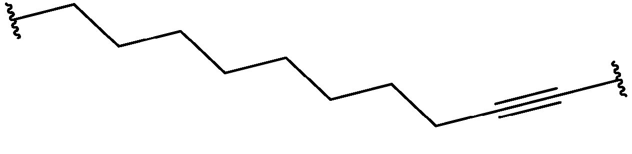 or

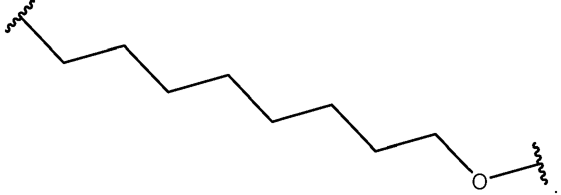

9. A compound, or a pharmaceutically acceptable salt, hydrate or solvate thereof, according to any one of claims 1 to 8, wherein Q is selected from:
(i) 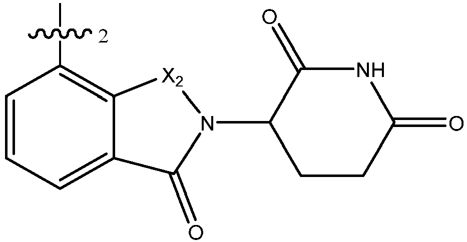 or
(ii) 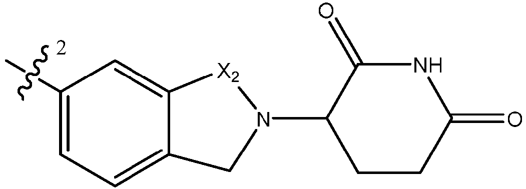
wherein X₂ is as defined in claim 1.

10. A compound, or a pharmaceutically acceptable salt, hydrate or solvate thereof, according to any one of claims 1 to 9, wherein Q is: 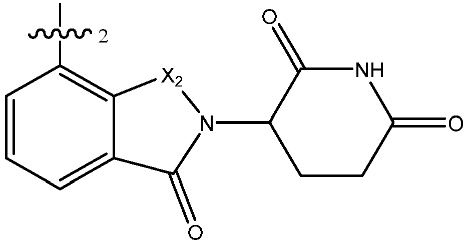 wherein X₂ is as defined in claim 1.

11. A compound, or a pharmaceutically acceptable salt, hydrate or solvate thereof, according to any one of claims 1 to 10, wherein X₂ is -CH₂-.

12. A compound according to claim 1, wherein the compound is selected from:
2-(2,6-Dioxopiperidin-3-yl)-4-(5-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pentyl)isoindoline-1,3-dione;
3-(4-(5-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pent-1-yn-1-yl)-1-oxoisoindolin-2-yl)piperidine-2,6-dione;
3-(4-(4-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)but-1-yn-1-yl)-1-oxoisoindolin-2-yl)piperidine-2,6-dione;
3-(4-(6-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hex-1-yn-1-yl)-1-oxoisoindolin-2-yl)piperidine-2,6-dione;
3-(4-(7-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hept-1-yn-1-yl)-1-oxoisoindolin-2-yl)piperidine-2,6-dione;
2-(2,6-dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)amino)isoindoline-1,3-dione;
3-(4-(3-(2-(2-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)ethoxy)ethoxy)prop-1-yn-1-yl)-1-oxoisoindolin-2-yl)piperidine-2,6-dione;
3-(4-(8-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)oct-1-yn-1-yl)-1-oxoisoindolin-2-yl)piperidine-2,6-dione;
3-(4-(9-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)non-1-yn-1-yl)-1-oxoisoindolin-2-yl)piperidine-2,6-dione;
2-(2,6-Dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)oxy)isoindoline-1,3-dione;
2-(2,6-Dioxopiperidin-3-yl)-4-(4-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)butoxy)isoindoline-1,3-dione;
3-(4-(10-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)dec-1-yn-1-yl)-1-oxoisoindolin-2-yl)piperidine-2,6-dione;
2-(2,6-Dioxopiperidin-3-yl)-4-((8-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)octyl)oxy)isoindoline-1,3-dione;
or a pharmaceutically acceptable salt, hydrate or solvate thereof.

13. A pharmaceutical composition comprising a compound according to claims 1 to 12, or a pharmaceutically acceptable salt or solvate thereof, in admixture with a pharmaceutically acceptable diluent or carrier.

14. A compound as defined in any one of claims 1 to 12, or a pharmaceutically acceptable salt or solvate thereof, or a pharmaceutical composition as defined in claim 13, for use in therapy.

15. A compound as defined in any one of claims 1 to 12, or a pharmaceutically acceptable salt or solvate thereof, or a pharmaceutical composition as defined in claim 13, for use in the treatment of:
a) a fibrotic disease, e.g. impaired wound healing, pulmonary fibrosis,
b) an Inflammatory disease e.g. psoriasis, asthma,
c) a proliferative condition, e.g. cancer, such as triple-negative breast cancer, mesothelioma, primary bone cancer, glioblastoma, lung cancer, squamous cell carcinoma,
d) diabetes,
e) pain, or
f) a central nervous system (CNS) disorder e.g. Parkinsons disease, dementia; optionally wherein the compound is for use in the treatment of cancer.

## Patentansprüche

1. Verbindung oder pharmazeutisch unbedenkliches Salz, Hydrat oder Solvat davon mit der nachstehend dargestellten Strukturformel (I): 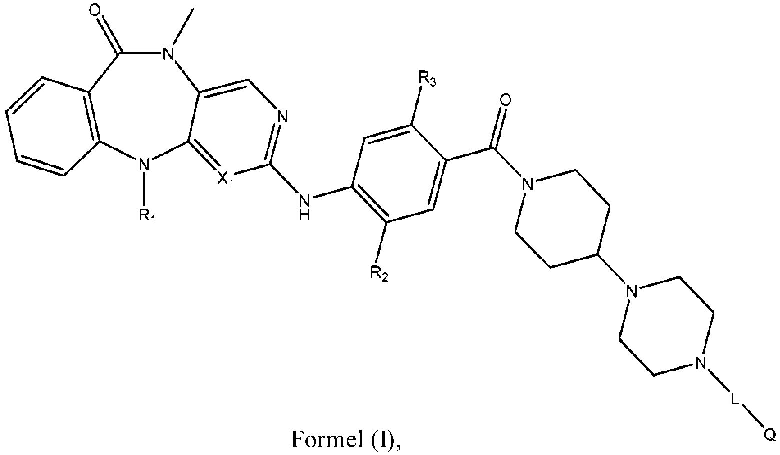 wobei
R₁ -S(O)₂-(1-3C)-Alkyl ist;
R₂ (1-4C)-Alkoxy ist;
R₃ ausgewählt ist aus Wasserstoff, Fluor, Methyl, Methoxy, Trifluormethyl oder Trifluormethoxy;
X₁ N oder CH ist;
L ein Linker mit der folgenden Formel ist: 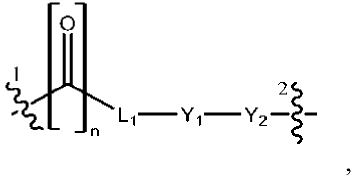
wobei: 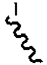
den Punkt der Anbindung an das N-Atom des Piperazinrings bezeichnet; 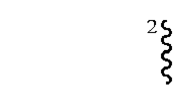
den Punkt der Anbindung an Q bezeichnet;
n 0 oder 1 ist;
L₁ ein (2-10C)-Alkylen-Linker oder eine PEG-Kette ist mit der Formel
-[CH₂CH₂-O]₁₋₃-[CH₂]₁₋₃-;
Y₁ und Y₂ zusammen Folgendes ausbilden:
eine Ethinylen-Gruppe (-C≡C-).
eine Ethylen-Gruppe (-CH=CH-);
-CH₂-O-;
-CH₂-NH-;
oder
-CH₂-CH₂-;
Q ausgewählt ist aus:
(i) 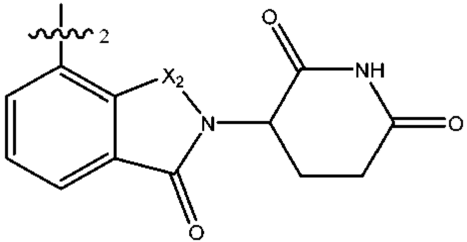
(ii) 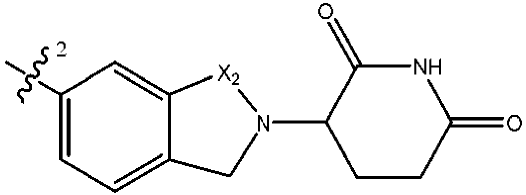
(iii) 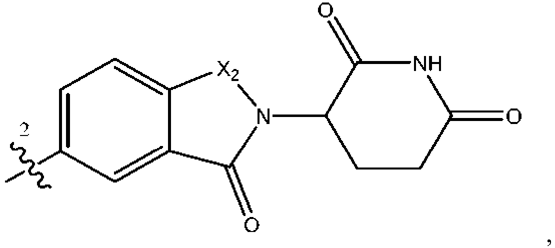
wobei: 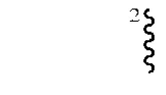
den Punkt der Anbindung an Y₂ oder Gruppe L bezeichnet;
X₂ ausgewählt ist aus -CH₂- oder -C(O)-; mit der Maßgabe, dass X₂ nicht -C(O)- ist, wenn Y₁und Y₂ zusammen eine Ethinylen-Gruppe (-C≡C-) ausbilden.

2. Verbindung oder pharmazeutisch unbedenkliches Salz, Hydrat oder Solvat davon nach Anspruch 1, wobei R₁ -S(O)₂-CH₃ ist.

3. Verbindung oder pharmazeutisch unbedenkliches Salz, Hydrat oder Solvat davon nach Anspruch 1 oder Anspruch 2, wobei entweder eine oder beide der folgenden Aussagen (A)-(B) gelten:
(A) R₂ ist (1-3C)-Alkoxy;
(B) R₂ ist Ethoxy.

4. Verbindung oder pharmazeutisch unbedenkliches Salz, Hydrat oder Solvat davon nach einem der Ansprüche 1 bis 3, wobei entweder eine oder beide der folgenden Aussagen (A)-(B) gelten:
(A) R₃ ist ausgewählt aus Wasserstoff, Fluor oder Methyl;
(B) R₃ ist Wasserstoff.

5. Verbindung oder pharmazeutisch unbedenkliches Salz, Hydrat oder Solvat davon nach einem der Ansprüche 1 bis 4, wobei X₁ N ist.

6. Verbindung oder pharmazeutisch unbedenkliches Salz, Hydrat oder Solvat davon nach einem der vorhergehenden Ansprüche mit der nachstehend dargestellten Strukturformel (Ia): 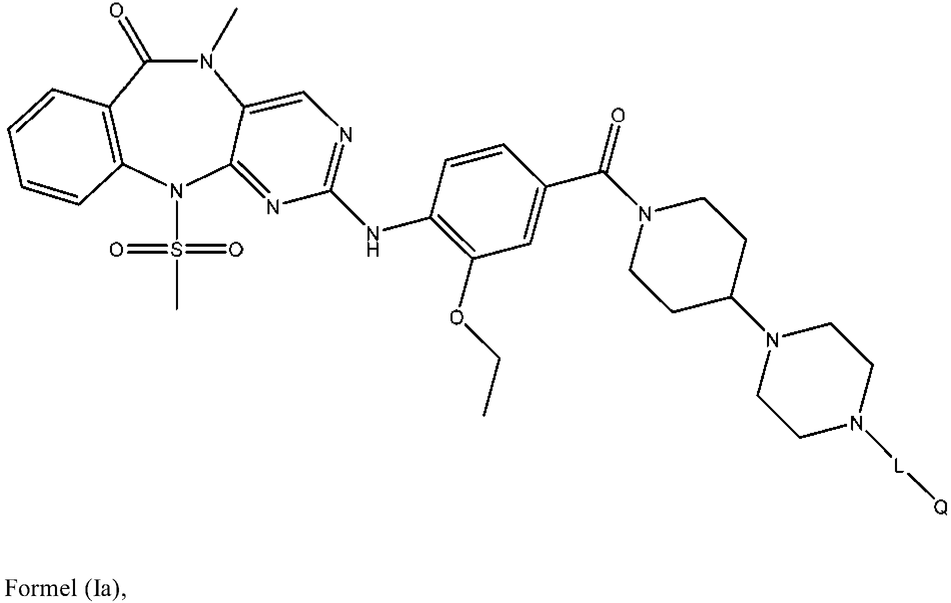 wobei L und Q wie in Anspruch 1 definiert sind.

7. Verbindung oder pharmazeutisch unbedenkliches Salz, Hydrat oder Solvat davon nach einem der Ansprüche 1 bis 6, wobei entweder eine oder mehrere der folgenden Aussagen (A)-(H) gelten:
(A) L ist ein Linker mit der folgenden Formel: 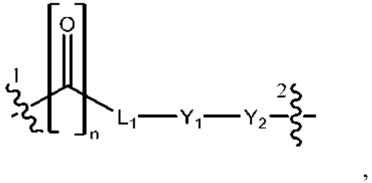 wobei: 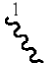
den Punkt der Anbindung an das N-Atom des Piperazinrings bezeichnet; 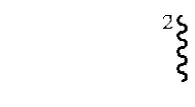
den Punkt der Anbindung an Q bezeichnet;
n 0 oder 1 ist;
L₁ ein (2-8C)-Alkylen-Linker oder eine PEG-Kette ist mit der Formel
-[CH₂CH₂-O]₁₋₃-[CH₂]₁₋₂-;
und
Y₁ und Y₂ zusammen Folgendes ausbilden:
eine Ethinylen-Gruppe (-C≡C-);
-CH₂-O-;
-CH₂-NH-;
oder
-CH₂-CH₂-;
(B) L ist ein Linker mit der folgenden Formel: 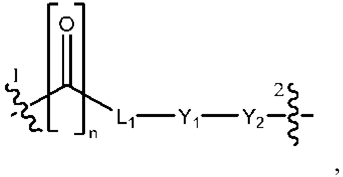 wobei: 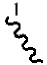
den Punkt der Anbindung an das N-Atom des Piperazinrings bezeichnet; 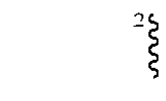
den Punkt der Anbindung an Q bezeichnet;
n 0 oder 1 ist;
L₁ ein (2-8C)-Alkylen-Linker oder eine PEG-Kette ist mit der Formel
-[CH₂CH₂-O]₁₋₂-[CH₂]₁₋₂-;
und
Y₁ und Y₂ zusammen Folgendes ausbilden:
eine Ethinylen-Gruppe (-C≡C-);
-CH₂-O-;
-CH₂-NH-;
oder
-CH₂-CH₂-;
(C) L ist ein Linker mit der folgenden Formel: 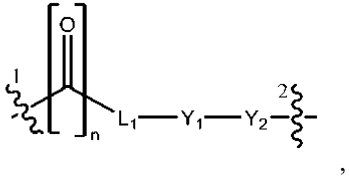 wobei: 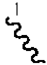
den Punkt der Anbindung an das N-Atom des Piperazinrings bezeichnet; 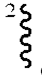
den Punkt der Anbindung an Q bezeichnet;
n 0 oder 1 ist;
L₁ ein (2-8C)-Alkylen-Linker ist; und
Y₁ und Y₂ zusammen Folgendes ausbilden:
eine Ethinylen-Gruppe (-C≡C-);
-CH₂-O-;
-CH₂-NH-;
oder
-CH₂-CH₂-;
(D) L ist ein Linker mit der folgenden Formel: 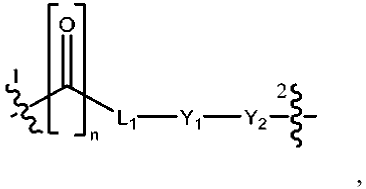 wobei: 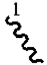
den Punkt der Anbindung an das N-Atom des Piperazinrings bezeichnet; 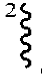
den Punkt der Anbindung an Q bezeichnet;
n 0 ist;
L₁ ein (2-8C)-Alkylen-Linker oder eine PEG-Kette ist mit der Formel
-[CH₂CH₂-O]₁₋₂-[CH₂]₁₋₂-;
und
Y₁ und Y₂ zusammen Folgendes ausbilden:
eine Ethinylen-Gruppe (-C≡C-);
-CH₂-O-;
-CH₂-NH-;
oder
-CH₂-CH₂-;
(E) L ist ein Linker mit der folgenden Formel: 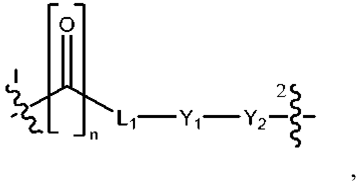 wobei: 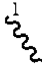
den Punkt der Anbindung an das N-Atom des Piperazinrings bezeichnet; 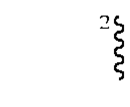
den Punkt der Anbindung an Q bezeichnet;
n 0 oder 1 ist;
L₁ ein (2-8C)-Alkylen-Linker oder eine PEG-Kette ist mit der Formel
-[CH₂CH₂-O]₁₋₂-[CH₂]₁₋₂-;
und
Y₁ und Y₂ zusammen Folgendes ausbilden:
eine Ethinylen-Gruppe (-C≡C-);
(F) L ist ein Linker mit der folgenden Formel: 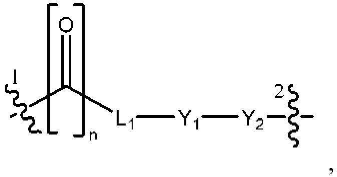 wobei: 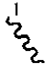
den Punkt der Anbindung an das N-Atom des Piperazinrings bezeichnet; 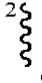
den Punkt der Anbindung an Q bezeichnet;
n 0 ist;
L₁ ein (3-8C)-Alkylen-Linker ist; und
Y₁ und Y₂ zusammen Folgendes ausbilden:
eine Ethinylen-Gruppe (-C≡C-);
-CH₂-O-;
-CH₂-NH-;
oder
-CH₂-CH₂-;
(G) L ist ein Linker mit der folgenden Formel: 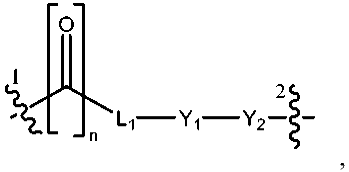 wobei: 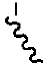
den Punkt der Anbindung an das N-Atom des Piperazinrings bezeichnet; 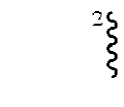
den Punkt der Anbindung an Q bezeichnet;
n 0 ist;
L₁ ein (3-8C)-Alkylen-Linker ist; und
Y₁ und Y₂ zusammen Folgendes ausbilden:
eine Ethinylen-Gruppe (-C≡C-);
(H) L ist ein Linker mit der folgenden Formel: 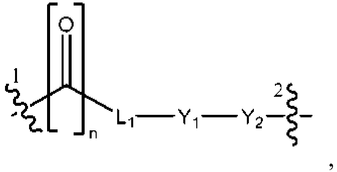 wobei: 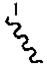
den Punkt der Anbindung an das N-Atom des Piperazinrings bezeichnet; 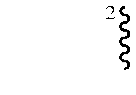
den Punkt der Anbindung an Q bezeichnet;
n 0 ist;
L₁ ein (5-8C)-Alkylen-Linker ist; und
Y₁ und Y₂ zusammen Folgendes ausbilden:
eine Ethinylen-Gruppe (-C≡C-).

8. Verbindung oder pharmazeutisch unbedenkliches Salz, Hydrat oder Solvat davon nach einem der Ansprüche 1 bis 7, wobei L ein Linker ist, der eine Formel aufweist, die ausgewählt ist aus: 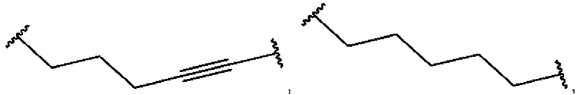 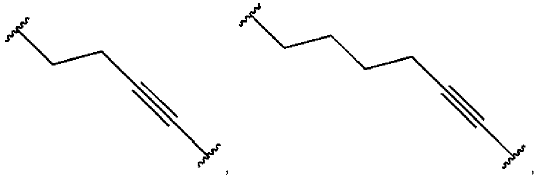 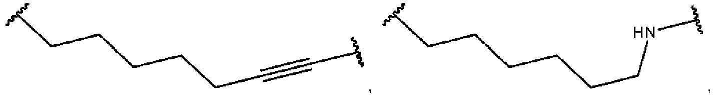 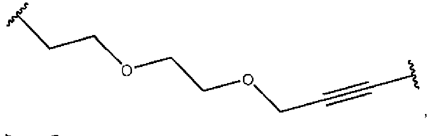 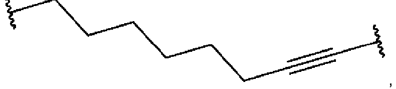 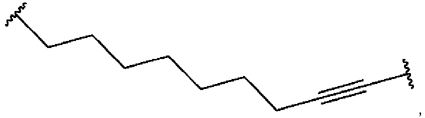 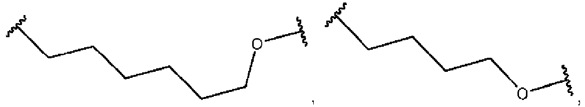 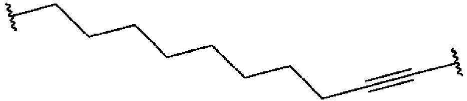 oder

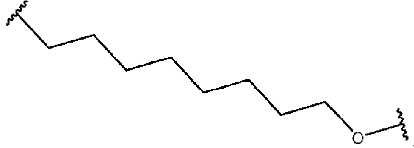

9. Verbindung oder pharmazeutisch unbedenkliches Salz, Hydrat oder Solvat davon nach einem der Ansprüche 1 bis 8, wobei Q ausgewählt ist aus:
(i) 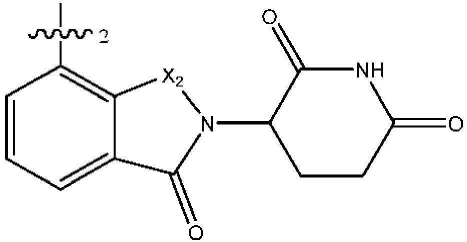 oder
(ii) 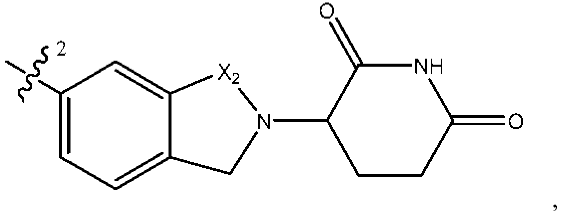
wobei X₂ wie in Anspruch 1 definiert ist.

10. Verbindung oder pharmazeutisch unbedenkliches Salz, Hydrat oder Solvat davon nach einem der Ansprüche 1 bis 9, wobei Q Folgendes ist: 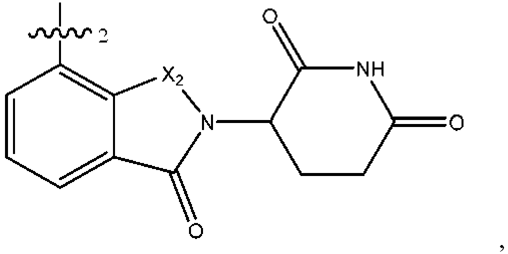 wobei X₂ wie in Anspruch 1 definiert ist.

11. Verbindung oder pharmazeutisch unbedenkliches Salz, Hydrat oder Solvat davon nach einem der Ansprüche 1 bis 10, wobei X2 -CH2- ist.

12. Verbindung nach Anspruch 1, wobei die Verbindung ausgewählt ist aus:
2-(2,6-Dioxopiperidin-3-yl)-4-(5-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pentyl)isoindolin-1,3-dion;
3-(4-(5-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)pent-1-yn-1-yl)-1-oxoisoindolin-2-yl)piperidin-2,6-dion;
3-(4-(4-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)but-1-yn-1-yl)-1-oxoisoindolin-2-yl)piperidin-2,6-dion;
3-(4-(6-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hex-1-yn-1-yl)-1-oxoisoindolin-2-yl)piperidin-2,6-dion;
3-(4-(7-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hept-1-yn-1-yl)-1-oxoisoindolin-2-yl)piperidin-2,6-dion;
2-(2,6-Dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)amino)isoindolin-1,3-dion;
3-(4-(3-(2-(2-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)ethoxy)ethoxy)prop-1-yn-1-yl)-1-oxoisoindolin-2-yl)piperidin-2,6-dion;
3-(4-(8-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)oct-1-yn-1-yl)-1-oxoisoindolin-2-yl)piperidin-2,6-dion;
3-(4-(9-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)non-1-yn-1-yl)-1-oxoisoindolin-2-yl)piperidin-2,6-dion;
2-(2,6-Dioxopiperidin-3-yl)-4-((6-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)hexyl)oxy)isoindolin-1,3-dion;
2-(2,6-Dioxopiperidin-3-yl)-4-(4-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)butoxy)isoindolin-1,3-dion;
3-(4-(10-(4-(1-(3-Ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)dec-1-yn-1-yl)-1-oxoisoindolin-2-yl)piperidin-2,6-dion;
2-(2,6-Dioxopiperidin-3-yl)-4-((8-(4-(1-(3-ethoxy-4-((5-methyl-11-(methylsulfonyl)-6-oxo-6,11-dihydro-5H-benzo[e]pyrimido[5,4-b][1,4]diazepin-2-yl)amino)benzoyl)piperidin-4-yl)piperazin-1-yl)octyl)oxy)isoindolin-1,3-dion;
oder einem pharmazeutisch unbedenklichen Salz, Hydrat oder Solvat davon.

13. Pharmazeutische Zusammensetzung, umfassend eine Verbindung nach den Ansprüchen 1 bis 12 oder ein pharmazeutisch unbedenkliches Salz oder Solvat davon in Beimengung mit einem pharmazeutisch unbedenklichen Verdünnungsmittel oder Träger.

14. Verbindung nach einem der Ansprüche 1 bis 12 oder ein pharmazeutisch unbedenkliches Salz oder Solvat davon oder pharmazeutische Zusammensetzung nach Anspruch 13 zur Verwendung in der Therapie.

15. Verbindung nach einem der Ansprüche 1 bis 12 oder ein pharmazeutisch unbedenkliches Salz oder Solvat davon oder pharmazeutische Zusammensetzung nach Anspruch 13 zur Verwendung bei der Behandlung von Folgendem:
a) einer fibrotischen Krankheit, z. B. gestörte Wundheilung, pulmonale Fibrose,
b) einer entzündlichen Krankheit, z. B. Psoriasis, Asthma,
c) einem proliferativen Zustand, z. B. Krebs, wie dreifach negativer Brustkrebs, Mesotheliom, primärer Knochenkrebs, Glioblastom, Lungenkrebs, Plattenepithelkarzinom,
d) Diabetes,
e) Schmerz oder
f) einer Störung des zentralen Nervensystems (ZNS), z. B. Parkinson-Krankheit, Demenz;
wobei optional die Verbindung zur Verwendung bei der Behandlung von Krebs ist.

## Revendications

1. Composé, ou sel, hydrate ou solvate pharmaceutiquement acceptable de celui-ci, présentant la Formule structurale (I), représentée ci-dessous : 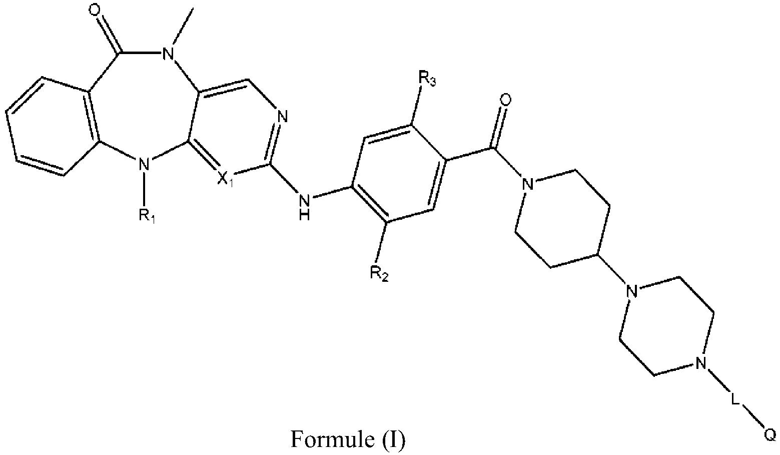 où
R₁ est -S(O)₂-alkyle en (C1-3) ;
R₂ est un alcoxy en (Cl-4) ;
R₃ est choisi parmi un hydrogène, un fluoro, un méthyle, un méthoxy, un trifluorométhyle ou un trifluorométhoxy ;
X₁ est N ou CH ;
L est un coupleur présentant la formule : 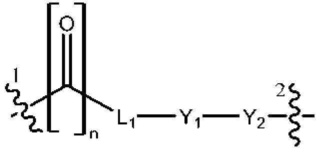
où : 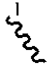
désigne le point de fixation à l'atome N du cycle pipérazine ; 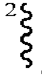
désigne le point de fixation à Q ;
n vaut 0 ou 1 ;
L₁ est un coupleur alkylène en (C2-10) ou une chaîne PEG présentant la formule
-[CH₂CH₂-O]₁₋₃-[CH₂]₁₋₃- ;
Y₁ et Y₂ forment ensemble :
un groupe éthynylène (-C≡C-);
un groupe éthylène (-CH=CH-) ;
-CH₂-O- ;
-CH₂-NH- ;
ou
-CH₂-CH₂- ;
Q est choisi parmi :
(i) 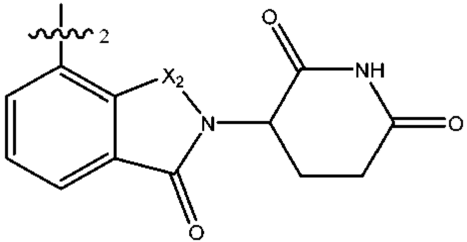
(ii) 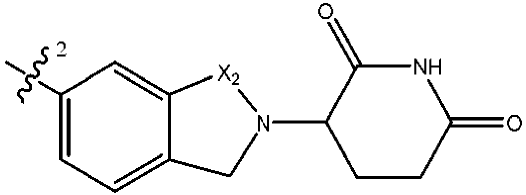
(iii) 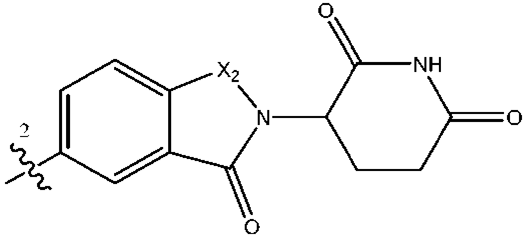
où : 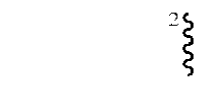
désigne le point de fixation à Y₂ ou au groupe L ;
X₂ est choisi parmi -CH₂- ou -C(O)- ; à condition que X₂ ne soit pas -C(O)- lorsque Y₁ et
Y₂ forment ensemble un groupe éthynylène (-C≡C-).

2. Composé, ou sel, hydrate ou solvate pharmaceutiquement acceptable de celui-ci, selon la revendication 1, dans lequel R₁ est -S(O)₂-CH₃.

3. Composé, ou sel, hydrate ou solvate pharmaceutiquement acceptable de celui-ci, selon la revendication 1 ou la revendication 2, dans lequel l'une quelconque ou les deux affirmations (A) et (B) suivantes s'appliquent :
(A) R₂ est un alcoxy en (C1-3) ;
(B) R₂ est un éthoxy.

4. Composé, ou sel, hydrate ou solvate pharmaceutiquement acceptable de celui-ci, selon l'une quelconque des revendications 1 à 3, dans lequel l'une quelconque ou les deux affirmations (A) et (B) suivantes s'appliquent :
(A) R₃ est choisi parmi un hydrogène, un fluoro ou un méthyle ;
(B) R₃ est un hydrogène.

5. Composé, ou sel, hydrate ou solvate pharmaceutiquement acceptable de celui-ci, selon l'une quelconque des revendications 1 à 4, dans lequel X₁ est N.

6. Composé, ou sel, hydrate ou solvate pharmaceutiquement acceptable de celui-ci, selon l'une quelconque des revendications précédentes, présentant la formule structurale (Ia) indiquée ci-dessous : 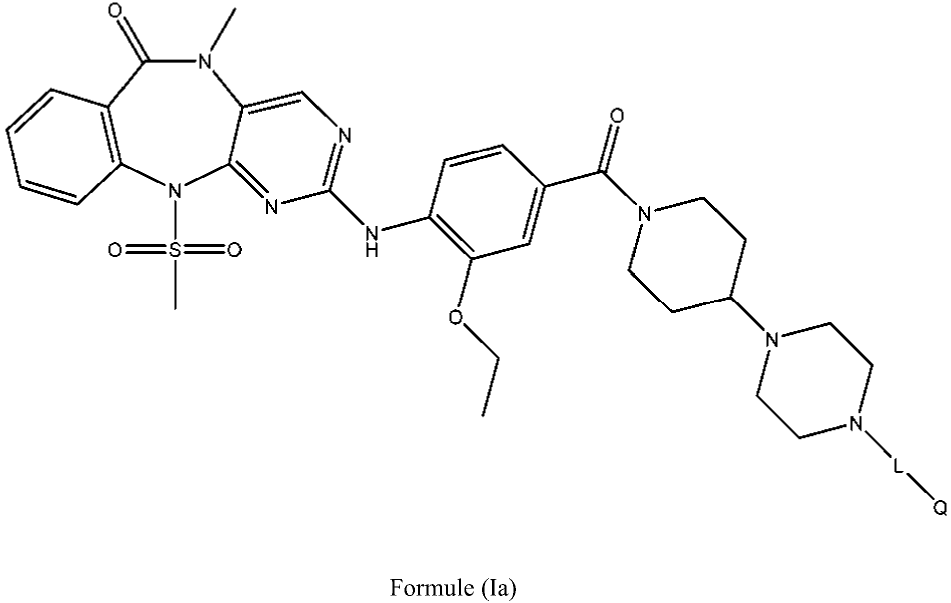 où L et Q sont tels que définis dans la revendication 1.

7. Composé, ou sel, hydrate ou solvate pharmaceutiquement acceptable de celui-ci, selon l'une quelconque des revendications 1 à 6, dans lequel l'une quelconque ou plusieurs des affirmations (A) à (H) suivantes s'appliquent :
(A) L est un coupleur présentant la formule : 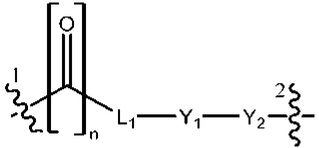 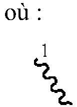
désigne le point de fixation à l'atome N du cycle pipérazine ; 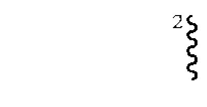
désigne le point de fixation à Q ;
n vaut 0 ou 1 ;
L₁ est un coupleur alkylène en (C2-8) ou une chaîne PEG présentant la formule
-[CH₂CH₂-O]₁₋₃-[CH₂]₁₋₂- ;
et
Y₁ et Y₂ forment ensemble :
un groupe éthynylène (-C≡C-);
-CH₂-O- ;
-CH₂-NH- ;
ou
-CH₂-CH₂- ;
(B) L est un coupleur présentant la formule : 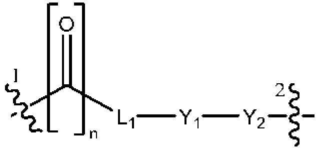 où : 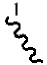
désigne le point de fixation à l'atome N du cycle pipérazine ; 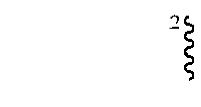
désigne le point de fixation à Q ;
n vaut 0 ou 1 ;
L₁ est un coupleur alkylène en (C2-8) ou une chaîne PEG présentant la formule
-[CH₂CH₂-O]₁₋₂-[CH₂]₁₋₂- ;
et
Y₁ et Y₂ forment ensemble :
un groupe éthynylène (-C≡C-);
-CH₂-O- ;
-CH₂-NH- ;
ou
-CH₂-CH₂- ;
(C) L est un coupleur présentant la formule : 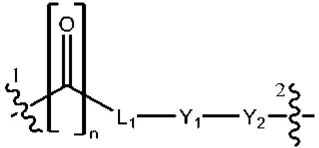 où : 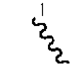
désigne le point de fixation à l'atome N du cycle pipérazine ; 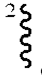
désigne le point de fixation à Q ;
n vaut 0 ou 1 ;
L₁ est un coupleur alkylène en (C2-8) ; et
Y₁ et Y₂ forment ensemble :
un groupe éthynylène (-C≡C-);
-CH₂-O- ;
-CH₂-NH- ;
ou
-CH₂-CH₂- ;
(D) L est un coupleur présentant la formule : 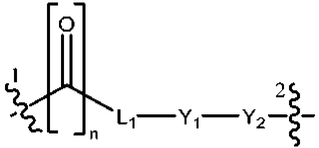 où : 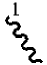
désigne le point de fixation à l'atome N du cycle pipérazine ; 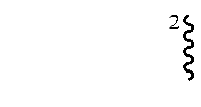
désigne le point de fixation à Q ;
n vaut 0 ;
L₁ est un coupleur alkylène en (C2-8) ou une chaîne PEG présentant la formule
-[CH₂CH₂-O]₁₋₂-[CH₂]₁₋₂- ;
et
Y₁ et Y₂ forment ensemble :
un groupe éthynylène (-C≡C-);
-CH₂-O- ;
-CH₂-NH- ;
ou
-CH₂-CH₂- ;
(E) L est un coupleur présentant la formule : 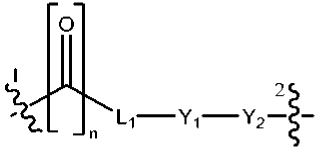 où : 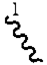
désigne le point de fixation à l'atome N du cycle pipérazine ; 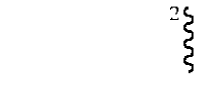
désigne le point de fixation à Q ;
n vaut 0 ou 1 ;
L₁ est un coupleur alkylène en (C2-8) ou une chaîne PEG présentant la formule
-[CH₂CH₂-O]₁₋₂-[CH₂]₁₋₂- ;
et
Y₁ et Y₂ forment ensemble :
un groupe éthynylène (-C≡C-);
(F) L est un coupleur présentant la formule : 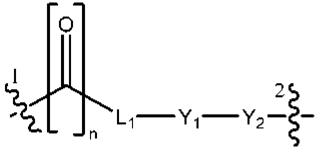 où : 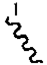
désigne le point de fixation à l'atome N du cycle pipérazine ; 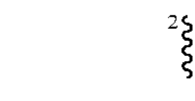
désigne le point de fixation à Q ;
n vaut 0 ;
L₁ est un coupleur alkylène en (C3-8) ; et
Y₁ et Y₂ forment ensemble :
un groupe éthynylène (-C≡C-);
-CH₂-O- ;
-CH₂-NH- ;
ou
-CH₂-CH₂- ;
(G) L est un coupleur présentant la formule : 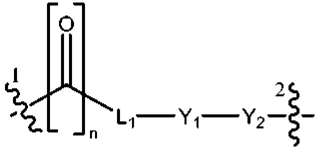 où : 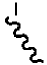
désigne le point de fixation à l'atome N du cycle pipérazine ; 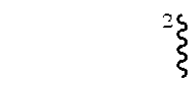
désigne le point de fixation à Q ;
n vaut 0 ;
L₁ est un coupleur alkylène en (C3-8) ; et
Y₁ et Y₂ forment ensemble :
un groupe éthynylène (-C≡C-);
(H) L est un coupleur présentant la formule : 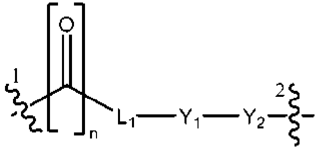 où : 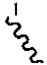
désigne le point de fixation à l'atome N du cycle pipérazine ; 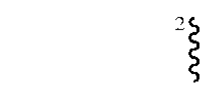
désigne le point de fixation à Q ;
n vaut 0 ;
L₁ est un coupleur alkylène en (C5-8) ; et
Y₁ et Y₂ forment ensemble :
un groupe éthynylène(-C≡C-).

8. Composé, ou sel, hydrate ou solvate pharmaceutiquement acceptable de celui-ci, selon l'une quelconque des revendications 1 à 7, dans lequel L est un coupleur présentant une formule choisie parmi : 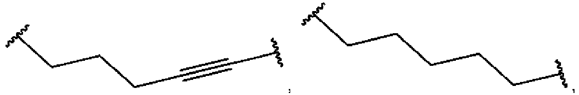 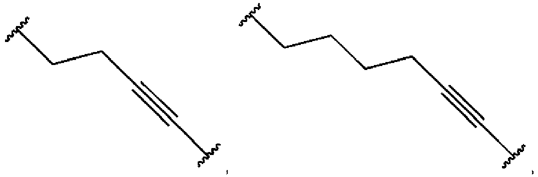 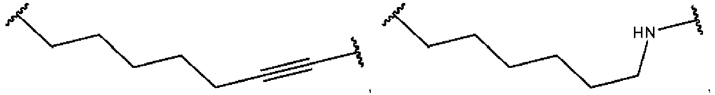 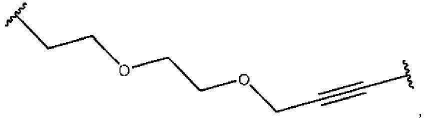 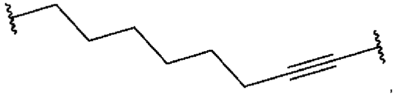 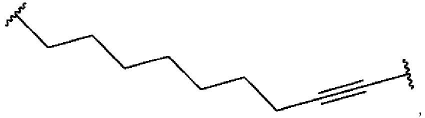 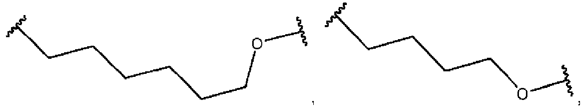 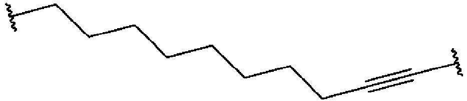 ou

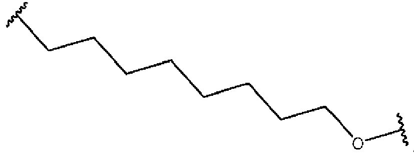

9. Composé, ou sel, hydrate ou solvate acceptable pharmaceutiquement de celui-ci, selon l'une quelconque des revendications 1 à 8, dans lequel Q est choisi parmi :
(i) 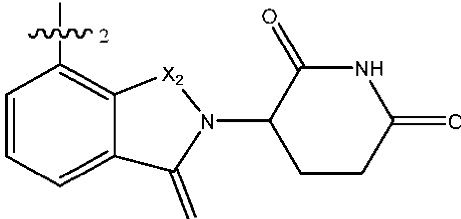 ou
(ii) 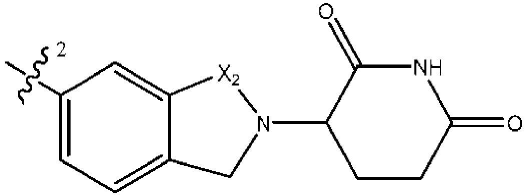 où X₂ est tel que défini dans la revendication 1.

10. Composé, ou sel, hydrate ou solvate pharmaceutiquement acceptable de celui-ci, selon l'une quelconque des revendications 1 à 9, dans lequel Q est : 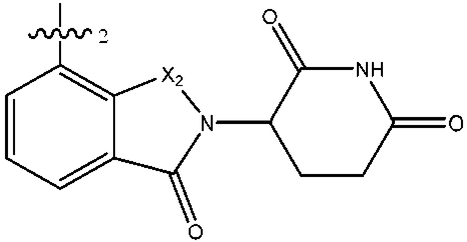 où X₂ est tel que défini dans la revendication 1.

11. Composé, ou sel, hydrate ou solvate acceptable pharmaceutiquement de celui-ci, selon l'une quelconque des revendications 1 à 10, dans lequel X₂ est -CH₂-.

12. Composé selon la revendication 1, dans lequel le composé est choisi parmi :
2-(2,6-dioxopipéridin-3-yl)-4-(5-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)pentyl)isoindoline-1,3-dione ;
3-(4-(5-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)pent-1-yn-1-yl)-1-oxoisoindolin-2-yl)pipéridine-2,6-dione ;
3-(4-(4-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)but-1-yn-1-yl)-1-oxoisoindolin-2-yl)pipéridine-2,6-dione ;
3-(4-(6-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)hex-1-yn-1-yl)-1-oxoisoindolin-2-yl)pipéridine-2,6-dione ;
3-(4-(7-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)hept-1-yn-1-yl)-1-oxoisoindolin-2-yl)pipéridine-2,6-dione ;
2-(2,6-dioxopipéridin-3-yl)-4-((6-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)hexyl)amino)isoindoline-1,3-dione ;
3-(4-(3-(2-(2-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)éthoxy)éthoxy)prop-1-yn-1-yl)-1-oxoisoindolin-2-yl)pipéridine-2,6-dione ;
3-(4-(8-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)oct-1-yn-1-yl)-1-oxoisoindolin-2-yl)pipéridine-2,6-dione ;
3-(4-(9-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)non-1-yn-1-yl)-1-oxoisoindolin-2-yl)pipéridine-2,6-dione ;
2-(2,6-dioxopipéridin-3-yl)-4-((6-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)hexyl)oxy)isoindoline-1,3-dione ;
2-(2,6-dioxopipéridine-3-yl)-4-(4-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridine-4-yl)pipérazine-1-yl)butoxy)isoindoline-1,3-dione ;
3-(4-(10-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)déc-1-yn-1-yl)-1-oxoisoindolin-2-yl)pipéridine-2,6-dione ;
2-(2,6-dioxopipéridin-3-yl)-4-((8-(4-(1-(3-éthoxy-4-((5-méthyl-11-(méthylsulfonyl)-6-oxo-6,11-dihydro-5H-benzo[e]pyrimido[5,4-b][1,4]diazépin-2-yl)amino)benzoyl)pipéridin-4-yl)pipérazin-1-yl)octyl)oxy)isoindoline-1,3-dione ;
ou sel, hydrate ou solvate pharmaceutiquement acceptable de celui-ci.

13. Composition pharmaceutique comprenant un composé selon les revendications 1 à 12, ou un sel ou un solvate pharmaceutiquement acceptable de celui-ci, en mélange avec un diluant ou un support pharmaceutiquement acceptable.

14. Composé tel que défini dans l'une quelconque des revendications 1 à 12, ou sel ou solvate pharmaceutiquement acceptable de celui-ci, ou composition pharmaceutique telle que définie dans la revendication 13, destiné(e) à être utilisé(e) en thérapie.

15. Composé tel que défini dans l'une quelconque des revendications 1 à 12, ou sel ou solvate pharmaceutiquement acceptable de celui-ci, ou composition pharmaceutique telle que définie dans la revendication 13, destiné(e) à être utilisé(e) dans le traitement :
a) d'une maladie fibrotique, par exemple une altération de la cicatrisation des plaies, une fibrose pulmonaire,
b) d'une maladie inflammatoire, par exemple le psoriasis, l'asthme,
c) d'une affection proliférative, par exemple un cancer, tel qu'un cancer du sein triple négatif, un mésothéliome, un cancer primitif des os, un glioblastome, un cancer du poumon, un carcinome épidermoïde,
d) du diabète,
e) de la douleur, ou
f) d'un trouble du système nerveux central (SNC), par exemple la maladie de Parkinson, la démence ; éventuellement dans lequel le composé est destiné à être utilisé dans le traitement du cancer.
